# FASCICULE DE BREVET EUROPEEN

(11) **EP 0 951 537 B1**
(45) Date de publication et mention de la délivrance du brevet: **02.11.2005**
(21) Numéro de dépôt: 97952089.7
(22) Date de dépôt: 17.12.1997
(51) Int. Cl.: C12N 15/00, C12N 15/82, A01H 5/00

(54) **COLLAGENES RECOMBINANTS PRODUITS PAR LES PLANTES, LEURS PROCEDES D'OBTENTION ET LEURS UTILISATIONS**
DURCH PFLANZEN HERGESTELLTES REKOMBINANTES KOLLAGEN, VERFAHREN ZU IHRER HERSTELLUNG UND VERWENDUNG
RECOMBINANT COLLAGEN PRODUCED BY PLANTS, METHODS FOR OBTAINING THEM AND USES

(30) Priorité: 17.12.1996 FR 9616224
(43) Date de publication de la demande: 27.10.1999
(73) Titulaire: MERISTEM THERAPEUTICS S.A., 63100 Clermont-Ferrand (FR)
(72) Inventeur: GRUBER, Véronique-Résidence Sainte Madeleine, F-63400 Chamalières (FR); EXPOSITO, Jean-Yves, F-38090 Bonnefamille (FR); RUGGIERO, Florence, F-69100 Villeurbanne (FR); COMTE, Jeanne, F-69110 Sainte Foy les Lyon (FR); GARRONE, Robert, F-69500 Bron (FR); MEROT, Bertrand, F-63530 Volvic (FR); BOURNAT, Philippe, F-63000 Clermont-Ferrand (FR)
(74) Mandataire: Thurgood, Alexander John
(86) Numéro de dépôt international: PCT/FR1997/002331
(87) Numéro de publication internationale: WO 1998/027202

(56) Documents cités:
- WO-A-93/07889
- WO-A-93/21320
- WO-A-95/14099
- WO-A-96/03051
- WO-A-96/21029
- WO-A-97/04123
- WO-A-98/06861
- FR-A- 2 685 347
- GOLDBERG, I., ET AL.: "Cloning and expression of a collagen-analog-encoding synthetic gene in Escherichia coli" GENE, vol. 80, 1989, pages 305-314, XP002042007
- GOMORD V ET AL: "PLANTE ET MEDICAMENT, UN NOUVEAU DEPART" BIOFUTUR, vol. 154, mars 1996, pages 26-30, XP002033486

## Description

La présente invention a pour objet la production par les plantes de collagènes recombinants, notamment du collagène de type I homocatenaire [α1 (I)₃], ainsi que leurs utilisations.

On connaît dans l'art antérieur le brevet WO 9603051 qui concerne la production de collagène dans le lait d'animaux transgéniques.

Le collagène est une protéine animale fibreuse extracellulaire, largement répandue dans les tissus animaux (récemment détectée également chez certains champignons). Certains organes en contiennent des quantités importantes : la peau (au niveau du derme), les tendons, les os. Il s'agit en fait d'un polymère dont les propriétés remarquables résultent à la fois des caractéristiques en triple hélice de certains domaines de sa molécule et de la régularité de ses assemblages supra-moléculaires. Elle est impliquée dans l'organisation de la matrice extracellulaire et regroupant une vingtaine de molécules différentes, appelées "types", et répertoriées en chiffres romains (actuellement de I à XIX) . Le domaine en triple hélice caractéristique est formé par l'enroulement de trois peptides, ou chaînes α, organisés en hélice gauche et relevant d'une conformation unique, l'hélice α du collagène. Cette conformation spécifique résulte de la répétition d'un triplet d'acides aminés, Gly-X-Y ou X est fréquemment représenté par la proline et Y par l'hydroxyproline. Ces aminoacides assurent la stabilité de ce type d'hélice. Dans une molécule de collagène, les trois chaînes α (identifiées par un indice en chiffre arabe) disposées en une super hélice droite, peuvent être identiques (α1 (ou alpha1)), de deux sortes (α1 et α2) ou toutes différentes (α1, α2, α3). Les molécules de collagène comportent donc des domaines hélicoïdaux (ou domaines collagènes) et des domaines non hélicoïdaux. Elles s'associent en polymères homo ou hétérotypiques. Ainsi, les fibrilles de collagène qui constituent l'essentiel du derme sont-elles composées majoritairement du collagène de type I [α1 (I)₂ α2 (I)] associé, à des collagènes de type III [α1 (III)₃], de type V [α1 (V)₂ α2 (V)] et recouvertes de collagènes de type XII (α1 (XII)₃) et/ou XIV [α1 (XIV)₃]. Des variantes peuvent exister : par exemple on connaît dans des tissus embryonnaires un collagène de type I homocatenaire [α1 (I)₃]. Au cours de la biosynthèse du collagène, certains résidus prolyl et lysil sont hydroxylés, une addition de galactose, éventuellement complétée par un glucose peut se produire sur certains résidus hydroxyl et des N et O glycosylations classiques sont susceptibles d'intervenir sur les domaines non hélicoïdaux. La reconnaissance des trois chaînes formant une molécule et le début de leur assemblage sont sous le contrôle de l'extrémité C-terminale (C-propeptide). Le collagène de type I subit une coupure enzymatique de ses extrémités non hélicoïdales à la suite du signal peptide clivé précédemment, les N et C propeptides terminaux sont excisés au cours de la maturation du collagène laissant subsister des courtes extensions terminales non hélicoïdales (les télopeptides). Ce sont ces molécules clivées qui s'assemblent en polymères ordonnés (les fibrilles de collagène) et qui au cours du temps subissent une réticulation croisée via les résidus hydroxylysyl d'une molécule et les télopeptides d'une molécule adjacente. Les propriétés mécaniques et biologiques du collagène ont été exploitées depuis longtemps : une fois réticulé de manière irréversible (procédé de tannage) il fournit le cuir ; dénaturé par chauffage, il donne naissance à la gélatine et à des colles. Mais ce n'est qu'au cours de la dernière décennie que le collagène à véritablement fourni des biomatériaux à usage pharmaceutique (compresses hémostatiques, éponges, pansements notamment cicatrisants), médicaux (prothèses telles que valves cardiaques, tendons et ligaments, substituts cutanés, agents de comblement), odontologiques (implants gingivaux) et cosmétiques (additif, microconteneur de substances parfumées).

La meilleure connaissance ultérieure de cette protéine et des méthodes de purification ont conduit à la préparation de collagènes bovin et placentaire humain sous une forme prédéfinie : gel, éponge, poudre, fil, et microsphère par exemple. L'ingénierie des matrices extracellulaires trouve également son application dans la constitution d'organoïdes renfermant des cellules transfectées pour des applications de thérapie génique par exemple. Le collagène majoritairement utilisé est le type I (en général associé à du type III) pour des raisons d'abondance et de faible coût de purification et les sources principales en ont été bovine (peaux impropres à la tannerie) ovine (peau et intestins) et humaine (placenta). Cette dernière source ayant été exclusivement réservée à des applications pharmaceutiques ou médicales.

Alors que les propriétés mécaniques et biologiques très utiles du collagène sont reconnues sans ambiguïté, l'utilisation de cette protéine est remise en question en raison des risques éventuels de contamination par des agents infectieux non conventionnels. En effet, si les risques posés par des contaminations bactériennes ou virales peuvent être parfaitement maîtrisés, il n'en est pas de même pour ceux associés aux agents de type prions. Ces agents infectieux qui semblent de nature protéique interviennent dans le développement d'encéphalopathies dégénératives animales (tremblante du mouton, encéphalopathie spongiforme bovine) et humaines (syndrome de Creutzfeld-Jacob, syndrome de Gertstmann-Straussler, kuru). Le délai de leur expression éventuelle est telle que les contrôles formels sont difficiles à réaliser. Ces risques ont déjà pratiquement bloqué toute commercialisation de collagène humain et les dispositions réglementaires édictées en ce qui concerne les collagènes animaux concernés compliquent les procédés de purification et augmentent leur coût.

Devant ces difficultés et face à une détérioration radicale de l'image du collagène de mammifère, une solution est la production de collagène recombinant que l'on pourrait purifier aisément dans un système non susceptible de provoquer des risques pathogènes pour l'homme et dont le coût industriel n'est pas prohibitif. Les inventeurs ont donc découvert et mis au point une production de collagène dans des espèces végétales. Par exemple, nous avons pu produire un collagène humain de type I. Sa molécule comporte une longue triple hélice ininterrompue et est très peu immunogène après purification. Les inventeurs ont par exemple fait exprimer la chaîne α1 (I) afin d'obtenir des molécules homocaténaires α1 (I)₃ semblables à celles qui existent dans certains tissus, notamment embryonnaires.

Les cellules animales sont, a priori, plus adaptées à l'expression de gènes de mammifères. Leur utilisation pose cependant des problèmes de maturation des protéines. L'équipement enzymatique qui réalise la maturation post-traductionnelle est différent d'un tissu, d'un organe ou d'une espèce à l'autre. Par exemple, il a été rapporté que la maturation post-traductionnelle d'une protéine plasmatique peut être différente lorsqu'elle est obtenue à partir du sang humain ou bien lorsqu'elle est produite par une cellule recombinante comme les cellules d'ovaires de hamster chinois ou dans le lait d'un animal transgénique. Par ailleurs, les faibles niveaux d'expression obtenus avec les cellules des mammifères impliquent des cultures in vitro en très grands volumes à des coûts élevés. La production de protéines recombinantes dans le lait des animaux transgéniques (souris, brebis et vaches) permet de diminuer les coûts de production et de surmonter les problèmes de niveau d'expression. Cependant, il reste des problèmes d'éthique et de contaminations virales et subvirales (prions).

Pour ces raisons, la transgénèse de gènes de mammifères dans une cellule végétale pourrait offrir une voie de production en grandes quantités de protéines recombinantes nouvelles, à un coût de production réduit et sans risque de contaminations virales ou subvirales. En 1983, plusieurs laboratoires ont découvert qu'il était possible de transférer un gène hétérologue dans le génome d'une cellule végétale et de régénérer des plantes transgéniques à partir de ces cellules génétiquement modifiées. Toutes les cellules de la plante possèdent alors le caractère génétiquement modifié qui est transmis à la descendance par fécondation sexuée.

Grâce à ces travaux, diverses équipes se sont intéressées à la production de protéines recombinantes de mammifères dans les cellules végétales ou dans des plantes transgéniques (Barta *et al*., 1986; Marx et al., 1982). L'un des premiers résultats vraiment significatif dans ce domaine a été la production d'anticorps dans les plantes de tabac transgéniques. Pour exprimer une protéine hétérologue dans la graine, lieu de stockage des protéines chez les végétaux, l'équipe de Vandekerckhove a fusionné la séquence codant pour la leu-enképhaline au gène codant pour l'albumine 2S *d'Arabidopsis thaliana*. Avec cette construction, des plantes d'Arabidopsis transgéniques ont été produites qui expriment la leu-enképhaline spécifiquement dans les graines à des niveaux d'expression de l'ordre de 0,1% des protéines totales. En 1990, le gène de la sérum albumine humaine a été transféré dans des cellules de tabac et de pomme de terre. Quelle que soit l'origine des peptides signaux (humaine ou végétale), des taux de sérum albumine humaine de l'ordre de 0,02% des protéines totales ont été obtenus notamment dans les feuilles de pomme de terre. D'autres protéines recombinantes de mammifères ont été aussi produites dans les plantes: l'antigène de surface de l'hépatite B, les interférons, un anticorps de souris anti-*Streptococcus mutans*, agent de la carie, des fragments d'anticorps scFV anti-cellules cancéreuses, un anticorps anti-Herpès, la toxine du choléra et le facteur de croissance de l'épiderme humain (E.G.F). L'ensemble de ces recherches a permis de montrer que la production de protéines recombinantes de mammifères dans les cellules végétales est possible et que les mécanismes de la synthèse des protéines à partir des séquences d'ADN sont similaires chez les cellules animales et les cellules végétales. De nombreuses différences existent néanmoins entre les cellules végétales.et animales, notamment au niveau de la maturation des glycannes polymannosidiques en glycannes complexes, ou encore au niveau des sites de clivage des peptides signaux, ne permettant pas ainsi de garantir l'obtention de protéines de mammifères actives ou suffisamment actives par transformation de cellules végétales.

Kirivikko et al (WO 93/07889) ont décrit l'expression de procollagènes humains (types I et II). Un exemple rapporte l'expression de la chaîne alpha-1 ou alpha-2 de procollagène I ou II dans les cellules de mammifère HT1080. Un autre exemple décrit l'expression des chaînes alpha et béta de prolyl-4-hydroxylase dans des cellules d'insectes Sf9. Des suggestions d'exemples pour l'expression de collagènes et de P4H recombinants sont données pour les levures S. cerevisiae et P. pastoris, sans démonstration de résultat.

Une équipe de l'Université Louis Pasteur (FR 2 685 347) a décrit l'expression d'oligomères peptidiques de type Pro-X-Y dans la bactérie E. Coli.

L'utilisation de céréales a été décrite par Rodriguez (WO 95/14099) pour la production de protéines recombinantes hétérologues dans les graines de céréales au cours d'un processus de maltage dans lequel l'expression de la protéine recombinante est induite.

Moloney (WO 96/21029) décrit la production de protéines dans les cellules de plantes sous forme de protéines de fusion avec l'oléosine.

Les inventeurs ont découvert que l'utilisation de cellules végétales transformées par une séquence nucléotidique recombinante appropriée, permet d'obtenir du collagène, notamment du collagène de type I homotrimérique [α1(I)]₃ , recombinant.

Un autre but de la présente invention est de fournir les outils pour la mise en oeuvre d'un tel procédé, notamment de nouvelles séquences nucléotidiques recombinantes, des cellules de plantes transformées génétiquement, des plantes ou parties de plantes (notamment feuilles, tiges, fruits, semences ou graines, racines) transformées génétiquement, et des fragments de ces plantes ou parties de plantes transformées génétiquement.

L'invention a également pour but de fournir de nouveaux collagènes produits par les plantes, notamment du collagène de type I homotrimérique [α1(I)]_{3.}

L'invention a également pour but de fournir de nouvelles compositions protéiques susceptibles d'être utilisées dans le cadre de la mise en oeuvre ou la fourniture de compositions pharmaceutiques, médicales, odontologiques, cosmétiques, biotechnologiques ou industrielles.

### DESCRIPTION DETAILLE DE L'INVENTION:

L'invention concerne :
- l'utilisation d'une séquence nucléotidique recombinante contenant, d'une part, un ADNc codant pour une ou plusieurs chaînes de collagène de mammifère notamment celle contenant à titre d'ADNc, celui de la chaîne du collagène α1, et d'autre part, les éléments permettant à une cellule végétale de produire la ou les chaînes de collagène, codées par ledit ADNc, notamment un promoteur et un terminateur de transcription reconnus par la machinerie transcriptionnelle des cellules végétales, pour la transformation de cellules de plantes en vue de l'obtention, à partir de ces cellules, ou de plantes obtenues à partir de ces dernières, de la ou des chaînes de collagène, le cas échéant sous forme de triple hélice,
- une séquence nucléotidique recombinante, caractérisée en ce qu'elle contient d'une part la séquence codante pour une ou plusieurs chaînes de collagène de mammifère, notamment celle de la chaîne du collagène α1, et d'autre part, les éléments permettant à une cellule végétale de produire la ou les chaînes de collagène de mammifère codées par ladite séquence, le cas échéant sous forme de triple hélice, dont un promoteur et un terminateur de transcription reconnus par la machinerie transcriptionnelle des cellules végétales.
- un vecteur, notamment un plasmide, contenant une séquence nucléotidique selon l'invention insérée en un site non essentiel pour sa réplication.
- un hôte cellulaire, notamment toute bactérie telle que Agrobacterium tumefaciens, transformé par un vecteur selon l'invention,
- un procédé d'obtention d'une ou plusieurs chaînes de collagène, le cas échéant sous forme de triple hélice, caractérisé en ce qu'il comprend :

- la transformation de cellules végétales, notamment à l'aide d'un hôte cellulaire selon l'invention, lui-même transformé par un vecteur selon l'invention, de manière à intégrer dans le génome de ces cellules une séquence recombinante selon l'invention,
- l'obtention de plantes transformées à partir des cellules transformées susmentionnées,
- la récupération de la ou des chaînes de collagène recombinantes produites dans lesdites cellules ou plantes transformées susmentionnées, par extraction, suivie, par une purification,
- une plante ou une partie de plante, feuilles et/ou fruits et/ou semences et/ou cellules de plantes, transformés génétiquement,
caractérisée en ce qu'elle contient une (ou plusieurs) séquence(s) nucléotidique(s) recombinante(s) selon l'invention, intégrée(s) de façon stable dans leur génome, ou exprimant du collagène recombinant codées par lesdites séquences nucléotidiques, ces plantes étant choisies notamment parmi le colza, le tabac, le maïs, le pois, la tomate, la carotte, le blé, l'orge, la pomme de terre, le soja, le tournesol, la laitue, le riz, la luzerne, et la betterave,
- une ou plusieurs chaînes de collagène recombinantes susceptible d'être obtenue selon le procédé de l'invention,
- un collagène (notamment un collagène de type I,II, III, IV ou V) caractérisé en ce qu'il est obtenu selon le procédé de l'invention,
- un produit, notamment gélatine, caractérisé en ce qu'il est obtenu à partir des chaînes de collagène, du collagène selon l'invention,
- une plante ou une partie de plantes, feuilles et/ou fruits et/ou semences et/ou cellules de plantes, transformés génétiquement, caractérisés en ce qu'ils contiennent des chaînes de collagène, du collagène selon l'invention, ces plantes étant choisies notamment parmi le colza, le tabac, le maïs, le pois, la tomate, la carotte, le blé, l'orge, la pomme de terre, le soja, le tournesol, la laitue, le riz, la luzerne, et la betterave.
- l'utilisation de plantes ou parties de plantes selon l'invention, et/ou de produits (chaînes de collagène, collagène) selon l'invention, pour l'obtention de compositions pharmaceutiques, médicales, odontologiques, cosmétiques ou biotechnologiques,
- un Biomatériau et une composition pharmaceutique, médicale, odontologique, cosmétique ou biotechnologique. caractérisée en ce qu'elle comprend des plantes, parties de plantes ou des chaînes de collagène, du collagène selon l'invention.

Une composition pharmaceutique selon l'invention comprend toute composition selon l'invention constituant (ou entrant dans la fabrication de) une composition permettant de prévenir ou de traiter toute pathologie liée à un dysfonctionnement du collagène, ainsi qu'une compresse pour cicatrisation de plaies, un pansement hémostatique, pansement anti-escarres, une poudre hémostatique ; ou un pansement pour brûlures.

Une composition médicale selon l'invention comprend notamment toute composition selon l'invention constituant (ou entrant dans la fabrication de) un dispositif de recouvrement cornéen, un film coagulant pour résection d'organes (foie en particulier), un matériau de comblement pour prévention d'adhérences et de fistules, une composition pour la confection de prothèses vasculaires et cardiaques (valvules), un conduit-guide pour régénération de nerfs, matériau de comblement osseux et intra corporel, un conteneur-relargueur de substances actives (hormones, facteurs de croissance, antibiotiques, anticancéreux, anti-inflammatoires par exemple), un dispositifs de compression (par exemple pour réduire l'incontinence urinaire), un substituts cutané, un fil chirurgical, un matériau injectable pour chirurgie esthétique (comblement de dépressions cutanées ou remodelage du visage par exemple).

Une composition odontologique selon l'invention comprend notamment toute composition selon l'invention constituant (ou entrant dans la fabrication de) un pansement gingival ou un matériel de comblement.

Une composition cosmétique selon l'invention comprend notamment toute composition selon l'invention constituant (ou entrant dans la fabrication de) un additif pour préparation (crèmes, pommades, fards, onguents).

Une composition médicale selon l'invention comprend notamment toute composition selon l'invention constituant (ou entrant dans la fabrication de) un système de : recouvrement de boîtes et flacons de culture cellulaire.

L'invention concerne également un produit, notamment la gélatine, caractérisé en ce qu'il est obtenu à partir des chaînes de collagène, du collagène ou de leurs protéines dérivées selon l'invention.

En effet la gélatine est normalement préparée à partir du collagène par chauffage des tissus animaux (notamment des os et de la peau) dans l'eau puis séchage (Parkany M, 1984). Cette préparation détruit la structure secondaire du collagène et conduit donc à des changements importants de la solubilité et des propriétés mécaniques du produit. La gélatine est le principal constituant de la colle. Dans l'eau froide, elle gonfle et est insoluble. Dans l'eau chaude, elle se dissout pour donner une solution très visqueuse, qui se gélifie après refroidissement lorsque le taux de gélatine est supérieur à 1%. Pour la préparation de prothèses chirurgicales, des solutions contenant de 10 à 35 % de gélatine sont utilisées.

La propriété de la gélatine d'être soluble dans de l'eau chaude la rend utile pour de nombreuses applications alimentaires et industrielles. Elle est souvent préparée sous forme de poudre ou de fines feuilles. Suivant les propriétés mécaniques recherchées, du glycérol du sorbitol ou des agents de réticulations peuvent par exemple être ajoutés lors de la préparation des blocs de gélatines.

La gélatine est également utilisable dans le domaine bio-médical comme éponges par exemple.

Avantageusement, les séquences nucléotidiques recombinantes selon l'invention contiennent une (ou plusieurs) séquence(s) codant pour un peptide responsable de l'adressage des polypeptides recombinants dans un compartiment déterminé de la cellule végétale, notamment dans le réticulum endoplasmique ou dans les vacuoles, ou bien même à l'extérieur de la cellule, dans la paroi pectocellulosique ou dans l'espace extracellulaire aussi appelé apoplasme.

Parmi les terminateurs de transcription susceptibles d'être utilisés pour la transformation de cellules de plantes dans le cadre de la présente invention, on peut citer le terminateur polyA 35S du virus de la mosaïque du chou-fleur (CaMV), ou le terminateur polyA NOS, qui correspond à la région en 3' non codante du gène de la nopaline synthase du plasmide Ti d'*Agrobacterium tumefaciens* souche à nopaline .

A ce titre, l'invention a pour objet toute séquence nucléotidique recombinante telle que décrite ci-dessus contenant en aval dudit ADNc ou de sa séquence dérivée, le terminateur polyA 35S du CaMV, ou le terminateur polyA NOS d'*Agrobacterium tumefaciens*.

Parmi les promoteurs de transcription susceptibles d'être utilisés pour la transformation de cellules de plantes dans le cadre de la présente invention, on peut citer :
- le promoteur 35S (P35S), ou avantageusement le promoteur constitutif double 35S (Pd35S) du CaMV, ces promoteurs permettant l'expression des polypeptides recombinants de l'invention dans l'ensemble de la plante obtenue à partir de cellules transformées selon l'invention, et sont décrits dans l'article de Kay *et al*., 1987,
- le promoteur PCRU du gène de la cruciférine de radis permettant l'expression des polypeptides recombinants de l'invention uniquement dans les semences (ou graines) de la plante obtenue à partir de cellules transformées selon l'invention, et décrit dans l'article de Depigny-This *et al*., 1992,
   les promoteurs PGEA1 et PGEA6 correspondant à la région 5' non codante des gènes de la protéine de réserve de graines, GEA1 et GEA6, respectivement, d'Arabidopsis thaliana (Gaubier et al., 1993), et permettant une expression spécifique dans les graines,
- le promoteur chimérique super-promoteur PSP (Ni M et al., 1995), constitué de la fusion d'une triple répétition d'un élément activateur transcriptionnel du promoteur du gène de l'octopine synthase d'Agrobacterium tumefaciens, d'un élément activateur transcriptionnel du promoteur du gène de mannopine synthase et du promoteur mannopine synthase d'Agrobacterium tumefaciens,
- le promoteur actine du riz suivi de l'intron actine de riz (PAR-IAR) contenu dans le plasmide pAct1-F4 décrit par McElroy et al. (1991),
- le promoteur HMGW (High Molecular Weight Glutenine) d'orge (Anderson O.D. et al, 1989),
- le promoteur du gène de γzéine de maïs (Pγ zéine) contenu dans le plasmide pγ63 décrit dans Reina et al. (1990), et permettant l'expression dans l'albumen des semences de maïs.

A ce titre, l'invention a pour objet toute séquence nucléotidique recombinante telle que décrite ci-dessus, contenant en amont dudit ADNc ou de sa séquence dérivée, le promoteur constitutif double 35S (Pd35S) du CaMV, ou le promoteur PCRU du gène de la cruciférine de radis, ou les promoteurs PGEA1 ou PGEA6 d'Arabidopsis thaliana, ou le super-promoteur PSP d'Agrobacterium tumefaciens, ou le promoteur PAR-IAR du riz, le promoteur HMGW d'orge ou le promoteur pγzéine du maïs.

Les séquences codant pour un peptide d'adressage utilisées dans le cadre de la présente invention, peuvent être d'origine végétale, humaine ou animale.

Parmi les séquences codant pour un peptide d'adressage d'origine végétale, on peut citer:
- la séquence nucléotidique de 69 nucléotides (indiquée dans les exemples qui suivent) codant pour le prépeptide (peptide signal) de 23 acides aminés de la sporamine A chez la patate douce, ce peptide signal permettant l'entrée des polypeptides recombinants de l'invention dans le système de sécrétion des cellules végétales transformées selon l'invention (à savoir principalement dans le réticulum endoplasmique),
- la séquence nucléotidique de 42 nucléotides (indiquée dans les exemples qui suivent) codant pour le propeptide N-terminal d'adressage vacuolaire de 14 acides aminés de la sporamine A chez la patate douce, permettant l'accumulation des polypeptides recombinants de l'invention dans les vacuoles des cellules végétales transformées selon l'invention,
- la séquence nucléotidique de 111 nucléotides (indiquée dans les exemples qui suivent) codant pour le prépropeptide de 37 acides aminés de la sporamine A constitué de la partie N-terminale vers la partie C-terminale des 23 acides aminés du peptide signal susmentionné suivis par les 14 acides aminés du propeptide susmentionné, ce prépropeptide permettant l'entrée de polypeptides recombinants de l'invention dans le système de sécrétion et leur accumulation dans les vacuoles des cellules végétales transformées selon l'invention,
   les trois séquences susmentionnées étant décrites dans les articles de Murakami *et al*., 1986, et Matsuoka et al, 1991,
- le propeptide carboxyterminal de la lectine d'orge décrit notamment dans les articles de Schroeder *et al*., 1993, et de Bednarek et al, 1991,
- et le PRS (Pathogenesis Related Protein, Cornelissen et al. 1986) permettant la sécrétion.

On peut également citer, parmi les séquences codant pour un peptide d'adressage, celle codant pour les peptides KDEL, SEKDEL et HDEL et permettant un adressage dans le réticulum endoplasmique.

L'invention a également pour objet toute séquence nucléotidique recombinante telle que décrite ci-dessus, contenant une séquence codant pour tout ou partie d'un peptide d'adressage vacuolaire, notamment celui de la sporamine A de la patate douce, cette séquence codant pour un peptide d'adressage vacuolaire étant située, dans ladite séquence nucléotidique recombinante, entre la séquence codant pour un peptide signal et celle codant pour ledit ADNc ou sa séquence dérivée, de telle sorte que le premier acide aminé N-terminal du peptide d'adressage vacuolaire soit lié au dernier acide aminé C-terminal du peptide signal, et que le dernier acide aminé C-terminal dudit peptide d'adressage soit lié au premier acide aminé N-terminal du polypeptide codé par ledit ADNc ou sa séquence dérivée, dans la protéine codée par ladite séquence nucléotidique recombinante.

L'invention a également pour objet toute séquence nucléotidique recombinante telle que décrite ci-dessus, contenant une séquence codant pour tout ou partie d'un peptide d'adressage vacuolaire, notamment celui de la lectine d'orge, cette séquence codant pour un peptide d'adressage vacuolaire étant située, dans ladite séquence nucléotidique recombinante, en aval de la séquence codant pour ledit ADNc ou sa séquence dérivée, de telle sorte que le premier acide aminé N-terminal du peptide d'adressage vacuolaire soit lié au dernier acide aminé C-terminal du polypeptide codé par ledit ADNc ou sa séquence dérivée, dans la protéine codée par ladite séquence nucléotidique recombinante.

### DESCRIPTION DETAILLEE DES FIGURES

Fig 1:
   Immunotransfert montrant un exemple de résultat positif issus du criblage de plants transformés par les constructions pBIOC706 (indiqué 706, plant n°45, pistes 2,3) et pBIOC707 (indiqué 707, plant n°2, pistes 4,5). Ces résultats sont à comparer avec ceux obtenus avec les plants témoins non transformés (pistes 6, 7). La piste 1 présente la migration électrophorètique du collagène I témoin: la bande correspondant à α 2(I) migre au niveau de la bande 116 KDa des poids moléculaires standards, la bande α1(I) migre légèrement au dessus. Les pistes 2; 4 6 correspondent aux surnageants d'extraction en milieu acide, les pistes 3, 5 et 7 correspondent aux culots. La construction pBIOC706 génère une bande majoritaire reconnue par l'anticorps anti-collagène I, migrant exactement au niveau de la bande α1(I) témoin (piste 1); la construction pBIOC707 une bande majoritaire migrant à 140 KDa. L'extraction en milieu acide semble cependant incomplète puisque les mêmes bandes se retrouvent dans les culots d'extraction correspondants (piste 3 pour pBIOC706, piste 5 pour pBIOC707). Les pistes correspondant au plant témoin (pistes 6 et 7) sont négatives.
Fig 2:
   Immunotransfert montrant quelques exemples de plants positifs transformés par les constructions PBIOC706 (plants n°40: pistes 2, 3 et n°45: pistes 4, 5) et PBIOC707 (plants n°1: pistes 6, 7; plant n°2: pistes 8, 9; plant n°14: pistes 10, 11) extraits en milieu neutre. La piste 1 présente la migration électrophorétique du collagène I témoin: la bande correspondant à α 2(I) migre au niveau de la bande 116KDa des poids moléculaires standards, la bande α1(I) migre légèrement au dessus. Les pistes 2, 4, 6, 8, et 10 correspondent aux surnageants d'extraction en milieu neutre, les pistes 3, 5, 7, 9 et 11 correspondent aux culots. Les plants 40 et 45 (construction PBIOC706) génèrent deux bandes majoritaires reconnues par l'anticorps anti-collagène I, migrant respectivement au niveau de la bande α1(I) témoin (piste 1) et à 140 KDa. Les plants 1, 2 et 14 (construction PBIOC707) génèrent 2 bandes majoritaires migrant respectivement à 140 KDa et 160 KDa. L'extraction en milieu neutre semble complète puisque aucune bande ne se retrouve dans les culots d'extraction correspondants (pistes 3 et 5 pour PBIOC706, pistes 7, 9 et 11 pour PBIOC707). Les pistes correspondant au plant témoin (non montrées) sont négatives. Tous les plants positifs exhibent le même profil électrophorétique.
Fig 3:
   Immunotransfert montrant les modifications des profils électrophorétiques de plants positifs transformés par les constructions PBIOC707 (panneau A et B) et PBIOC706 (panneau C) en présence (+) ou non (-) d'agent réducteur (DTT). Les pistes 1 et 8 présentent la migration électrophorétique du collagène I témoin. Panneau A: plant n° 2 extrait en milieu acide. La bande unique après réduction (piste 2) que l'on observe après extraction en milieu acide montre une migration légèrement plus rapide en l'absence d'agent réducteur (piste 3). Ce résultat suggère que la bande observée correspond à la chaîne α1(I) comprenant le N-propeptide (présence de ponts di-sulfures intrachaînes). Panneau B: plant n°2 (pistes 4 et 5) et plant n°1 (pistes 6 et 7) extrait en milieu neutre. En l'absence d'agent réducteur (pistes 5 et 7) la bande supérieure, migrant à 160 KDa en présence d'agent réducteur (pistes 4 et 6), se retrouve au niveau des bandes γ (3 chaînes α associées). La bande migrant à 140 KDa, en présence d'agent réducteur, migre légèrement plus rapidement en l'absence réducteur comme observé panneau A. Panneau C: plant n° 40 (pistes 9 et 10) et plant n°45 (pistes 11 et 12) extraits en milieu neutre. Dans ce cas à nouveau, seule la migration de la bande supérieure est modifiée en l'absence de l'agent réducteur et se retrouve en majorité, comme observée panneau B, au niveau des bandes γ . Ces résultats indiquent que la bande supérieure pour chacune des constructions, PBIOC706 et PBIOC707, comprend le C-propeptide. Ainsi les ponts di-sulfures interchaines qui se font entre les C-propeptides de la molécule native ont pu être réalisés chez la plante. Le pontage des C-propeptides est connu pour permettre une mise en registre correcte des 3 chaînes et initier ainsi la formation de la triple hélice. Les bandes inférieures (140 KD pour la construction PBIOC707 et 120 KDa pour la construction PBIOC706) résulterait donc du clivage du domaine C-propeptidique de la chaîne α1(I) recombinante. Ce clivage est une étape importante dans la maturation du collagène natif puisqu'il est nécessaire pour la polymérisation en fibre du collagène.

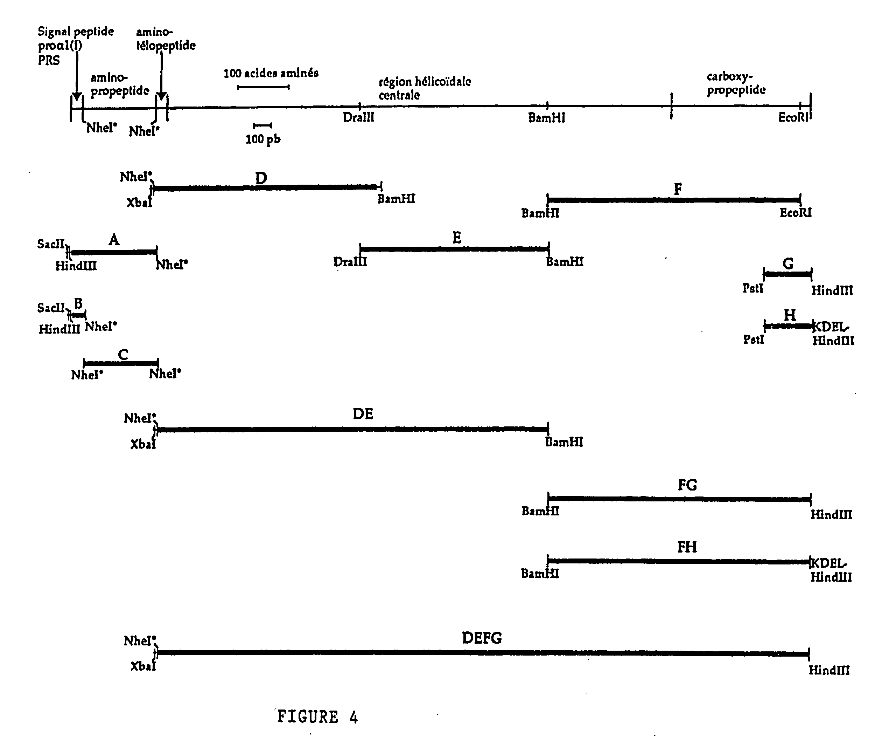

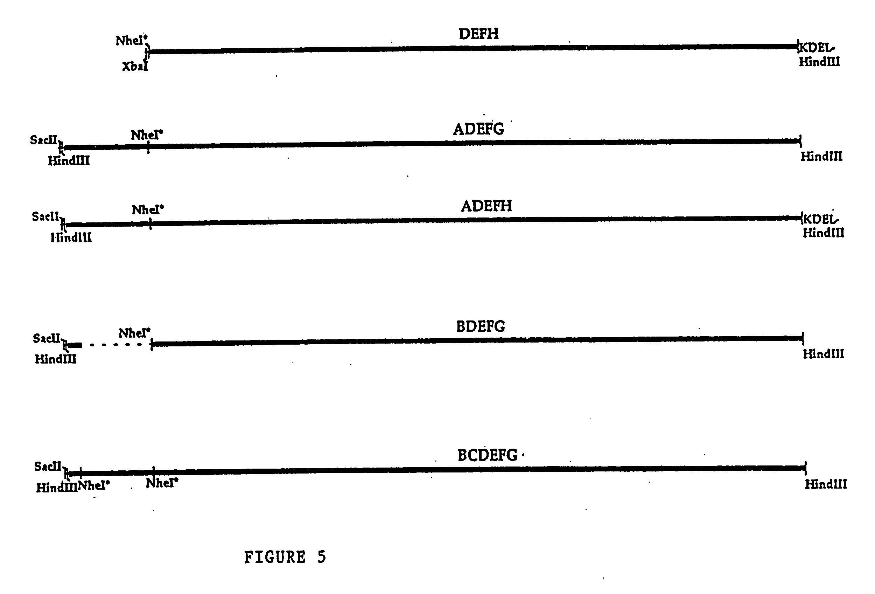

Figures 4 et 5 : représentent les fragments utilisés par les constructions pBIOC21-collagène.
Figure 6 :
   - Partie A: Digestion par la trypsine de collagène extrait de plants transformés avec la construction pBIOC706 (plant 45) analysée par électrophorèse sur gel acrylamide 6%. Piste 1: collagène non digéré. Piste 2: collagène digéré par la trypsine à 25°C, la bande correspondant au collagène est toujours présente attestant la structure en triple hélice du collagène extrait. Piste 3: collagène dénaturé par la chaleur (50°C, 20 minutes) puis digéré par la trypsine, la bande correspondant au collagène disparait témoin de l'efficacité de la trypsine lorsque le collagène est dénaturé.
   - Partie B: Micrographie d'une réplique obtenue après ombrage tournant du collagène purifié à partir du plant 45. Les molécules de collagène sont observées sous forme de bâtonnets de 280-300 nm de long et de 1.4 nm de diamètre, caractéristique du repliement en triple hélice de tout le domaine central du collagène I. Grandissement: X 75 000.

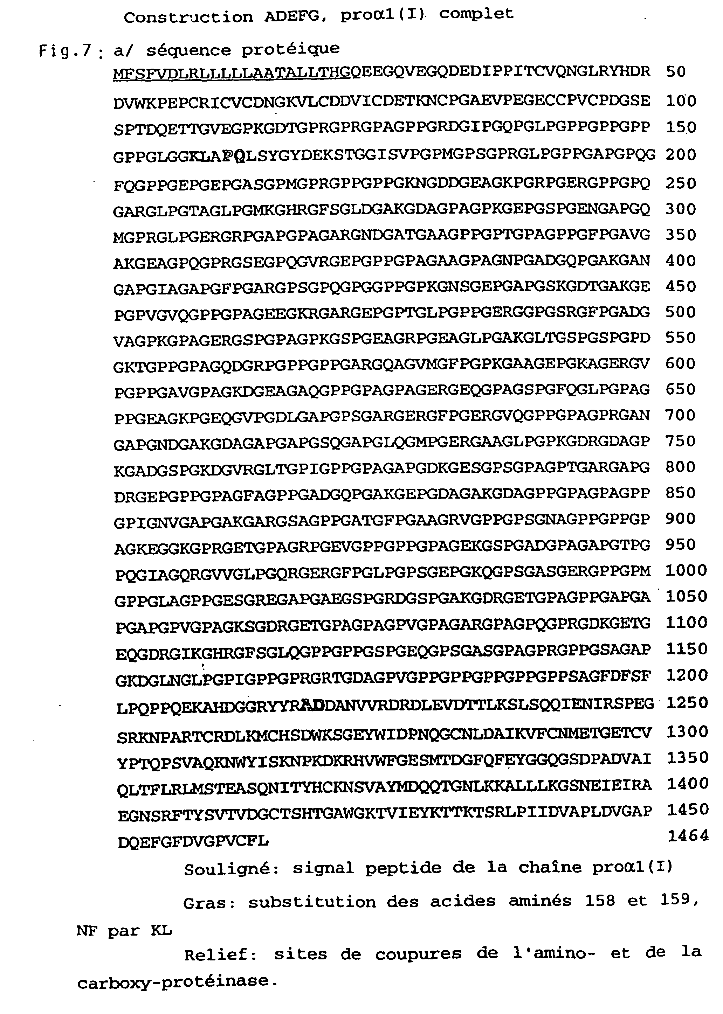

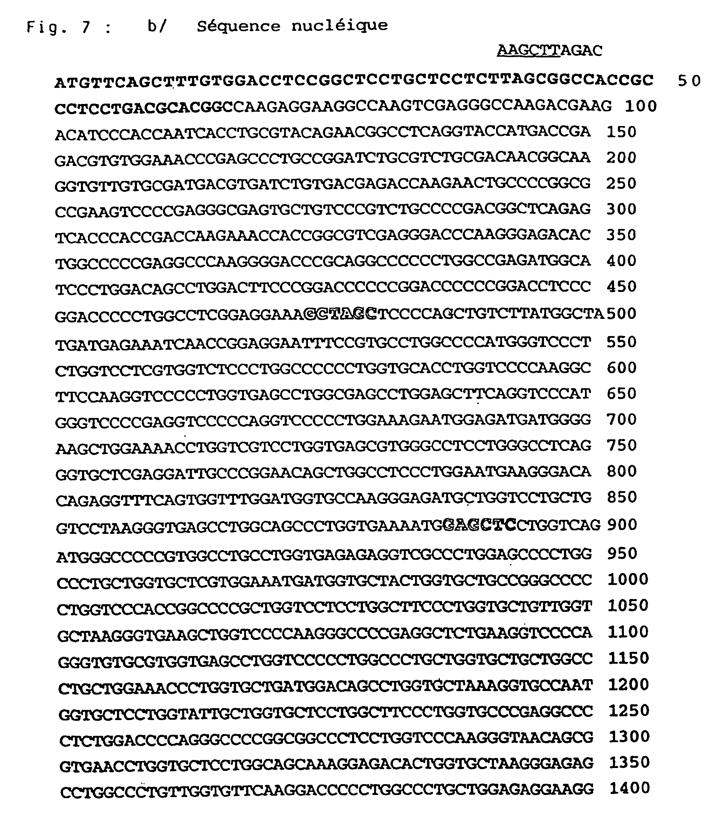

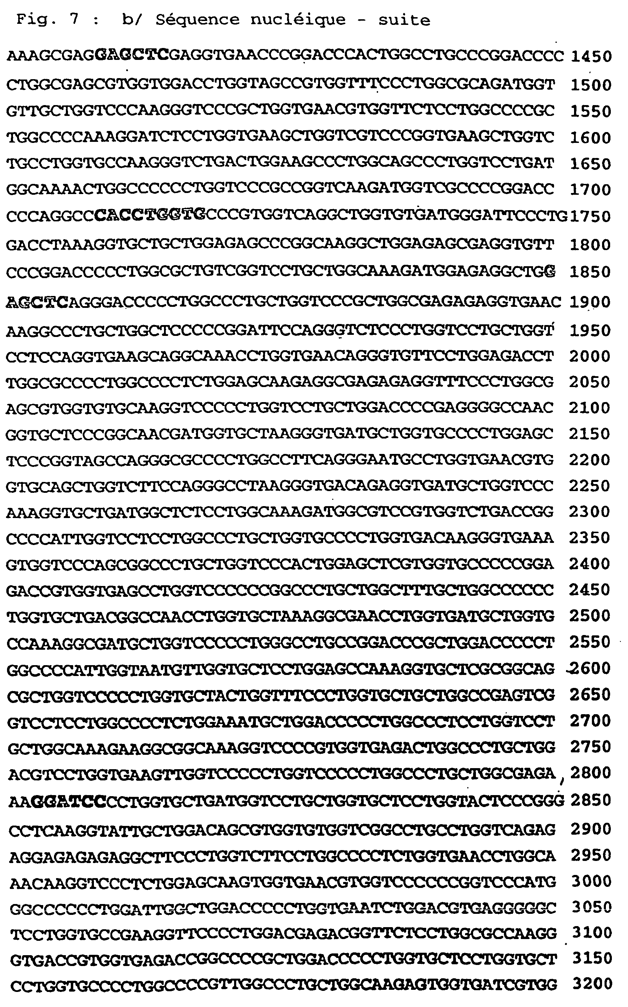

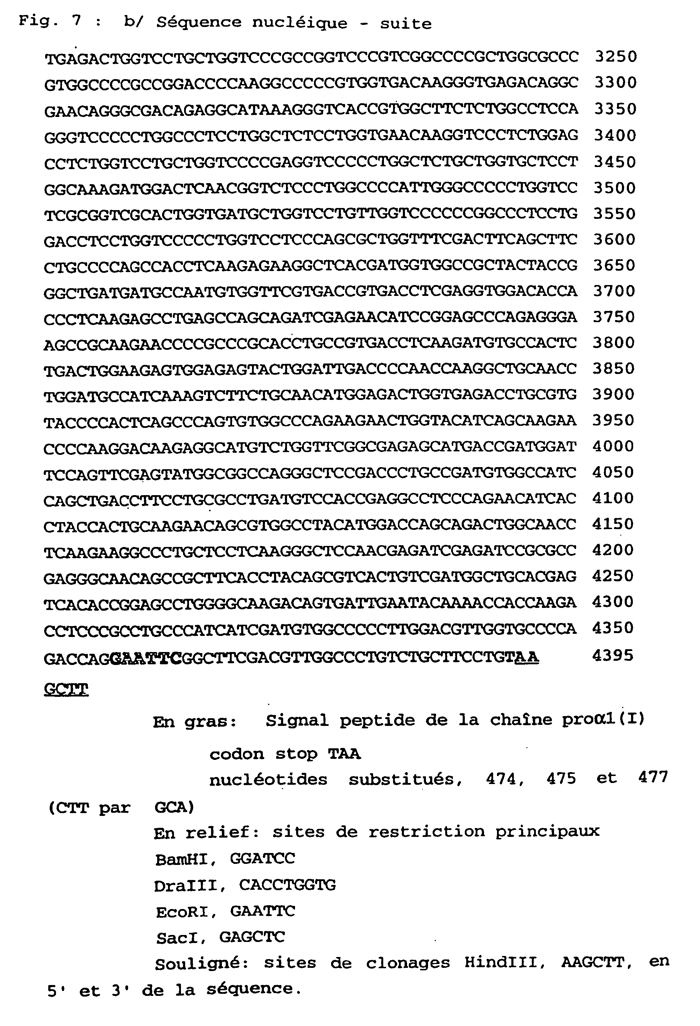

Figure 7 : Construction ADEFG, proα1(I) complet : séquence protéique et nucléique

### PREPARATION I : clonage d'ADNc codant pour la totalité de la chaîne proα1(I) humaine

### 1/ Préparation d'une banque ADNc MG-63

Les cellules MG-63 (N°ATCC-CRL 1427) proviennent d'un ostéosarcome humain. Environ 28 millions de cellules MG-63 cultivées en milieu DMEM (Dulbecco's modified Eagle medium, Sigma TM) sont décollées des boîtes de cultures par traitement à la trypsine 0,25% EDTA 0,05% (Ethylene diamine tetra acetic acid). Les cellules sont centrifugées à 1000 g pendant 10 min à 4°C, et le culot cellulaire est traité avec le kit RNA-PlusTM (BIOPROBE TM Systems) qui permet de purifier les ARN totaux. Cette manipulation a été réalisée en accord avec les recommandations fournies par BIOPROBE TM. Une quantité de 950 µg d'ARN totaux a ainsi été purifiée.

Les ARN poly(A)+ ont été séparés par chromatographie sur colonne oligo-dT cellulose (Boehringer Mannhein) en suivant la technique décrite par (Aviv et al 1972). Une suspension de soude 0,1 N contenant 0,2 g d'oligo dT cellulose est déposée dans une colonne. Cette colonne est alors lavée par 3 ml d'eau milli-Q stérile, puis par 15 ml d'un tampon porteur 1X (Tris-Cl 20 mM, pH 7,6; NaCl 0,5 M; EDTA 1 mM, pH 8; sodium lauryl sarcosinate 0,1%). L'effluent de la colonne ainsi équilibrée est alors à un pH inférieure à 8.

A une solution d'ARN totaux (950 µg) incubée 5 min à 65°C est ajouté un volume de tampon porteur 2X (Tris-C1 40 mM, pH 7,6; NaCl 1 M; EDTA 2 mM, pH 8; sodium lauryl sarcosinate 0,2%). Ce mélange est refroidi à température ambiante avant d'être déposé dans la colonne oligo dT cellulose. L'éluat est récupéré, incubé 5 min à 65 °C, refroidi à température ambiante et repassé dans la colonne oligo-dT cellulose. La colonne est alors lavée par 10 ml de tampon porteur 1X (Tris-Cl 20 mM, pH 7,6; NaCl 0,5 M; EDTA 1 mM, pH 8; sodium lauryl sarcosinate 0,1%), ce qui permet l'élution des ARN poly(A)-, les ARN poly(A)+ restant fixés à la matrice dT cellulose par leur extrémité 3' poly-adénylée. A la colonne oligo dT-ARN poly(A)+ sont alors ajoutés 2 ml de tampon de décrochage (Tris-Cl 10 mM, pH 7,6; EDTA 1 mM, pH 8; Lauryl sulfate 0,05%), et l'éluat d'ARN poly(A)+ est ajusté à 0,3 M en acétate de sodium pH 5,2. A cette solution sont apportés 2,2 volumes d'éthanol 100, et les ARN poly(A)+ sont précipités pendant 24 heures à - 20°C.

Après précipitation, la solution est centrifugée à 12000 g pendant 30 min à 4°C. Le culot d'ARN poly(A)+ est lavé par 10 ml d'éthanol 70, lyophilisé et repris par 50 µl d'eau milli-Q stérile. La concentration de cette fraction d'ARN poly(A)+ est de 1,2 µg/µl.

Pour réaliser la banque ADNc, nous avons utilisé des kits commercialisés par Amersham. Ce sont les kits:
- cDNA synthesis system plus, RPN 1256
- cDNA rapid adaptator ligation module, RPN 1712
- cDNA rapid cloning module, RPN 1716
- lambda-DNA *in vitro* packaging module, RPN 1717

Chaque étape est réalisée en suivant le protocole fourni par la fabricant.

La solution d'ARN poly(A)+ est incubée 5 min à 65°C, puis refroidie dans la glace afin d'éliminer les structures secondaires présentes dans certains ARN. Cette solution est divisée en deux fractions, et après appariement soit d'amorce dT (12-18 mer), soit d'amorces aléatoires (hexanucléotides) avec les ARN poly(A)+, la synthèse du premier brin ADN (complémentaire à la matrice ARN) va être réalisée par la réverse transcriptase. Au mélange hybride ARN/ADN est ajouté la ribonucléase H et l'ADN polymérase I d'*Escherichia coli*. La ribonucléase H permet de couper aléatoirement le brin ARN dans un complexe ARN/ADN. L'ADN polymérase ajoute des nucléotides aux extrémités 3'-OH libres créées par la ribonucléase H, grâce à son activité polymérase 5'-3'. L'activité exonucléase 5'-3' de l'ADN polymérase I permet d'éliminer le ribonucléotide en 5' et de poursuivre la synthèse du second brin ADNc. Après inactivation des enzymes par traitement à 70°C pendant 10 min, l'addition de T4 DNA polymérase permet d'obtenir des ADN double brin à extrémités franches grâce à son activité exonucléase 3'-5'. Des adaptateurs EcoRI sont alors ligués aux extrémités franches des ADNc (ADN complémentaires) . Les adaptateurs libres sont éliminés sur une colonne fournie par Amersham TM , et les ADNc adaptés en EcoRI sont ligués dans le site EcoRI du vecteur lambda MOSElox. Après ligation, une encapsidation *in vitro* est réalisée, ce qui est la dernière étape avant l'obtention de la banque ADNc. Environ 800000 clones ADNc indépendants ont ainsi été obtenus.

### 2/ clonage d' ADNc codant pour la chaîne proα1(I) humaine.

### a/Préparation de bactéries ER1647 aptes a être infectées par les phages

Cette souche bactérienne est recD-, mrc A-(Amersham TM). Ces bactéries sont maintenues dans des boîtes de L-broth/agar 1,5% (L-broth: pour 1 litre, 10 g de tryptone, 5 g d'extraits de levure, 10 g de NaCl) supplémentée en tétracycline (50 µg/ml). Une colonie ER1647 est ensemencée dans 10 ml de L-broth/tétracycline (50 µg/ml), et la culture est réalisée sous agitation à 37°C pendant 8 heures. La culture ER1647 est ensuite centrifugée à 4°C pendant 10 min à 3000 g, et le culot bactérien est resuspendu dans 2 ml de MgSO4 10 mM froid.

### b/ préparation de répliques de nitrocellulose

Pour le criblage, 4 boîtes (12X12 cm) L-broth/agar 1,5% de 25000 clones ADNc indépendants ont été réalisées. Pour cela, à 250 µl de bactéries ER1647 traitées au MgSO4 10 mM, ont été apportés 25000 phages de la banque ADNc. L'ensemble est incubé à 37°C pendant 15 min, temps nécessaire à une infection efficace des bactéries. Après apport de 7 ml de L-broth/agarose medium 0,7% (Sigma TM) maintenu à l'état liquide à 45°C, l'ensemble est déposé sur les boîtes de culture (12X12 cm).

Ces boîtes sont incubées à 37°C pendant 7-8 heures, temps nécessaire à l'apparition des plages de lyse. Elles sont alors utilisables pour le transfert de l'ADN des plages de lyse sur des répliques de nitrocellulose. La nitrocellulose utilisée est Hybond-C d'Amersham TM. Pour chaque boîte, deux répliques sont réalisées par simple contact plages de lyses/réplique de nitrocellulose. Le temps de contact est de 2 min pour la première réplique, et de 4 min pour la seconde. L'ADN de phages présents sur les répliques est dénaturé pendant 4 min dans une solution de soude 0,5 M, NaCl 1,5 M. La soude est alors neutralisée par deux bains successifs de 3 min dans une solution NaCl 1,5 M, Tris-Cl 0,5 M pH 7. Les répliques de nitrocellulose sont rincées dans une solution de 2X SSC (NaCl 0,3 M, citrate trisodique 30 mM), séchées sur papier Whatman 3MM, et l'ADN dénaturé de phages est fixé sur les répliques de nitrocellulose par à une incubation de 2 heures à 80°C.

### c/ préparation de sondes spécifiques du collagène de type I humain

Deux oligonucléotides de synthèse ont été utilisés pour obtenir des sondes spécifiques de la chaîne proα1(I).

Oligonucléotides BIOC5, 5'-CTCGGGTTTCCACACGT-3', séquence complémentaire aux nucléotides 152 à 178 spécifique de la chaîne proα1(I).

Oligonucléotide BIOC7, 5'-GCAAGACAGTGATTGAA-3', séquence 4268 à 4274 spécifique de la chaîne pro[α1(I).

Ces oligonucléotides ont été marqué au 32P, en utilisant du gamma P32ATP à 3000 Ci/mmol (Amersham TM) et de la T4 polynucléotide Kinase (Promega).

| | | |
|---|---|---|
| Conditions de marquage, | 50 ng BIOC5 ou BIOC7 | 10 µl |
| | H20 milli-Q | 4,5 µl |
| | gamma32PATP, 50 µCI | 5 µl |
| | T4 Polynucléotide kinase (10 u/µl; Promega TM) | 0,5 µl |

Ce mélange est incubé pendant 30 min à 37°C. La réaction est stoppée par ajout de 1 µl EDTA 0,5 M, pH 8.

### d/ hybridation des répliques de nitrocellulose

Les répliques de nitrocellulose sont déposées dans 50 ml d'une solution de préhybridation, et incubées à 50°C pendant 15 min.

| Solution de préhybridation: |
|---|
| 6XSSC (NaCl 0,3 M, citrate trisodique 90 mM) |
| Lauryl sulfate 0,1% |
| Sodium pyrophosphate 0,05% |
| polyvinypyrolidone 0,02% |
| albumine sérique bovine 0,02% |
| Ficoll 400 0,02% |
| ADN dénaturé de sperme de saumon, |
| à 100 µg/ml (Boehringer Mannhein) |

Les deux sondes BIOC5 et BIOC7 sont alors ajoutées, et l'hybridation est réalisée pendant 2 heures à 45°C. Après hybridation, les répliques de nitrocellulose subissent trois bains successifs de 15 min afin d'éliminer les hybridations aspécifiques.

Premier bain: 6XSSC (NaCl 0,9 M, citrate trisodique 90 mM), sodium pyrophosphate 0,05%, 15 min à température ambiante.

Second bain: 3XSSC (NaCl 0,45 M, citrate trisodique 45 mM), sodium pyrophosphate 0,05%, 15 min à température ambiante.

Troisième bain: 6XSSC (NaCl 0,9 M, citrate trisodique 90 mM), sodium pyrophosphate 0,05%, 15 min à 45°C.

Les filtres sont alors déposés sur un plastique, entouré d'un film de plastique transparent et soumis à autoradiographie à -80°C pendant 24 heures en présence d'un film.

Cette manipulation nous a permis d'obtenir plusieurs clones positifs en duplica. Chaque plage de lyse positive dans les deux répliques est récupérée à l'aide d'une pipette pasteur, et déposée dans 1 ml de tampon SM (NaCl 100mM, MgSO4 8 mM, Tris-Cl 50 mM pH 7,5, gélatine 0,01%).

Un second criblage nous a permis de purifier les différents clones positifs, les conditions d'hybridation et de lavages des répliques de nitrocellulose étant les mêmes que pour le premier criblage. Les clones purifiés lors de ce second criblage sont prélevés et déposés dans 500 µl de SM.

### e/ Sous clonage automatique des clones ADNc en plasmide par le système cre-lox.

Le vecteur bactériophagique lambda MOSElox comporte un plasmide interne flanqué à ses deux extrémités par les sites lox de 34 pb, dérivés du bactériophage P1. Ainsi, des sous-clones plasmidiens peuvent être obtenus en infectant une souche bactérienne exprimant la cre recombinase P1, qui reconnaît les séquences lox entourant le plasmide interne au bactériophage lambda MOSElox et qui permettra l'excision du plasmide par recombinaison spécifique au niveau des séquences lox.

Dans notre cas, nous avons utilisé la souche bactérienne BM25.8 qui est lysogène pour P1 et lambda-imm434kan, ces deux plasmides étant maintenus en ajoutant au milieu de croissance de la souche BMB25.8, de la kanamycine (50µg/ml) et du chloramphénicol (50 µg/ml).

En pratique, 10 ml de L-broth complémentés en kanamycine et chloramphénicol (50 µg/ml) sont ensemencés avec les bactéries BM25.8 et incubés à 37°C sous agitation. Lorsque la DO600 de cette culture est de 0,9, l'incubation est stoppée et les bactéries peuvent être conservées pendant 3 jours à 4°C.

Une fraction aliquote de la suspension phagique des clones ADNc positifs (10 µl) sont mis en contact avec 200 µl de bactéries BM25.8, et le mélange incubé à 37°C pendant 15 min afin d'obtenir une infection efficace. Le mélange est alors déposé dans une boîte L-Broth/agar 1,5% supplémentée en ampicilline (50 µg/ml), et les boîtes sont incubées à 37°C pendant 12 heures. La présence d'ampicilline permet de sélectionner les bactéries ayant intégré un bactériophage, et plus spécialement celles où s'est produite la recombinaison spécifique cre-lox, le plasmide résultant possédant un gène de résistance à l'ampicilline, et l'insert ADNc au niveau du site de clonage EcoRI.

### f/ caractérisation des clones ADNc alpha22 et lalpha3

Deux des clones ADN complémentaires positifs ont été utilisés ultérieurement. Ce sont les clones ADNc alpha 22 et lalpha3.

En vue de séquencer ces clones, 3 ml de L-broth supplémenté en ampicilline (50 µg/ml) ont été ensemencés soit par alpha22, soit par 1alpha3. Ces mélanges ont été incubés sous agitation à 37°C pendant une nuit.

Pour purifier l'ADN plasmidien, la culture est tout d'abord centrifugée à 1500 g pendant 10 min à 4°C, et le culot bactérien est traité avec le kit Wizard (Promega TM), qui permet de purifier de l'ADN plasmidien super enroulé. Cette manipulation est réalisée suivant le protocole fourni par le fabricant. Environ 10 µg d'ADN plasmidien est purifié grâce à ce kit, lorsque le plasmide utilisé est le plasmide MOSElox.

La dernière étape est de séquencer les deux extrémités des clones ADNc alpha22 et 1alpha3, afin de déterminer quelles informations ils comportent.

Pour réaliser 2 réactions de séquence, 40 µl d'ADN, soit 7-8 µg, purifié par le système Wizard sont dénaturés par ajout de 10 µl de NaOH 2N et incubés 10 min à température ambiante. Après neutralisation par apport de 12 µl d'acétate de sodium 3 M pH 5,2, l'ADN monocaténaire est précipité par apport de 2,5 volume d'éthanol 100 et incubation à -20°C pendant 30 min. Après précipitation, l'ADN est centrifugé à 4°C pendant 10 min à 12 000 g, lavé par 1 ml d'éthanol 70, lyophilisé et repris par 20 µl d'eau milli-Q.

Les réactions de séquence ont été réalisées avec le kit "T7 sequencing" (Pharmacia) qui utilise la technique de terminaison de chaînes et la T7 DNA polymerase. Le radio-élément est le [α-35S]-dATP (Amersham TM) à 1350 Ci/mmol. Les réactions de séquences ont été réalisées en suivant les recommandation du fabricant.

Principe. Après appariement d'une amorce sur une matrice d'ADN monocaténaire, une réaction d'élongation est réalisée par la T7 DNA polymérase en présence de dGTP, dTTP, dCTP et de 35-SdATP. L'apport d'un excès des 4 dNTP froids, ainsi que du ddATP (dideoxyadenosine triphosphate) ou du ddGTP ou du ddCTP ou du ddTTP, permet de réaliser séparément 4 réactions de terminaison. Lors de l'incorporation d'un ddNTP, l'absence du groupement hydroxyl bloque la réaction d'élongation.

Dans notre cas, les amorces utilisées sont les oligonucléotides T7 gene 10 et T7 terminator (Amersham TM) qui se situent de part et d'autre des clones ADNc alpha22 et 1alpha3..

T7 gene primer, 5'- TGAGGTTGTAGAAGTTCCG-3'

T7 terminator primer, 5'- GCTAGTTATTGCTCAGCGG-3'

L'analyse des séquences est réalisée sur gel polyacrylamide-urée (6%-7M).

### Gel de séquence

La solution de départ est: 39,6 g d'urée, 8,25 ml de TBE 10X (Tris borate 0,9 M, EDTA 20 mM, pH 8), 12,32 ml d'acrylamide 40% (38 g d'acrylamide, 2 g de bis-acrylamide, qsp 82,5 ml eau milli-Q).

Après dissolution de l'urée, 400 µl de persulfate d'ammonium 10% et 66 µl de TEMED (N, N, N', N'-tétraméthyl-éthylène diamine) sont ajoutés et le gel est coulé. Après polymérisation, le gel est préchauffé pendant 30 min à 60 kW. Les échantillons sont alors déposés et soumis à séparation sous une puissance de 60 kW. Après électrophorèse, le gel est transféré sur du papier Whatman 3MM, séché pendant 30 min sous vide à 80°C, et soumis à autoradiographie directe à température ambiante avec un La lecture des séquences nous a permis de confirmer que les clones ADNc alpha22 et 1alpha3 codent pour la chaîne proα1(I) humaine.

Plus précisément, le clone ADNc 1alpha 3 comporte 83 pb de la partie 5' non traduite et les 1920 premières bases codant pour la chaîne proα1(I) humaine. Le clone ADNc Alpha 22 comporté la séquence codant pour les acides aminés 171 à 1454 de la chaîne proα1(I) humaine et environ 500 pb de la région 3' non-traduite.

La conformité du gène obtenu est vérifiée par rapport aux séquences décrites (LI S-W, 1994)

### PREPARATION II : clonage de fragments d'ADN nécessaires à la réalisation des constructions selon l'invention.

### 1/ Fragment A

Le fragment A possède 4 nucléotides (-4 à -1) en amont du codon initiateur de traduction ATG, la totalité de la séquence codant pour le signal peptide (nucléotides 1 à 66) et pour le domaine amino-propeptide (nucléotides 67 à 479) de la chaîne proα1(I) humaine. Les nucléotides 474, 475 et 477, CTT, sont substitués par les bases GCA, ce qui permet la création d'un site de restriction NheI (GCTAGC) de la position 474 à 479. Cette création du site NheI entraîne la substitution des acides aminés Asn et Phe par une lysine et une leucine en position 158 et 159 de la chaîne proα1(I) humaine.

### Amplification du fragment A

Deux oligonucléotides de synthèse ont été utilisés.

Oligonucléotide sens, BIOC85,

5'TATCCGCGGAAGCTTAGACATGTTCAGCTTTGTGGACCTCCGGCTC
CTGC-3'

Cet oligonucléotide comporte en 5' (nucléotides soulignés) les séquences permettant la création de deux sites de restriction, SacII (CCGCGG) et HindIII (AAGCTT) qui seront utilisés comme sites de clonage. A la suite de ces deux sites sont inclus les nucléotides -4 à -1 et 1 à 31 spécifiques de la séquence codante de la chaîne proα(I) humaine.

Oligonucléotide antisens, BIOC83,

5'TATTCTAGAGCTAGCTTTCCTCCGAGGCCAGGGGTCCGGGAGGT-3'

Cet oligonucléotide présente en 5' (nucléotides soulignés) les séquences permettant la création des sites de restriction XbaI (TCTAGA) et NheI (GCTAGC). Le site XbaI sera utilisé pour le clonage du fragment A, alors que le site NheI, décrit dans la partie information, sert à délimiter la séquence codant pour le domaine amino-propeptide. Suite à ces deux sites est retrouvée la séquence complémentaire aux nucléotides 445 à 474 de la séquence codante de la chaîne proal(I) humaine.

La matrice ADN utilisée est le clone ADNc 1alpha3 qui comporte 83 pb de la partie 5' non traduite et les 1920 premières bases codant pour la chaîne proα1(I) humaine.

Pour obtenir le fragment A, les conditions d'amplification par PCR (Polymerase Chain Reaction) ont été:

| | | |
|---|---|---|
| 25 cycles | Dénaturation, | 30s à 94°C |
| | Appariement, | 30s à 55°C |
| | Elongation, | 30s à 72°C. |

A la fin des 25 cycles, une extension supplémentaire de 5 min à 72°C est réalisée.

| Solution de départ: |
|---|
| 75 mM Tris-Cl pH 9,0 (à 25°C) |
| 20 mM (NH4)2SO4 |
| 0,01% (Poids : volume) Tween 20 |
| 1,5 mM MgCl2 |
| BIOC85, 0,5 µM |
| BIOC83, 0,5 µM |
| chaque dNTP, 0,2 mM |
| matrice, 1 ng du clone ADNc 1alpha3 |
| 0,25 unité de DNA polymerase) |

### Clonage du fragment A

Après amplification, la solution est extraite par un volume de phénol/chloroforme/alcool isoamylique (rapport 50/48/2). Après deux minutes de centrifugation à 10 000 g, la phase aqueuse est reprise et extraite par un volume de chloroforme/alcool isoamylique (rapport 24/1). Après une centrifugation de quelques secondes à 10 000 g, la phase aqueuse est reprise, ajustée à une concentration en NaCl de 0,2 M et deux volumes d'alcool 100 sont ajoutés. L'ADN est précipité pendant 2 heures à -20°C, puis est centrifugé à 4°C pendant 10 min à 12 000 g. Le culot d'ADN est lavé avec 1 ml d'éthanol 70, lyophilisé, et resuspendu dans 34 µl d'eau milli-Q stérile.

A cette solution d'ADN sont ajoutés 4 µl de tampon de digestion C (Promega TM), 1 µl de l'endonucléase de restriction SacII (10 u/µl; Promega TM) et 1 µl d'enzyme XbaI (12 u/µl; Promega). La digestion est réalisée pendant 2 heures à 37°C.

Suite à la digestion enzymatique, 8 µl de tampon stop (30% sucrose, 0,25% bleu de bromophénol) sont ajoutés à la solution d'ADN qui est analysée par électrophorèse sur gel d'agarose 2% à bas point de fusion Nu Sieve-GTG (FMC) à un voltage constant de 60 volts. Le tampon d'électophorèse est du TBE 0,5X (Tris borate 45 mM, EDTA (Acide éthylène-diamine-tétra-acétique) 1mM, pH 8). Après migration, le gel est coloré pendant 15 min dans une solution de TBE 0,5X contenant 40 ng/ml de bromure d'ethidium.

La bande correspondant au fragment A est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) et centrifugée pendant 2 min à 10 000 g. La phase aqueuse est extraite au chloroforme/alcool isoamylique (rapport 24/1) et centrifugée quelques secondes à 10 000 g. La phase aqueuse est reprise, ajustée à une concentration finale en NaCl de 0,2 M, et précipitée 16 heures à -20°C après apport de deux volumes d'éthanol 100.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega TM) sont analysés sur gel d'agarose type II (Sigma TM) 2%, ce qui nous a permis d'estimer la concentration de la solution purifiée du fragment A SacII/XbaI à 30 ng/µl.

Le plasmide utilisé pour cloner le fragment A est le vecteur pBluescript II SK(+) commercialisé par Stratagene TM . Ce plasmide comporte un gène de résistance à l'ampicilline.

Une quantité de 200 ng du plasmide pBluescript II SK(+) est soumise à une digestion par les endonucléases de restriction SacII et XbaI. Cette digestion est réalisée dans un volume de 20 µl en présence de 2 µl de tampon C (Promega TM), 1 µl d'enzyme SacII (10 u/µl; Promega TM) et 1 µl d'enzyme XbaI (12 u/µl; Promega TM), pendant 2 heures à 37°C. Après digestion, 2 µl de tampon porteur (30% sucrose, 0,25% bleu de bromophénol) sont ajoutés au mélange et analysé sur gel d'agarose Seaplaque GTG (FMC, Rockland) 0,8% à un voltage constant de 60 volts, le tampon d'électrophorèse étant du TBE 0,5X.Après électrophorèse, le gel est coloré pendant 15 min dans une solution de bromure d'ethidium à 40 ng/ml. La bande d'ADN correspondant au pBluescript II SK(+) linéarisé par SacII et XbaI est prélevée et déposée dans 350 µl d'une solution de Tris 20mM pH 8, EDTA 1 mM pH 8. Cet échantillon est incubé pendant 5 min à 65°C, extrait par un volume de phénol et centrifugé pendant 5 min à 10 000 g. La phase aqueuse est reprise, extraite par un volume de phénol/chloroforme/ alcool isoamylique (rapport 50/48/2) et centrifugée pendant 2 min à 10 000 g. La phase aqueuse est extraite au chloroforme/alcool isoamylique (rapport 24/1) et centrifugée quelques secondes à 10 000 g. La phase aqueuse est reprise, ajustée à une concentration finale en NaCl de 0,2 M, et précipitée 16 heures à -20°C après apport de deux volumes d'éthanol 100.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 10 ng/µl du plasmide pBluescript II SK(+) SacII/XbaI.

La ligation du fragment A (SacII/XbaI) au plasmide pBluescript II SK(+) (SacII/XbaI) est réalisée pendant 4 heures à température ambiante.

Pour la transformation, les bactéries compétentes utilisées sont les DH5 alpha (Gibco BRL, Paris). Le génotype de cette souche est: supE 44 Δ-lacU 169(∅80 lacZΔM15) hsdR 17 recA 1 endA 1 gyrA 96 thi- 1 relA 1. Pour obtenir des bactéries compétentes, 5 ml d'une culture en phase exponentielle (DO600 de 0.7) sont centrifugés à 1500 g pendant 15 min à 4°C. Le culot bactérien est alors resuspendu dans 500 µl de CaCl2 100 mM froid. Pour la transformation, 10 µl du mélange de ligation est mélangé à 200 µl de bactéries DH5 alpha compétentes et l'échantillon est placé à 4°C pendant 30 min. Après un choc thermique à 42°C pendant 40 s, 500 µl de L-broth (10 g de tryptone, 5 g d'extrait de levure, 10 g de NaCl, par litre de milieu) sont ajoutés, et le mélange est incubé pendant 30 min à 37°C.

Ce mélange ainsi que 20 µl d'IPTG (Isopropylthyo-beta-D-galactoside; 200mM) et 20 µl de X-Gal (4 chloro-3 indolyl-beta-D galactoside; 20 mg/ml) sont ensuite déposés sur une boîte de L-Broth-agar (L-Broth plus agar 1.5%) supplémentée en ampicilline (50 µg/ml), et la boîte est incubée pendant 15 heures à 37°C. L'apport d'IPTG et de X-Gal permet l'utilisation du test de coloration qui permet de reconnaître les plasmides ayant incorporé un insert.

En effet, la souche bactérienne DH5 alpha est Lac-, et produit une forme non fonctionnelle de la beta-galactosidase. Le vecteur pBluescript II SK(+) possède un segment d'ADN de l'opéron lactose *d'Escherichia coli* qui code pour la partie amino-terminale de cette enzyme. L'induction de synthèse par l'IPTG permet une complémentation alpha qui restaure l'activité de cette galactosidase. En présence d'un analogue du galactose, le X-Gal, les bactéries produisent des pigments bleus qui entraînent la formation d'une colonie bactérienne colorée. Le vecteur pBluescript II SK(+) possède une région de multiples clonages (comprenant les sites SacII/XbaI) située dans le gène LacZ. L'insertion du fragment A au sein de cette région de clonage, abolit la complémentation alpha, ce qui entraîne l'apparition de colonies bactériennes blanches.

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega), qui permet de purifier de l'ADN plasmidien super enroulé. Cette manipulation est réalisée suivant le protocole fourni par le fabricant. Environ 10 µg d'ADN plasmidien est purifié grâce à ce kit, lorsque le plasmide utilisé est le pBluescript II SK(+).

Une fraction aliquote (2 µl sur les 60 obtenus) est soumise à une digestion enzymatique par les endonucléases de restriction SacII et XbaI, afin de vérifier le clonage du fragment A. Après électrophorèse, le gel est coloré pendant 15 min dans une solution de bromure d'éthidium à 40 ng/ml, et la présence du fragment A est vérifiée sous UV.

La dernière étape est de vérifier l'intégrité de la séquence du fragment A, la technique d'amplification par PCR (polymerase chain reaction) pouvant entraîner des mutations.

Les réactions de séquence réalisées avec le kit "T7 sequencing" (Pharmacia TM) qui utilise la technique de terminaison de chaînes et la T7 DNA polymerase (cf PREPARATION 1) permet de valider l'intégrité de la séquence du fragment A.

### 2/ Fragment B

Le fragment B possède 3 bases (TAA) en amont de la séquence codant pour le signal peptide PRS (PATHOGENESIS-RELATED PROTEIN S) (nucléotides 1 à 75), et les bases 67 à 77 de la séquence codant pour la partie amino-terminale du domaine amino-propeptide de la chaîne proα1(I) humaine. Les nucléotides 73, 74 et 77, GAG, sont substitués par les bases CTC, ce qui permet la création d'un site de restriction NheI (GCTAGC) de la position 72 à 77. Cette création du site NheI entraîne la substitution des acides aminés Glu et Gly par une leucine et une alanine en position 25 et 26 de la chaîne proα1(I) humaine.

### Amplification du fragment B

Trois oligonucléotides de synthèse ( BIOC95 et BIOC93 et 045) ont été utilisés.

Oligonucléotide matrice, 045,

5'-TAAATGAACTTCCTCAAAAGTTTCCCCTTTTATGCCTTCCTT
TGTTTTGGCCAATACTTTGTAGCTGTTACTCATGCTGCC-3'

Cet oligonucléotide comporte en gras la séquence correspondant à celle du peptide signal de PRS (PATHOGENESIS-RELATED PROTEIN S). Il servira de matrice ADN lors de l'amplification par PCR (Polymerase Chain Reaction) du fragment B

Oligonucléotide sens, BIOC95,

5'-TATCCGCGGAAGCTTTAAATGAACTTCCTCAAAAGTTTCCCC-3'

Cet oligonucléotide comporte en 5' (nucléotides soulignés) les séquences permettant la création de deux sites de restriction, SacII (CCGCGG) et HindIII (AAGCTT) qui seront utilisés comme sites de clonage. A la suite de ces deux sites sont inclus les nucléotides -3 à -1 et 1 à 24 spécifiques de la séquence codant pour le signal peptide de PRS (PATHOGENIC-RELATED PROTEIN S).

Oligonucléotide antisens, BIOC93,

5'- ATGCTAGCTCTTGAGCATGAGTAACAGCTACAAAGTA-3'

Cet oligonucléotide présente en 5' (nucléotides soulignés) la séquence permettant la création du site de restriction NheI (GCTAGC). Le site NheI, décrit dans la partie information, sert à délimiter la séquence codant pour le domaine amino-propeptide. Après le site NheI est retrouvée la séquence complémentaire aux nucléotides 67 à 71 de la séquence codante de la chaîne proα1(I) humaine et 52 à 75 de la séquence codant pour le signal peptide de PRS (PATHOGENESIS-RELATED PROTEIN S).

### Clonage du fragment B

Après l'amplification PCR, la solution est extraite par un volume de phénol/chloroforme/alcool isoamylique (rapport 50/48/2), comme décrit dans préparation II1. Le culot d'ADN est lavé avec 1 ml d'éthanol 70, lyophilisé, et resuspendu dans 40 µl d'eau milli-Q stérile.

Une fraction aliquote de 4 µl, ainsi que 500 ng des fragments HindIII-EcoRI du phage lambda (Promega TM) sont analysés sur gel d'agarose type II (Sigma TM) 2%, ce qui nous a permis d'estimer la concentration de la solution purifiée du fragment B à 20 ng/µl.

Le plasmide utilisé pour cloner le fragment B est le vecteur pBluescript II SK(+) commercialisé par Stratagene. Ce plasmide comporte un gène de résistance à l'ampicilline.

Une quantité de 200 ng du plasmide pBluescript II SK(+) est soumise à une digestion par l'endonucléase de restriction EcoRV (GATATC), qui permet d'obtenir un vecteur linéarisé à extrémités franches. Après digestion, 80 µl de TE (Tris 10 mM, EDTA 1 mM, pH 8) sont ajoutés et la solution est extraite par un volume de phénol/chloroforme/ alcool isoamylique (rapport 50/48/2) comme décrit dans préparation II1.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 10 ng/µl du plasmide pBluescript II SK(+) EcoRV.

La ligation du fragment B au plasmide pBluescript II SK(+) (EcoRV) est réalisée pendant 4 heures à température ambiante.

Pour la transformation, les bactéries compétentes utilisées sont les DH5 alpha (Gibco BRL, Paris) cf ci-dessus.

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega TM), suivant le protocole fourni par le fabricant.

Une fraction aliquote (2 µl sur les 60 obtenus) est soumise à une digestion enzymatique par les endonucléases de restriction BamHI et HindIII, afin de vérifier le clonage du fragment B. Les sites de restriction BamHI et HindIII sont localisés de part et d'autre du site de clonage EcoRV. Après électrophorèse, le gel est coloré pendant 15 min dans une solution de bromure d'éthidium à 40 ng/ml, et la présence du fragment B est vérifiée sous UV.

Les réactions de séquence réalisées avec le kit "T7 sequencing" (Pharmacia TM) qui utilise la technique de terminaison de chaînes et la T7 DNA polymerase (cf PREPARATION 1) permet de valider l'intégrité de la séquence du fragment B.

### 3/ Fragment C

Le fragment C possède la quasi totalité de la séquence codant pour le domaine amino-propeptide (nucléotides 72 à 479) de la chaîne proα1(I) humaine. Les nucléotides 73, 74 et 77, GAG, sont substitués par les bases CTC, ce qui permet la création d'un site de restriction NheI (GCTAGC) de la position 72 à 77. Cette création du site NheI entraîne la substitution des acides aminés Glu et Gly par une leucine et une alanine en position 25 et 26 de la chaîne proα1(I) humaine. Les nucléotides 474, 475 et 477, CTT, sont substitués par les bases GCA, ce qui permet la création d'un site de restriction NheI (GCTAGC) de la position 474 à 479. Cette création du site NheI entraîne la substitution des acides aminés Asn et Phe par une lysine et une leucine en position 158 et 159 de la chaîne proα1(I) humaine.

### Amplification du fragment C

Deux oligonucléotides de synthèse ont été utilisés.

Oligonucléotide sens, BIOC855,

5'- ATGCTAGCCCAAGTCGAGGGCCAAGACGAAG-3'

Cet oligonucléotide comporte en 5' (nucléotides soulignés) la séquence permettant la création d'un site de restriction NheI (GCTAGC) qui sera utilisé comme site de clonage. Le site NheI, décrit dans la partie information, sert à délimiter la séquence codant pour le domaine amino-propeptide (extrémité amino-terminale). A la suite de ce site sont inclus les nucléotides 78 à 100 spécifiques de la séquence codante de la chaîne proα1(I) humaine.

Oligonucléotide antisens, BIOC83,

5'TATTCTAGAGCTAGCTTTCCTCCGAGGCCAGGGGGTCCGGGA
GGT-3'

Cet oligonucléotide présente en 5' (nucléotides soulignés) les séquences permettant la création des sites de restriction XbaI (TCTAGA) et NheI (GCTAGC). Le site XbaI utilisé lors du clonage du fragment A, ne sera pas utilisé dans le cadre du fragment C. Le site NheI, décrit dans la partie information, sert à délimiter la séquence codant pour le domaine amino-propeptide (extrémité carboxy-terminale). Suite à ces deux sites est retrouvée la séquence complémentaire aux nucléotides 445 à 474 de la séquence codante de la chaîne proα1(I) humaine.

La matrice ADN utilisée est le clone ADNc lalpha3 qui comporte 83 pb de la partie 5' non traduite et les 1920 premières bases codant pour la chaîne proα1(I) humaine.

### Clonage du fragment C

Après amplification, la solution est extraite par un volume de phénol/chloroforme/alcool isoamylique (rapport 50/48/2) comme décrit dans préparation II1. Le culot d'ADN est lavé avec 1 ml d'éthanol 70, lyophilisé, et resuspendu dans 40 µl d'eau milli-Q stérile.

Une fraction aliquote de 4 µl, ainsi que 500 ng des fragments HindIII-EcoRI du phage lambda (Promega TM) sont analysés sur gel d'agarose type II (Sigma TM) 2%, ce qui nous a permis d'estimer la concentration de la solution purifiée du fragment C à 20 ng/µl.

Le plasmide utilisé pour cloner le fragment C est le vecteur pBluescript II SK(+) commercialisé par Stratagene TM (La jolla). Ce plasmide comporte un gène de résistance à l'ampicilline.

Une quantité de 200 ng du plasmide pBluescript II SK(+) est soumise à une digestion par l'endonucléase de restriction EcoRV (GATATC), qui permet d'obtenir un vecteur linéarisé à extrémités franches. Après digestion, 80 µl de TE (Tris 10 mM, EDTA 1 mM, pH 8) sont ajoutés et la solution est extraite par un volume de phénol/chloroforme/ alcool isoamylique (rapport 50/48/2) comme décrit dans PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 10 ng/µl du plasmide pBluescript II SK(+) EcoRV.

La ligation du fragment C au plasmide pBluescript II SK(+) (EcoRV) est réalisée pendant 4 heures à température ambiante.

Pour la transformation, les bactéries compétentes utilisées sont les DH5 alpha (Gibco BRL, Paris).

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega TM), suivant le protocole fourni par le fabricant.

Une fraction aliquote (2 µl sur les 60 obtenus) est soumise à une digestion enzymatique par les endonucléases de restriction BamHI et HindIII, afin de vérifier le clonage du fragment C. Les sites de restriction BamHI et HindIII sont localisés de part et d'autre du site de clonage EcoRV. Après électrophorèse, le gel est coloré pendant 15 min dans une solution de bromure d'éthidium à 40 ng/ml, et la présence du fragment C est vérifiée sous UV.

Les réactions de séquence réalisées avec le kit "T7 sequencing" (Pharmacia TM) qui utilise la technique de terminaison de chaînes et la T7 DNA polymerase (cf PREPARATION 1) permet de valider l'intégrité de la séquence du fragment C.

### 4/ Fragment D

Le fragment D comporte la totalité de la séquence codant pour le domaine amino-télopeptide (nucléotides 474 à 534) et la séquence codant pour la partie amino-terminale de la région hélicoïdale (nucléotides 535 à 1920) de la chaîne proα1(I) humaine. Les nucléotides 474, 475 et 477, CTT, sont substitués par les bases GCA, ce qui permet la création d'un site de restriction NheI (GCTAGC) de la position 474 à 479. Cette création du site NheI entraîne la substitution des acides aminés Asn et Phe par une lysine et une leucine en position 158 et 159 de la chaîne proα1(I) humaine. Un site de restriction DraIII est localisé de la position 1709 à 1717 (CACCTGGTG), alors qu'un site BamHI dû au plasmide lambda MoSElox (Amersham TM) est situé en extrémité 3'.

### Amplification du fragment D

Deux oligonucléotides de synthèse ont été utilisés.

Oligonucléotide sens, BIOC65,

5'- TATTCTAGAGCTAGCTCCCAGCTGTCTTATGGCTATGATGAG-
3'

Cet oligonucléotide présente en 5' (nucléotides soulignés) les séquences permettant la création des sites de restriction XbaI (TCTAGA) et NheI (GCTAGC). Le site XbaI sera utilisé pour le clonage du fragment D, alors que le site NheI, décrit dans la partie information, sert à délimiter la séquence codant pour le domaine amino-propeptide. Après ces deux sites est retrouvée la séquence ( nucléotides 480 à 507) codant pour le début du domaine amino-télopeptide de la chaîne proα1(I) humaine.

Oligonucléotide antisens, T7 gene 10 primer,

5'- CTGAGGTTGTAGAAGTTCCG-3'

Cet oligonucléotide est localisé en juste en aval de l'extrémité 3' du clone ADNc 1alpha3, et d'un site de restriction BamHI (GGATCC) qui sera utilisé pour le clonage du fragment D.

La matrice ADN utilisée est le clone ADNc 1alpha3 qui comporte 83 pb de la partie 5' non traduite et les 1920 premières bases codant pour la chaîne proα1(I) humaine.

### Clonage du fragment D

Après amplification, la solution est extraite par un volume de phénol/chloroforme/alcool isoamylique (rapport 50/48/2) comme dans PREPARATION II1. Le culot d'ADN est lavé avec 1 ml d'éthanol 70, lyophilisé, et resuspendu dans 34 µl d'eau milli-Q stérile.

La digestion par Bam H1 Xba1 est réalisée pendant 2 heures à 37°C dans un volume final de 40 µl.

Suite à la digestion enzymatique, 8 µl de tampon stop (30% sucrose, 0,25% bleu de bromophénol) sont ajoutés à la solution d'ADN qui est analysée par électrophorèse sur gel d'agarose 0,8% à bas point de fusion SeaPlaque-GTG (FMC, Rockland) à un voltage constant de 60 volts. Le tampon d'électophorèse est du TBE 0,5X (Tris borate 45 mM, EDTA (Acide éthylène-diamine-tétra-acétique) 1mM, pH 8). Après migration, le gel est coloré pendant 15 min dans une solution de TBE 0,5X contenant 40 ng/ml de bromure d'ethidium.

La bande correspondant au fragment D est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) comme décrit dans préparation II1.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega TM) sont analysés sur gel d'agarose type II (Sigma TM) 0,8%, ce qui nous a permis d'estimer la concentration de la solution purifiée du fragment D BamHI/XbaI à 50 ng/µl.

Le plasmide utilisé pour cloner le fragment D est le vecteur pUC 18 commercialisé par Promega (TM). Ce plasmide comporte un gène de résistance à l'ampicilline.

Une quantité de 200 ng du plasmide pUC 18 est soumise à une digestion par les endonucléases de restriction BamHI et XbaI. Pendant 2 heures à 37°C. Après digestion, 2 µl de tampon porteur (30% sucrose, 0,25% bleu de bromophénol) sont ajoutés au mélange et analysé sur gel d'agarose Seaplaque GTG (FMC, Rockland) 0,8%. La bande d'ADN correspondant au pUC18 linéarisé par BamHI et XbaI est prélevée et déposée dans 350 µl d'une solution de Tris 20mM pH 8, EDTA 1 mM pH 8. Cet échantillon est incubé pendant 5 min à 65°C, extrait par un volume de phénol et centrifugé pendant 5 min à 10 000 g. La phase aqueuse est reprise, extraite par un volume de phénol/chloroforme/alcool isoamylique (rapport 50/48/2)comme décrit dans PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 10 ng/µl du plasmide pUC18 BamHI/XbaI.

La ligation du fragment D (BamHI/XbaI) au plasmide pUC18 (BamHI/XbaI) est réalisée pendant 4 heures à température ambiante.

Pour la transformation, les bactéries compétentes utilisées sont les DH5 alpha (Gibco BRL, Paris).

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega TM), qui permet de purifier de l'ADN plasmidien super enroulé. Cette manipulation est réalisée suivant le protocole fourni par le fabricant. Environ 10 µg d'ADN plasmidien est purifié grâce à ce kit, lorsque le plasmide utilisé est le pUC18.

Une fraction aliquote (2 µl sur les 60 obtenus) est soumise à une digestion enzymatique par les endonucléases de restriction BamHI et XbaI, afin de vérifier le clonage du fragment D. Après électrophorèse, le gel est coloré pendant 15 min dans une solution de bromure d'éthidium à 40 ng/ml, et la présence du fragment D est vérifiée sous UV.

Les réactions de séquence réalisées avec le kit "T7 sequencing" (Pharmacia TM) qui utilise la technique de terminaison de chaînes et la T7 DNA polymerase (cf PREPARATION 1) permet de valider l'intégrité de la séquence du fragment D.

### 5/ fragment E

Le fragment E est compris entre les sites DraIII (CACCTGGTG, nucléotides 1709 à 1717) et BamHI (GGATCC, nucléotides 2803 à 2808) présents dans le clone ADNc alpha22. Ce fragment code pour les acides aminés 567 à 936 compris dans le domaine hélicoïdal central de la chaîne proα1(I) humaine.

### 6/ fragment F

Le fragment F est compris entre les sites BamHI (GGATCC, nucléotides 2803 à 2808) et EcoRI (GAATTC, nucléotides 4357 à 4362) présents dans le clone ADNc alpha22. Ce fragment code pour les acides aminés 936 à 1192 compris dans le domaine hélicoïdal central et 1193 à 1454 du domaine carboxy-propeptide de la chaîne proα1(I) humaine.

### 7/ Fragment G

Le fragment G comporte la séquence (nucléotides 4040 à 4392) codant pour la partie carboxy-terminale du domaine carboxy-propeptide (acides aminés 1346 à 1464) de la chaîne proα1(I) humaine. Ce fragment comporte aussi les nucléotides TAA (codon stop, bases 4393 à 4395) couplés à un site de restriction HinIII (AAGCTT).

### Amplification du fragment G

Deux oligonucléotides de synthèse ont été utilisés.

Oligonucléotide sens, BIOC25,

5'- TATCTGCAGATGTGGCCATCCAGCTGACCT-3'-3'

Cet oligonucléotide comporte en 5' (nucléotides soulignés) la séquence permettant la création du site de restriction PstI (CTGCAG) qui sera utilisé comme site de clonage. A la suite de ce site, sont inclus les nucléotides 4040 à 4060 spécifiques de la séquence codante de la chaîne proα1(I) humaine.

Oligonucléotide antisens, BIOC23,

5'- TATAAGCTTACAGGAAGCAGACAGGGCCAA-3'

Cet oligonucléotide présente en 5' (nucléotides soulignés) la séquence permettant la création du site de restriction HindIII (AAGCTT). En gras, est indiquée la séquence complémentaire du codon stop TAA, suivi du brin complémentaire des nucléotides 4373 à 4392 de la séquence codant pour la chaîne proal(I) humaine.

La matrice ADN utilisée est le clone ADNc alpha22 qui comporte la séquence codant pour les acides aminés 171 à 1454 de la chaîne proα1(I) humaine et environ 500 pb de la région 3' non-traduite.

### Clonage du fragment G

Après amplification, la solution est extraite par un volume de phénol/chloroforme/alcool isoamylique (rapport 50/48/2). Le culot d'ADN est lavé avec 1 ml d'éthanol 70, lyophilisé, et resuspendu dans 35 µl d'eau milli-Q stérile.

La digestion par Pst1 est réalisée pendant 2 heures à 37°C dans le volume final de 40 µl.

Suite à la digestion enzymatique, 60 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) comme décrit dans PREPARATION II1.

Après centrifugation à 4°C pendant 10 min à 12 000 g, le culot d'ADN est lavé par 1 ml d'éthanol 70, lyophilisé et repris dans 35 µl d'eau milli-Q stérile. La digestion par Hind III est réalisée pendant 2 heures à 37°C dans un volume final de 20 µl.

Suite à la digestion enzymatique, 8 µl de tampon stop (30% sucrose, 0,25% bleu de bromophénol) sont ajoutés à la solution d'ADN qui est analysée par électrophorèse sur gel d'agarose 2% à bas point de fusion Nu Sieve-GTG (FMC, Rockland)cf. PREPARATION II2.

La bande correspondant au fragment G est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) comme décrit dans PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega TM) sont analysés sur gel d'agarose type II (Sigma TM) 2%, ce qui nous a permis d'estimer la concentration de la solution purifiée du fragment G HindIII/PstI à 30 ng/µl.

Le plasmide utilisé pour cloner le fragment G est le vecteur pBluescript II SK(+) commercialisé par Stratagene. Ce plasmide comporte un gène de résistance à l'ampicilline.

Une quantité de 200 ng du plasmide pBluescript II SK(+) est soumise à une digestion par l'endonucléase de restriction PstI., dans un volume final de 20 µl, pendant 2 heures à 37°C. Après digestion, 80 µl de tampon TE (tris 10 mM, EDTA 1 mM, pH 8) sont ajoutés à la solution d'ADN qui est extraite par un volume de phénol/chloroforme/alcool isoamylique (rapport 50/48/2)comme décrit dans préparation II1.

Après centrifugation à 4°C pendant 10 min à 12 000 g, le culot d'ADN est lavé par 1 ml d'éthanol 70, lyophilisé et repris dans 17 µl d'eau milli-Q stérile. La digestion par Hind III est réalisée pendant 2 heures à 37°C dans un volume final de 20 µl.

Après digestion, 4 µl de tampon porteur (30% sucrose, 0,25% bleu de bromophénol) sont ajoutés au mélange et analysé sur gel d'agarose Seaplaque GTG (FMC, Rockland) 0,8% cf PREPARATION II4. La bande d'ADN correspondant au pBluescript II SK(+) linéarisé par HindIII et PstI est prélevée et déposée dans 350 µl d'une solution de Tris 20mM pH 8, EDTA 1 mM pH 8. Cet échantillon est incubé pendant 5 min à 65°C, extrait par un volume de phénol et centrifugé pendant 5 min à 10 000 g. La phase aqueuse est reprise, extraite par un volume de phénol/chloroforme/ alcool isoamylique (rapport 50/48/2) comme décrit dans PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 10 ng/µl du plasmide pBluescript II SK(+) HindIII/PstI.

La ligation du fragment G (HindIII/PstI) au plasmide pBluescript II SK(+) (HindIII/PstI) est réalisée pendant 4 heures à température ambiante.

Pour la transformation, les bactéries compétentes utilisées sont les DH5alpha (Gibco BRL, Paris).

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega TM) suivant le protocole fourni par le fabricant.

Une fraction aliquote (2 µl sur les 60 obtenus) est soumise à une digestion enzymatique par les endonucléases de restriction HindIII et BamHI, afin de vérifier le clonage du fragment G.

Après électrophorèse, le gel est coloré pendant 15 min dans une solution de bromure d'éthidium à 40 ng/ml, et la présence du fragment G est vérifiée sous UV.

Les réactions de séquence réalisées avec le kit "T7 sequencing" (Pharmacia TM) qui utilise la technique de terminaison de chaînes et la T7 DNA polymerase (cf PREPARATION 1) permettent de valider l'intégrité de la séquence du fragment G.

### 8/ Fragment H

Le fragment H comporte la séquence (nucléotides 4031 à 4383) codant pour la partie carboxy-terminale du domaine carboxy-propeptide (acides aminés 1343 à 1461) de la chaîne proα1(I) humaine. Ce fragment comporte aussi les nucléotides TAA (codon stop, bases 4384 à 4386) couplés à un site de restriction HindIII (AAGCTT). En amont du codon stop sont inclus les codons Lys, Asp, Glu, Leu, site de rétention dans le réticulum endoplasmisque.

### b/ Amplification du fragment H

Deux oligonucléotides de synthèse ont été utilisés.

Oligonucléotide sens, BIOC25,

5'- TATCTGCAGATGTGGCCATCCAGCTGACCT-3'-3'

Cet oligonucléotide comporte en 5' (nucléotides soulignés) la séquence permettant la création du site de restriction PstI (CTGCAG) qui sera utilisé comme site de clonage. A la suite de ce site, sont inclus les nucléotides 4031 à 4051 spécifiques de la séquence codante de la chaîne proα1(I) humaine.

Oligonucléotide antisens, BIOCKDEL,

5'TATAAGCTTATAGCTCATCTTTCAGGAAGCAGACAGGGCCAA
CGTCGAAGC-3'

Cet oligonucléotide présente en 5' (nucléotides soulignés) la séquence permettant la création du site de restriction HindIII (AAGCTT). En gras, est indiquée la séquence complémentaire du codon stop TAA et des acides aminés KDEL, suivi du brin complémentaire des nucléotides 4353 à 4383 de la séquence codant pour la chaîne proα1(I) humaine.

La matrice ADN utilisée est le clone ADNc alpha22 qui comporte 110 pb de la partie 5' non traduite et les 1911 premières bases codant pour la chaîne proα1(I) humaine.

### Clonage du fragment H

Après amplification, la solution est extraite par un volume de phénol/chloroforme/alcool isoamylique (rapport 50/48/2) comme décrit dans PREPARATION II1. Le culot d'ADN est lavé avec 1 ml d'éthanol 70, lyophilisé, et resuspendu dans 35 µl d'eau milli-Q stérile.

La digestion par Pst1 est réalisée pendant 2 heures à 37°C dans un volume final de 40 µl.

Suite à la digestion enzymatique, 60 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) comme décrit dans PREPARATION II1.

Après centrifugation à 4°C pendant 10 min à 12 000 g, le culot d'ADN est lavé par 1 ml d'éthanol 70, lyophilisé et repris dans 35 µl d'eau milli-Q stérile. La digestion par HIND III est réalisée pendant 2 heures à 37°C dans un volume final de 40 µl.

Suite à la digestion enzymatique, 8 µl de tampon stop (30% sucrose, 0,25% bleu de bromophénol) sont ajoutés à la solution d'ADN qui est analysée par électrophorèse sur gel d'agarose 2% à bas point de fusion Nu Sieve-GTG (FMC, Rockland) comme décrit dans PREPARATION II1.

La bande correspondant au fragment H est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) comme décrit dans PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega TM) sont analysés sur gel d'agarose type II (Sigma TM) 2%, ce qui nous a permis d'estimer la concentration de la solution purifiée du fragment H HindIII/PstI à 20 ng/µl.

Le plasmide utilisé pour cloner le fragment H est le vecteur pBluescript II SK(+) commercialisé par Stratagene TM. Ce plasmide comporte un gène de résistance à l'ampicilline.

Une quantité de 200 ng du plasmide pBluescript II SK(+) est soumise à une digestion par l'endonucléase de restriction PstI. Dans un volume de 20 µl pendant 2 heures à 37°C. Après digestion, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) comme décrit dans PREPARATION II1.

Après centrifugation à 4°C pendant 10 min à 12 000 g, le culot d'ADN est lavé par 1 ml d'éthanol 70, lyophilisé et repris dans 17 µl d'eau milli-Q stérile. La digestion par HINDIII est réalisée pendant 2 heures à 37°C dans un volume final de 20 µl.

Après digestion, 4 µl de tampon porteur (30% sucrose, 0,25% bleu de bromophénol) sont ajoutés au mélange et analysé sur gel d'agarose Seaplaque GTG (FMC, Rockland) 0,8% cf. PREPARATION II4. La bande d'ADN correspondant au pBluescript II SK(+) linéarisé par HindIII et PstI est prélevée et déposée dans 350 µl d'une solution de Tris 20mM pH 8, EDTA 1 mM pH 8. Cet échantillon est incubé pendant 5 min à 65°C, extrait par un volume de phénol et centrifugé pendant 5 min à 10 000 g. La phase aqueuse est reprise, extraite par un volume de phénol/chloroforme/ alcool isoamylique (rapport 50/48/2) comme décrit dans PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 10 ng/µl du plasmide pBluescript II SK(+) HindIII/PstI.

La ligation du fragment H (HindIII/PstI) au plasmide pBluescript II SK(+) (HindIII/PstI) est réalisée pendant 4 heures à température ambiante.

Pour la transformation, les bactéries compétentes utilisées sont les DH5alpha (Gibco BRL, Paris).

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega TM), suivant le protocole fourni par le fabricant.

Une fraction aliquote (2 µl sur les 60 obtenus) est soumise à une digestion enzymatique par les endonucléases de restriction HindIII et BamHI, afin de vérifier le clonage du fragment H. Après électrophorèse, le gel est coloré pendant 15 min dans une solution de bromure d'éthidium à 40 ng/ml, et la présence du fragment H est vérifiée sous UV.

La dernière étape est de vérifier l'intégrité de la séquence du fragment H, la technique d'amplification par PCR (polymerase chain reaction) pouvant entraîner des mutations. Les réactions de séquence réalisées avec le kit "T7 sequencing" (Pharmacia TM) qui utilise la technique de terminaison de chaînes et la T7 DNA polymerase (cf PREPARATION 1) permettent de valider l'intégrité de la séquence du fragment G.

### EXEMPLE 1

### Construction de gènes chimériques codant pour la protéine recombinante du collagène humain et permettant une expression dans les feuilles de tabac et les graines de tabac ou de colza.

### 1/Obtention des fragment FG et FH

Cette étape consiste à cloner le fragment BamHI/EcoRI de 1554 pb (nucléotides 2803 à 4357) du clone alpha22 entre les sites BamHI (du vecteur pBluescript II SK(+)) et EcoRI (fragment G ou H) du clone pBluescript II SK(+)-fragment G ou H. Il en résulte l'obtention des clones pBluescript II SK(+)-FG et pBluescript II SK(+)-FH qui comportent entre les sites BamHI et HindIII la séquence codant pour les acides aminés 936 à 1192 du domaine hélicoïdal central, la totalité du domaine carboxy-propeptide (acides aminés 1193 à 1464) de la chaîne proα1(I) humaine et le codon stop TAA couplé au site de restriction HindIII. Dans le cas du clone pBluescript II SK(+)-fragment FH, 4 codons spécifiques de la séquence KDEL (Lys, Asp, Glu, Leu) sont insérés entre la séquence codant pour le domaine carboxy-propeptide et le codon stop TAA.

### Clonage du fragment F (BamHI/EcoRI) avec la partie EcoRI/HindIII du fragment G ou H.

Environ 1 µg du clone ADNc alpha22 sont digérés par l'endonucléase de restriction BamHI pendant 90 min à 37°C. Les conditions utilisées sont: 1 µg alpha22, 2 µl de tampon de digestion E (Promega TM), 1 µl de l'endonucléase de restriction BamHI (12 u/µl; Promega TM), qsp 20 µl H2O milli-Q. La digestion est réalisée pendant 2 heures à 37°C.

Suite à la digestion enzymatique, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) comme décrit dans préparation II1.

Après centrifugation à 4°C pendant 10 min à 12 000 g, le culot d'ADN est lavé par 1 ml d'éthanol 70, lyophilisé et repris dans 35 µl d'eau milli-Q stérile. La digestion par PstI est réalisée pendant 2 heures à 37°C dans un volume final de 40 µl.

Suite à la digestion enzymatique, 8 µl de tampon stop (30% sucrose, 0,25% bleu de bromophénol) sont ajoutés à la solution d'ADN qui est analysée par électrophorèse sur gel d'agarose 1% à bas point de fusion Sea-plaque-GTG (FMC, Rockland) à un voltage constant de 60 volts. Le tampon d'électophorèse est du TBE 0,5X (Tris borate 45 mM, EDTA (Acide éthylène-diamine-tétra-acétique) 1mM, pH 8). Après migration, le gel est coloré pendant 15 min dans une solution de TBE 0,5X contenant 40 ng/ml de bromure d'ethidium.

La bande correspondant au fragment BamHI/EcoRI de 1554 bp est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) comme décrit dans PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega TM) sont analysés sur gel d'agarose type II (Sigma TM) 1%, ce qui nous a permis d'estimer la concentration de la solution purifiée du fragment BamHI/EcoRI du clone ADNc alpha22 à 12 ng/µl.

Le plasmide utilisé pour cloner le fragment BamHI/EcoRI du clone ADNc alpha22 est le clone pBluescript II SK(+)-fragment G ou H digéré par les enzymes BamHI et EcoRI. Ce clone comporte un gène de résistance à l'ampicilline.

Une quantité de 500 ng du plasmide pBluescript II SK(+)-fragment G ou H est soumise à une digestion par l'endonucléase de restriction BamHI. Dans un volume de 20 µl, pendant 2 heures à 37°C. Après digestion, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) comme décrit dans PREPARATION II1.

Après centrifugation à 4°C pendant 10 min à 12 000 g, le culot d'ADN est lavé par 1 ml d'éthanol 70, lyophilisé et repris dans 17 µl d'eau milli-Q stérile: La digestion par EGRI est réalisée pendant 2 heures à 37°C dans un volume final de 20 µl.

Après digestion, 4 µl de tampon porteur (30% sucrose, 0,25% bleu de bromophénol) sont ajoutés au mélange et analysé sur gel d'agarose Seaplaque GTG (FMC, Rockland) 0,8% cf. PREPARATION II4. La bande d'ADN correspondant au pBluescript II SK(+)-fragment G ou H digéré par BamHI et EcoRI est prélevée et déposée dans 350 µl d'une solution de Tris 20mM pH 8, EDTA 1 mM pH 8. Cet échantillon est incubé pendant 5 min à 65°C, extrait par un volume de phénol et centrifugé pendant 5 min à 10 000 g. La phase aqueuse est reprise, extraite par un volume de phénol/chloroforme/ alcool isoamylique (rapport 50/48/2) comme décrit dans PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 20 ng/µl du plasmide pBluescript II SK(+)-fragment G ou H (BamHI/EcoRI).

La ligation du fragment BamHI/EcoRI du clone ADNc alpha22 au clone pBluescript II SK(+)-fragment G ou H (BamHI/EcoRI) est réalisée pendant 4 heures à température ambiante.

Pour la transformation, les bactéries compétentes utilisées sont les DH5alpha (Gibco BRL, Paris).

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega TM), suivant le protocole fourni par le fabricant.

Une fraction aliquote (2 µl sur les 60 obtenus) est soumise à une digestion enzymatique par les endonucléases de restriction HindIII et BamHI, afin de vérifier le clonage du fragment BamHI/EcoRI du clone ADNc alpha22 au clone pBluescript II SK(+)-fragment G ou H (BamHI/EcoRI). Le site de restriction HindIII est couplé au codon stop TAA en 3' du site EcoRI.

Après électrophorèse, le gel est coloré pendant 15 min dans une solution de bromure d'éthidium à 40 ng/ml, et la présence du fragment BamHI/HindIII de 1596 bp dans le cas du fragment FG et de 1608 pb dans le cas du fragment FH est vérifiée sous UV.

Les réactions de séquence ont été réalisées avec le kit "T7 sequencing" (Pharmacia TM) qui utilise la technique de terminaison de chaînes et la T7 DNA polymerase permettent de valider l'intégrité des clones pBluescript II SK(+)-fragment FG et pBluescript II SK(+)-fragment FH.

### 2/Obtention du fragment DE

Cette étape consiste à cloner le fragment DraIII/BamHI (nucléotides 1714 à 2803) de 1089 pb du clone alpha22 entre les sites DraIII (du fragment D) et BamHI (du plasmide pUC18) du clone pUC18-fragment D. Il en résulte l'obtention du clone pUC18-DE qui comporte entre les sites XbaI et BamHI la séquence codant pour les acides aminés 160 à 179 du domaine amino-télopeptide, les acides aminé 180 à 936 de la région hélicoïdale centrale de la chaîne proα1(I) humaine.

### Clonage du fragment E (DraIII/BamHI) avec la partie DraIII/BamHI du fragment D.

Environ 1 µg du clone ADNc alpha22 sont digérés par l'endonucléase de restriction BamHI pendant 90 min à 37°C.

Suite à la digestion enzymatique, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) et centrifugée pendant 2 min à 10 000 g. La phase aqueuse est extraite au chloroforme/alcool isoamylique (rapport 24/1) et centrifugée quelques secondes à 10 000 g. La phase aqueuse est reprise, ajustée à une concentration finale en NaCl de 0,2 M, et précipitée 16 heures à -20°C après apport de deux volumes d'éthanol 100.

Après centrifugation à 4°C pendant 10 min à 12 000 g, le culot d'ADN est lavé par 1 ml d'éthanol 70, lyophilisé et repris dans 35 µl d'eau milli-Q stérile. La digestion par DraIII est réalisée pendant 2 heures à 37°C dans un volume final de 40 µl.

Suite à la digestion enzymatique, 8 µl de tampon stop (30% sucrose, 0,25% bleu de bromophénol) sont ajoutés à la solution d'ADN qui est analysée par électrophorèse sur gel d'agarose 1% à bas point de fusion Sea-plaque-GTG (FMC, Rockland) cf EXEMPLE I1.

La bande correspondant au fragment DraIII/BamHI de 1089 pb du clone ADNc alpha22 est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) cf PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega TM) sont analysés sur gel d'agarose type II (Sigma TM) 2%, ce qui nous a permis d'estimer la concentration de la solution purifiée du fragment DraIII/BamHI du clone ADNc alpha22 à 20 ng/µl.

Le plasmide utilisé pour cloner le fragment DraIII/BamHI du clone ADNc alpha22 est le clone pUC18-fragment D digéré par les enzymes DraIII et BamHI. Ce clone comporte un gène de résistance à l'ampicilline.

Une quantité de 500 ng du plasmide pUC18-fragment D est soumise à une digestion par l'endonucléase de restriction BamHI. Dans un volume de 20 µl pendant 2 heures à 37°C. Après digestion, 80 µl de tampon TE (tris 10 mM, EDTA 1 mM, pH 8) sont ajoutés à la solution d'ADN qui est extraite par un volume de phénol/chloroforme/alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 17 µl d'eau milli-Q stérile. La digestion par DraIII est réalisée pendant 2 heures à 37°C dans un volume final de 20 µl.

Après digestion, 4 µl de tampon porteur (30% sucrose, 0,25% bleu de bromophénol) sont ajoutés au mélange et analysé sur gel d'agarose Seaplaque GTG (FMC, Rockland) 0,8%. La bande d'ADN correspondant au pUC18-fragment D digéré par DraIII et BamHI est prélevée et déposée dans 350 µl d'une solution de Tris 20mM pH 8, EDTA 1 mM pH 8. Cet échantillon est incubé pendant 5 min à 65°C, extrait par un volume de phénol et centrifugé pendant 5 min à 10 000 g. La phase aqueuse est reprise, extraite par un volume de phénol/chloroforme/ alcool isoamylique (rapport 50/48/2) cf PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 20 ng/µl du plasmide pUC18-fragment D (DraIII/BamHI).

La ligation du fragment DraIII/BamHI du clone ADNc alpha22 au clone pUC18-fragment D (DraIII/BamHI est réalisée pendant 4 heures à température ambiante.

Pour la transformation, les bactéries compétentes utilisées sont les DH5alpha (Gibco BRL, Paris).

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega TM), suivant le protocole fourni par le fabricant.

Une fraction aliquote (2 µl sur les 60 obtenus) est soumise à une digestion enzymatique par les endonucléases de restriction XbaI et BamHI, afin de vérifier le clonage du fragment DraIII/BamHI du clone ADNc alpha22 au clone pUC18-fragment D (DraIII/BamHI). Le site de restriction XbaI est situé en 5' du clone pUC18-fragment D. Après électrophorèse, le gel est coloré pendant 15 min dans une solution de bromure d'éthidium à 40 ng/ml, et la présence du fragment BamHI/XbaI de 2336 pb est vérifiée sous UV.

Les réactions de séquence ont été réalisées avec le kit "T7 sequencing" (Pharmacia TM) qui utilise la technique de terminaison de chaînes et la T7 DNA polymerase permettent de valider l'intégrité du clone pUC18-fragment DE.

### 3/Obtention des fragments DEFG et DEFH

Cette étape consiste à cloner le fragment XbaI/BamHI (nucléotides 474 à 2803) de 2329 pb du clone pUC18-fragment DE entre les sites XbaI (du plasmide pBluescript II SK(+)) et BamHI (du fragment FG ou FH) du clone pBluescript II SK(+)-fragment FG ou FH. Il en résulte l'obtention des clones pBluescript II SK(+)-fragment DEFG et pBluescript II SK(+)-fragment DEFH qui comporte entre les sites XbaI et HindIII la séquence codant pour les acides aminés 160 à 179 du domaine amino-télopeptide, la totalité de la région helicoïdale centrale (acides aminés 180 à 1192), la totalité du domaine carboxy-propeptide (acides aminés 1193 à 1464 de la chaîne proα1(I) humaine, et le codon stop TAA couplé au site de restriction HindIII. Dans le cas du clone pBluescript II SK(+)-fragment DEFH, 4 codons spécifiques de la séquence KDEL (Lys, Asp, Glu, Leu) sont insérés entre la séquence codant pour le domaine carboxy-propeptide et le codon stop TAA.

### Clonage du fragment DE (XbaI/BamHI) avec la partie BamHI/HindIII du fragment FG ou FH.

Environ 1 µg du clone pUC18-fragment DE sont digérés par l'endonucléase de restriction BamHI pendant 90 min à 37°C.

Suite à la digestion enzymatique, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) cf PREPARATION II1.

Après centrifugation à 4°C pendant 10 min à 12 000 g, le culot d'ADN est lavé par 1 ml d'éthanol 70, lyophilisé et repris dans 35 µl d'eau milli-Q stérile. La digestion par XbaI est réalisée pendant 2 heures à 37°C dans un volume final de 20 µl.

Suite à la digestion enzymatique, 8 µl de tampon stop (30% sucrose, 0,25% bleu de bromophénol) sont ajoutés à la solution d'ADN qui est analysée par électrophorèse sur gel d'agarose 1% à bas point de fusion Sea-plaque-GTG (FMC, Rockland) EXEMPLE I1.

La bande correspondant au fragment XbaI/BamHI de 2329 pb est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega TM) sont analysés sur gel d'agarose type II (Sigma TM) 1%, ce qui nous a permis d'estimer la concentration de la solution purifiée du fragment XbaI/BamHI du clone pUC18-fragment DE à 20 ng/µl.

Le plasmide utilisé pour cloner le fragment XbaI/BamHI du clone puC18-fragment DE est le clone pBluescript II SK(+)-fragment FG ou FH digéré par les enzymes XbaI et BamHI. Ce clone comporte un gène de résistance à l'ampicilline.

Une quantité de 500 ng du plasmide pBluescript II SK(+)-fragment FG ou FH est soumise à une digestion par l'endonucléase de restriction BamHI. Pendant 2 heures à 37°C. Après digestion, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) cf. PREPARATION III.

Après centrifugation à 4°C pendant 10 min à 12 000 g, le culot d'ADN est lavé par 1 ml d'éthanol 70, lyophilisé et repris dans 17 µl d'eau milli-Q stérile. La digestion par baI est réalisée pendant 2 heures à 37°C.

Après digestion, 4 µl de tampon porteur (30% sucrose, 0,25% bleu de bromophénol) sont ajoutés au mélange et analysé sur gel d'agarose Seaplaque GTG (FMC, Rockland) 0,8%. La bande d'ADN correspondant au pBluescript II SK(+)-fragment FG ou FH digéré par XbaI et BamHI est prélevée et déposée dans 350 µl d'une solution de Tris 20mM pH 8, EDTA 1 mM pH 8. Cet échantillon est incubé pendant 5 min à 65°C, extrait par un volume de phénol et centrifugé pendant 5 min à 10 000 g. La phase aqueuse est reprise, extraite par un volume de phénol/chloroforme/ alcool isoamylique (rapport 50/48/2) cf PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 20 ng/µl du plasmide pBluescript II SK(+)-fragment FG ou FH (XbaI/BamHI).

La ligation du fragment XbaI/BamHI du clone pUC18-fragment DE au clone pBluescript II SK(+)-fragment FG ou FH (XbaI/BamHI) est réalisée pendant 4 heures à température ambiante.

Pour la transformation, les bactéries compétentes utilisées sont les DH5alpha (Gibco BRL, Paris).

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega TM), suivant le protocole fourni par le fabricant.

Une fraction aliquote (2 µl sur les 60 obtenus) est soumise à une digestion enzymatique par les endonucléases de restriction XbaI et HindIII, afin de vérifier le clonage du fragment XbaI/BamHI du clone pUC18-fragment DE au clone pBluescript II SK(+)-fragment FG ou FH (BamHI/HindIII). Après électrophorèse, le gel est coloré pendant 15 min dans une solution de bromure d'éthidium à 40 ng/ml, et la présence du fragment XbaI/HindIII d'environ 3900 bp est vérifiée sous UV.

Les réactions de séquence ont été réalisées avec le kit "T7 sequencing" (Pharmacia TM) qui utilise la technique de terminaison de chaînes et la T7 DNA polymerase permettent de valider l'intégrité des clones pBluescript II SK(+)-fragment DEFG et pBluescript II SK(+)-fragment DEFH.

### 4/Obtention des fragments ADEFG et ADEFH clones ADNc complets spécifiques de la chaîne proα1(I) humaine.

Cette étape consiste à cloner le fragment SacII/NheI (nucléotides -4 à 479) de 495 pb du clone pBluescript II SK(+)-fragment A entre les sites SacII (du plasmide pBluescript II SK(+)) et NheI (du fragment DEFG ou DEFH) du clone pBluescript II SK(+)-fragment DEFG ou DEFH. Il en résulte l'obtention des clones pBluescript II SK(+)-fragment ADEFG et pBluescript II SK(+)-fragment ADEFH qui comportent entre les sites SacII et HindIII la séquence codant pour totalité de la chaîne proα1(I) humaine, et le codon stop TAA couplé au site de restriction HindIII. Un autre site HindIII est localisé juste après le site SacII, ce qui permettra de sortir les fragments ADEFG et ADEFH par simple digestion par l'endonucléase de restriction HindIII. Dans le cas du clone pBluescript II SK(+)-fragment ADEFH, 4 codons spécifiques de la séquence KDEL (Lys, Asp, Glu, Leu) sont insérés entre la séquence codant pour le domaine carboxy-propeptide et le codon stop TAA. Le site NheI entraîne la substitution des acides aminés Asn et Phe par une lysine et une leucine en position 158 et 159 de la chaîne proα1(I) humaine.

### Clonage du fragment A (SacII/NheI) avec la partie NheI/HindIII du fragment DEFG ou DEFH.

Environ 1 µg du clone pBluescript II SK(+)-fragment A sont digérés par l'endonucléase de restriction SacII pendant 90 min à 37°C.

Suite à la digestion enzymatique, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 35 µl d'eau milli-Q stérile. A cette solution d'ADN sont ajoutés 4 µl de tampon de digestion B (Promega TM), 1 µl de l'endonucléase de restriction NheI (12 u/µl; Promega TM). La digestion est réalisée pendant 2 heures à 37°C.

Suite à la digestion enzymatique, 8 µl de tampon stop (30% sucrose, 0,25% bleu de bromophénol) sont ajoutés à la solution d'ADN qui est analysée par électrophorèse sur gel d'agarose 2% à bas point de fusion NueSieve-GTG (FMC, Rockland) à un voltage constant de 60 volts. Le tampon d'électophorèse est du TBE 0,5X (Tris borate 45 mM, EDTA (Acide éthylène-diamine-tétra-acétique) 1mM, pH 8). Après migration, le gel est coloré pendant 15 min dans une solution de TBE 0,5X contenant 40 ng/ml de bromure d'ethidium.

La bande correspondant au fragment SacII/NheI de 495 bp est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega TM) sont analysés sur gel d'agarose type II (Sigma TM) 2%, ce qui nous a permis d'estimer la concentration de la solution purifiée du fragment SacII/NheI du clone pBluescript II SK(+)-fragment A à 5 ng/µl.

Le plasmide utilisé pour cloner le fragment SacII/NheI du clone pBluescript II SK(+)-fragment A est le clone pBluescript II SK(+)-fragment DEFG ou DEFH digéré par les enzymes SacII et NheI. Ce clone comporte un gène de résistance à l'ampicilline.

Une quantité de 500 ng du plasmide pBluescript II SK(+)-fragment DEFG ou DEFH est soumise à une digestion par l'endonucléase de restriction SacII. Dans un volume de 20 µl pendant 2 heures à 37°C. Après digestion, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) cf PREPARATION II1.

Après centrifugation à 4°C pendant 10 min à 12 000 g, le culot d'ADN est lavé par 1 ml d'éthanol 70, lyophilisé et repris dans 17 µl d'eau milli-Q stérile. La digestion par NhcI est réalisée pendant 2 heures à 37°C.

Après digestion, 4 µl de tampon porteur (30% sucrose, 0,25% bleu de bromophénol) sont ajoutés au mélange et analysé sur gel d'agarose Seaplaque GTG (FMC, Rockland) 0,8% cf. PREPARATION II4. La bande d'ADN correspondant au pBluescript II SK(+)-fragment DEFG ou DEFH digéré par SacII et NheI est prélevée et déposée dans 350 µl d'une solution de Tris 20mM pH 8, EDTA 1 mM pH 8. Cet échantillon est incubé pendant 5 min à 65°C, extrait par un volume de phénol et centrifugé pendant 5 min à 10 000 g. La phase aqueuse est reprise, extraite par un volume de phénol/chloroforme/ alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 20 ng/µl du plasmide pBluescript II SK(+)-fragment DEFG ou DEFH (SacII/NheI).

La ligation du fragment SacII/NheI du clone pBluescript II SK(+)-fragment A au clone pBluescript II SK(+)-fragment DEFG ou DEFH (SacII/NheI) pendant 4 heures à température ambiante.

Pour la transformation, les bactéries compétentes utilisées sont les DH5alpha (Gibco BRL, Paris).

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega TM), suivant le protocole fourni par le fabricant.

Une fraction aliquote (2 µl sur les 60 obtenus) est soumise à une digestion enzymatique par l'endonucléase de restriction HindIII, afin de vérifier le clonage du fragment SacII/NheI du clone pBluescript II SK(+)-fragment A au clone pBluescript II SK(+)-fragment DEFG ou DEFH (NheI/HindIII). L'apparition d'un fragment HindIII de 4400 pb valide ces clonages. Après électrophorèse, le gel est coloré pendant 15 min dans une solution de bromure d'éthidium à 40 ng/ml, et la présence du fragment HindIII/HindIII de 4400 bp est vérifiée sous UV.

Les réactions de séquence ont été réalisées avec le kit "T7 sequencing" (Pharmacia TM) qui utilise la technique de terminaison de chaînes et la T7 DNA polymerase permettent de valider l'intégrité des clones pBluescript II SK(+)-fragment ADEFG et pBluescript II SK(+)-fragment ADEFH.

Pour rappel, le clone pBluescript II SK(+)-fragment ADEFG comprend entre ces deux sites HindIII, la totalité de la séquence codant pour la chaîne proα1(I) humaine.

Le clone pBluescript II SK(+)-fragment ADEFH comprend entre ces deux sites HindIII, la totalité de la séquence codant pour la chaîne proα1(I) humaine. De plus, il génère en partie carboxy-terminale de cette chaîne, la séquence de rétention dans le réticulum endoplasmique Lys-Asp-Glu-Leu.

### 5/Obtention du fragment BDEFG, clone ADNc codant pour le signal peptide PRS de plante et la totalité de chaîne proα1(I) humaine à l'exception du domaine amino-propeptide.

Cette étape consiste à cloner le fragment SacII/NheI (nucléotides -3 à 75) de 90 pb du clone pBluescript II SK(+)-fragment B entre les sites SacII (du plasmide pBluescript II SK(+)) et NheI (du fragment DEFG) du clone pBluescript II SK(+)-fragment DEFG. Il en résulte l'obtention du clone pBluescript II SK(+)-fragment BDEFG qui comporte entre les sites SacII et HindIII la séquence codant pour le signal peptide PRS de plante, la totalité de la chaîne proα1(I) humaine à l'exception du domaine amino-propeptide, et le codon stop TAA couplé au site de restriction HindIII. Un autre site HindIII est localisé juste après le site SacII, ce qui permettra de sortir le fragment BDEFG par simple digestion par l'endonucléase de restriction HindIII. Le site NheI entraîne la substitution des acides aminés Glu et Gly par une leucine et une Alanine en position 25 et 26 de la chaîne proα1(I) humaine.

Le signal peptide PRS (Pathogenesis-Related Protein S) de plante remplace le signal peptide propre à la chaîne proα1(I) humaine.

### Clonage du fragment B (SacII/NheI) avec la partie NheI/HindIII du fragment DEFG.

Environ 2 µg du clone pBluescript II SK(+)-fragment B sont digérés par l'endonucléase de restriction SacII pendant 90 min à 37°C.

Suite à la digestion enzymatique, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 34 µl d'eau milli-Q stérile. La digestion par NhcI est réalisée pendant 2 heures à 37°C.

Suite à la digestion enzymatique, 8 µl de tampon stop (30% sucrose, 0,25% bleu de bromophénol) sont ajoutés à la solution d'ADN qui est analysée par électrophorèse sur gel d'agarose 2% à bas point de fusion NueSieve-GTG (FMC, Rockland) cf. PREPARATION II1.

La bande correspondant au fragment SacII/NheI de 90 bp est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega TM) sont analysés sur gel d'agarose type II (Sigma TM) 2%, ce qui nous a permis d'estimer la concentration de la solution purifiée du fragment SacII/NheI du clone pBluescript II SK(+)-fragment B à 1 ng/µl.

Le plasmide utilisé pour cloner le fragment SacII/NheI du clone pBluescript II SK(+)-fragment B est le clone pBluescript II SK(+)-fragment DEFG digéré par les enzymes SacII et NheI. Ce clone comporte un gène de résistance à l'ampicilline.

Une quantité de 500 ng du plasmide pBluescript II SK(+)-fragment DEFG est soumise à une digestion par l'endonucléase de restriction SacII. Dans un volume de 20 µl pendant 2 heures à 37°C. Après digestion, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 17 µl d'eau milli-Q stérile. La digestion par NhcI est réalisée pendant 2 heures à 37°C.

Après digestion, 4 µl de tampon porteur (30% sucrose, 0,25% bleu de bromophénol) sont ajoutés au mélange et analysé sur gel d'agarose Seaplaque GTG (FMC, Rockland) 0,8% cf. PREPARATION II4. La bande d'ADN correspondant au pBluescript II SK(+)-fragment DEFG digéré par SacII et NheI est prélevée et déposée dans 350 µl d'une solution de Tris 20mM pH 8, EDTA 1 mM pH 8. Cet échantillon est incubé pendant 5 min à 65°C, extrait par un volume de phénol et centrifugé pendant 5 min à 10 000 g. La phase aqueuse est reprise, extraite par un volume de phénol/chloroforme/ alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 20 ng/µl du plasmide pBluescript II SK(+)-fragment DEFG (SacII/NheI).

La ligation du fragment SacII/NheI du clone pBluescript II SK(+)-fragment B au clone pBluescript II SK(+)-fragment DEFG (SacII/NheI) est pendant 4 heures à température ambiante.

Pour la transformation, les bactéries compétentes utilisées sont les DH5alpha (Gibco BRL, Paris).

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega TM), suivant le protocole fourni par le fabricant.

Une fraction aliquote (2 µl sur les 60 obtenus) est soumise à une digestion enzymatique par l'endonucléase de restriction HindIII, afin de vérifier le clonage du fragment SacII/NheI du clone pBluescript II SK(+)-fragment B au clone pBluescript II SK(+)-fragment DEFG (NheI/HindIII). L'apparition d'un fragment HindIII de 4000 pb valide ces clonages. Après électrophorèse, le gel est coloré pendant 15 min dans une solution de bromure d'éthidium à 40 ng/ml, et la présence du fragment HindIII/HindIII de 4000 bp est vérifiée sous UV.

Les réactions de séquence ont été réalisées avec le kit "T7 sequencing" (Pharmacia TM) qui utilise la technique de terminaison de chaînes et la T7 DNA polymerase permettent de valider l'intégrité du clone pBluescript II SK(+)-fragment BDEFG.

Pour rappel, le clone pBluescript II SK(+)-fragment BDEFG comprend entre ces deux sites HindIII, la séquence codant pour le signal peptide PRS de plante, et la totalité de la chaîne proα1(I) humaine, à l'exception du domaine amino-propeptide.

La création du site NheI, entraîne la substitution des acides aminés Glu et Gly par une leucine et une alanine en position 25 et 26 de la chaîne proα1(I) humaine.

### 6/Obtention du fragment BCDEFG, clone ADNc codant pour le signal peptide PRS de plante et la totalité de chaîne proα1(I) humaine.

Cette étape consiste à cloner le fragment NheI/NheI (nucléotides 72 à 479) de 402 pb du clone pBluescript II SK(+)-fragment C dans le site NheI (du fragment BDEFG) du clone pBluescript II SK(+)-fragment BDEFG. Il en résulte l'obtention du clone pBluescript II SK(+)-fragment BCDEFG qui comporte entre les sites SacII et HindIII la séquence codant pour le signal peptide PRS de plante, la totalité de la chaîne proα1(I) humaine, et le codon stop TAA couplé au site de restriction HindIII. Un autre site HindIII est localisé juste après le site SacII, ce qui permettra de sortir le fragment BCDEFG par simple digestion par l'endonucléase de restriction HindIII. Les sites NheI entraîne la substitution des acides aminés Glu et Gly par une leucine et une alanine en position 25 et 26 de la chaîne proα1(I) humaine, et la substitution des acides aminés Asn et Phe par une lysine et une leucine en position 158 et 159 de la chaîne proα1(I) humaine.

Le signal peptide PRS de plante remplace le signal peptide propre à la chaîne proα1(I) humaine.

### Clonage du fragment C (NheI/NheI) dans le site NheI du fragment BDEFG.

Environ 1 µg du clone pBluescript II SK(+)-fragment C sont digérés par l'endonucléase de restriction NheI pendant 90 min à 37°C.

Suite à la digestion enzymatique, 4 µl de tampon stop (30% sucrose, 0,25% bleu de bromophénol) sont ajoutés à la solution d'ADN qui est analysée par électrophorèse sur gel d'agarose 2% à bas point de fusion NueSieve-GTG (FMC, Rockland) cf. PREPARATION II1.

La bande correspondant au fragment NheI/NheI de 402 bp est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega TM) sont analysés sur gel d'agarose type II (Sigma TM) 2%, ce qui nous a permis d'estimer la concentration de la solution purifiée du fragment NheI/NheI du clone pBluescript II SK(+)-fragment C à 5 ng/µl.

Le plasmide utilisé pour cloner le fragment NheI/NheI du clone pBluescript II SK(+)-fragment C est le clone pBluescript II SK(+)-fragment BDEFG linéarisé par l'enzyme NheI. Ce clone comporte un gène de résistance à l'ampicilline.

Une quantité de 250 ng du plasmide pBluescript II SK(+)-fragment BDEFG est soumise à une digestion par l'endonucléase de restriction NheI. Dans un volume de 20 µl, pendant 2 heures à 37°C. Après digestion, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 17 µl d'eau milli-Q stérile. A cette solution d'ADN sont ajoutés 2 µl de tampon 10X de la phosphatase alcaline d'intestinc de veaux (CIP, Promega TM), 1 µl de CIP (1 u/µl; Promega TM). La déphosphoration est réalisée pendant 1 heures à 37°C. Cette étape permettra d'éviter l'autoligation de ce clone pendant l'étape de ligation.

Après digestion, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 10 ng/µl du plasmide pBluescript II SK(+)-fragment BDEFG (NheI, déphosphorylé).

La ligation du fragment NheI/NheI du clone pBluescript II SK(+)-fragment C au clone pBluescript II SK(+)-fragment BDEFG (NheI, déphosphorylé) est réalisée pendant 4 heures à température ambiante.

Pour la transformation, les bactéries compétentes utilisées sont les DH5alpha (Gibco BRL, Paris).

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega TM), suivant le protocole fourni par le fabricant.

Une fraction aliquote (2 µl sur les 60 obtenus) est soumise à une digestion enzymatique par l'endonucléase de restriction NheI, afin de vérifier le clonage du fragment NheI/NheI du clone pBluescript II SK(+)-fragment C au clone pBluescript II SK(+)-fragment BDEFG (NheI). L'apparition d'un fragment NheI de 402 pb valide ces clonages. Après électrophorèse, le gel est coloré pendant 15 min dans une solution de bromure d'éthidium à 40 ng/ml, et la présence du fragment NheI de 402 pb est vérifiée sous UV.

Les réactions de séquence ont été réalisées avec le kit "T7 sequencing" (Pharmacia TM) qui utilise la technique de terminaison de chaînes et la T7 DNA polymerase permettent de valider l'intégrité du clone pBluescript II SK(+)-fragment BCDEFG.

Pour rappel, le clone pBluescript II SK(+)-fragment BCDEFG comprend entre ces deux sites HindIII, la séquence codant pour le signal peptide PRS de plante, et la totalité de la chaîne proα1(I) humaine.

La création des sites NheI, entraîne la substitution des acides aminés Glu et Gly par une leucine et une alanine en position 25 et 26 de la chaîne proα1(I) humaine, et la substitution des acides aminés Asn et Phe par une lysine et une leucine en position 158 et 159 de la chaîne proα1(I) humaine.

### 7/ Obtention des 4 constructions pBIOC21-collagène humain recombinant.

Les constructions des différents plasmides via l'utilisation des techniques d'ADN recombinant dérivent de pBIOC4. Le plasmide binaire dérive de pGA442 (An, 1986).Le plasmide dérivant de pBIOC4 et contenant la cassette d'expression ''PD35S-T35S'' est le plasmide pBIOC21.

Les différents éléments permettant de reproduire ces constructions sont par exemple contenus dans la description de la demande de brevet WO9633277 qui est incorporée par référence.

L'obtention des quatre constructions pBIOC21-collagène humain recombinant a consisté à passer les 4 constructions du plasmide pBluescript II SK(+) au plasmide pBIOC21.

Le plasmide pBIOC21 est utilisé pour permettre l'expression du collagène recombinant dans les feuilles et les graines de tabac ou de colza. Il apporte les séquences régulatrices suivantes:
a) le promoteur constitutif double 35S (PD35S) du virus de la mosaïque du chou-fleur (CaMV). Il correspond à une duplication de la séquence activant la transcription située en amont de l'élément TATA du promoteur 35S naturel.
b) la séquence terminatrice de transcription, le terminateur polyA 35S qui correspond à la séquence 3' non codante de la séquence du virus à ADN bicaténaire circulaire du CaMV produisant le transcrit 35S.

### Clonage des fragments (HindIII/HindIII) des constructions pBluescrint, dans le site de clonage HindIII du vecteur pBIOC21.

Environ 1 µg du clone pBluescript II SK(+)-fragment ADEFG ou ADEFH ou BDEFG ou BCDEFG sont digérés par l'endonucléase de restriction HindIII pendant 90 min à 37°C.

Suite à la digestion enzymatique, 4 µl de tampon stop (30% sucrose, 0,25% bleu de bromophénol) sont ajoutés à la solution d'ADN qui est analysée par électrophorèse sur gel d'agarose 0,8% à bas point de fusion SeaPlaque-GTG (FMC, Rockland) cf. PREPARATION II4.

La bande correspondant au fragment HindIII/HindIII de 4400 bp est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) cf. PREPARATION II1.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega TM) sont analysés sur gel d'agarose type II (Sigma TM) 0,8%, ce qui nous a permis d'estimer la concentration de la solution purifiée du fragment HindIII/HindIII du clone pBluescript II SK(+)-fragment ADEFG ou ADEFH ou BDEFG ou BCDEFG à 25 ng/µl.

Le plasmide utilisé pour cloner le fragment HindIII/HindIII du clone pBluescript II SK(+)-fragment ADEFG ou ADEFH ou BDEFG ou BCDEFG est le clone pBIOC21 linéarisé par l'enzyme HindIII. Ce clone comporte un gène de résistance à la tétracycline.

Une quantité de 1 µg du plasmide pBIOC21 est soumise à une digestion par l'endonucléase de restriction HindIII. Dns un volume de 20 µl pendant 2 heures à 37°C. Après digestion, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) cf. PREPARATION II1

Après centrifugation à 4°C pendant 10 min à 12 000 g, le culot d'ADN est lavé par 1 ml d'éthanol 70, lyophilisé et repris dans 17 µl d'eau milli-Q stérile. A cette solution d'ADN sont ajoutés 2 µl de tampon 10X de la phosphatase alcaline d'intestins de veaux (CIP, Promega TM), 1 µl de CIP (1 u/µl; Promega TM). La déphosphorylation est réalisée pendant 1 heures à 37°C. Cette étape permettra d'éviter l'autoligation de ce clone pendant l'étape de ligation.

Après digestion, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) cf. PREPARATION II1

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 40 ng/µl du plasmide pBIOC21 (HindIII, déphosphorylé).

La ligation du fragment HindIII/HindIII du clone pBluescript II SK(+)-fragment ADEFG ou ADEFH ou BDEFG ou BCDEFG au clone pBIOC21 (HindIII, déphosphorylé) est réalisée pendant 4 heures à température ambiante.

Pour la transformation, les bactéries compétentes utilisées sont les DH5alpha (Gibco BRL, Paris).

Plusieurs colonies blanches sont testées. Pour cela, elles sont individuellement déposées dans 3 ml de L-Broth supplémenté en tétracycline (40 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega TM), suivant le protocole fourni par le fabricant. Environ 1-2 µg d'ADN plasmidien est purifié grâce à ce kit, lorsque le plasmide utilisé est le pBIOC21.

Une fraction aliquote (5 µl sur les 60 obtenus) est soumise à une digestion enzymatique par l'endonucléase de restriction KpnI, afin de vérifier l'orientation du clonage du fragment HindIII du clone pBluescript II SK(+)-fragment ADEFG ou ADEFH ou BCDEFG au clone pBIOC21 (HindIII). L'apparition d'un fragment KpnI de 950 pb est spécifique d'une orientation correcte. Ce fragment KpnI de 950 pb résulte d'un site de restriction KpnI en position 2.8 kb du vecteur pBIOC21 et d'un site KpnI en position 137 de la séquence codant pour la chaîne pro[α1(I) humaine. Après électrophorèse, le gel est coloré pendant 15 min dans une solution de bromure d'éthidium à 40 ng/ml, et la présence du fragment KpnI de 950 bp est vérifiée sous UV.

Dans le cas de la construction pBIOC21-fragment BDEFG, nous avons utilisé l'enzyme NheI et comparé son mode de digestion avec le vecteur pBIOC21)-fragment BCDEFG digéré par NheI. La seule différence est la présence d'un fragment NheI/NheI surnuméraire dans le cas du vecteur pBIOC21-fragment BCDEFG.

Les réactions de séquence ont été réalisées avec le kit "T7 sequencing" (Pharmacia TM) qui utilise la technique de terminaison de chaînes et la T7 DNA polymerase permettent de valider l'intégrité des 4 constructions pBIOC21 réalisées.

Les séquences des 4 constructions vont être maintenant indiquées. Dans chaque cas, le signal peptide PRS de plante ou de la chaîne proα1(I) humaine sont soulignés, les substitutions d'acides aminés dû à la création d'un ou de deux sites de restriction NheI sont indiquées en gras ainsi que la séquence de rétention dans le réticulum endoplasmique KDEL. Les sites de reconnaissance à l'amino- et la carboxy-proteinase sont indiqués en relief.

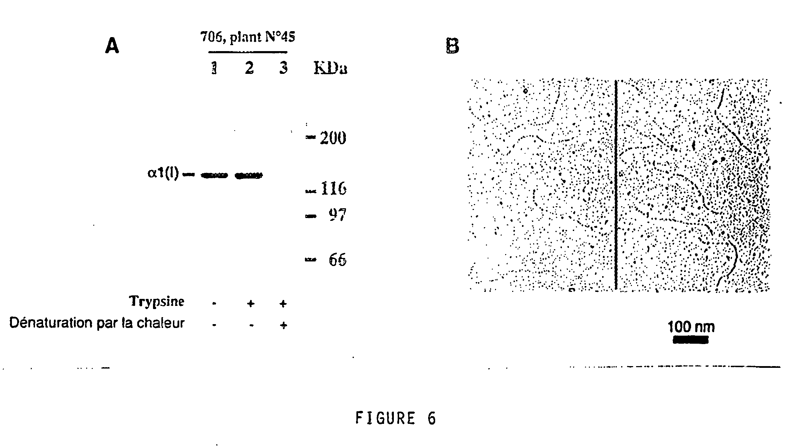

Les constructions pBIOC21-collagène ainsi obtenues (fig 5 et 6) seront nommées ci après:
- pBIOC704 pour la construction contenant le fragment ADEFG
- pBIOC705 pour la construction contenant le fragment ADEFH
- pBIOC706 pour la construction contenant le fragment BDEFG
- pBIOC707 pour la construction contenant le fragment BCDEFG

L'ADN plasmidique des plasmides binaires, pBIOC704, pBIOC705, pBIOC706 et pBIOC707, a été introduit par transformation directe dans la souche LBA4404 d'Agrobacterium Tumefaciens selon le procédé de Holsters (1978). La validité des clones retenus est vérifiée par digestion enzymatique de l'ADN plasmidique introduit.

Restauration des acides aminés Asn et Phe, en position 158 et 159 de la chaîne proα1(I) humaine, dans les les clones ADEFG, ADEFH et BCDEFG.

L'ensemble de ces clones sont inclus dans le vecteur pBluescript II SK(+) (Stratagene, TM). L'opération est d'échanger la séquence comprise entre les sites KpnI (bases 137 à 142) et DraIII (bases 1709 à 1720) de ces constructions par la séquence KpnI-DraIII du clone ADNc 1alpha3. Cette opération entraîne la restauration des acides aminés 158 et 159 de la chaîne proα1(I) (Asn et Phe). Cette opération a été mené puisque Morikawa (1980) ont montré que le résidu 159 (Phe) localisé trois acides aminés en amont du site de clivage Pro-Gln semblait jouer un rôle important pour la coupure de la chaîne proα1(I) par l'aminoprotéinase.

### 1/ Elimination du site de restriction KpnI du vecteur pUC18

Le vecteur pUC18 a été utilisé puisqu'il ne possède pas de site de restriction DraIII interne, à la différence du vecteur pBluescript II SK (+). L'élimination du site de restriction KpnI permettra l'utilisation des enzymes DraIII et KpnI, dans le vue de l'échange des fragments recombinants KpnI-DraIII des clones ADEFG ou ADEFH ou BCDEFG par le fragment sauvage KpnI-DraIII du clone ADNc lalpha3.

Pour cela, 100 ng du vecteur pUC18 sont digérés par l'endonucléase de restriction KpnI (Promega, TM) en suivant les recommandations du fabricant. Le vecteur linéarisé en KpnI a été ensuite traité par la nucléase Mung Bean (Promega, TM) selon les recommandations du fabricant afin d'obtenir un vecteur pUC18 KpnI à extrémités franches. Les étapes de ligation, transformation et de séquençage des clones obtenus sont réalisées comme présentées dans la partie préparation II.

### 2/ Transfert des fragments ADEFG, ADEFH et BCDEFG dans le vecteur pUC18minus KpnI

Pour cela 200 ng des constructions pBluescript II SK(+)-ADEFG ou ADEFH ou BCDEFG sont digérées par l'endonucléase de restriction HindIII (Promega, TM) selon les recommandations du fabricant.

Suite à la digestion enzymatique, 4 µl de tampon stop (30% sucrose, 0,25% bleu de bromophénol) sont ajoutés à la solution d'ADN qui est analysée par électrophorèse sur gel d'agarose 0,8% à bas point de fusion SeaPlaque-GTG (FMC, TM) à un voltage constant de 60 volts. Le tampon d'électophorèse est du TBE 0,5X (Tris borate 45 mM, EDTA (Acide éthylène-diamine-tétra-acétique) 1mM, pH 8). Après migration, le gel est coloré pendant 15 min dans une solution de TBE 0,5X contenant 40 ng/ml de bromure d'éthidium.

La bande correspondant au fragment ADEFG ou ADEFH ou BCDEFG est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) et centrifugée pendant 2 min à 10 000 g. La phase aqueuse est extraite au chloroforme/alcool isoamylique (rapport 24/1) et centrifugée quelques secondes à 10 000 g. La phase aqueuse est reprise, ajustée à une concentration finale en NaCl de 0,2 M, et précipitée 16 heures à -20°C après apport de deux volumes d'éthanol 100.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega, TM) sont analysés sur gel d'agarose type II (Sigma, TM) 0,8%, ce qui nous a permis d'estimer la concentration de la solution purifiée du fragment D BamHI/XbaI à 5 ng/µl.

Le plasmide utilisé pour cloner le fragment HindIII du clone pBluescript II SK(+)-fragment ADEFG ou ADEFH ou BCDEFG est le clone pUC18minus KpnI linéarisé par l'enzyme HindIII. Ce clone comporte un gène de résistance à l'ampicilline.

Une quantité de 250 ng du plasmide pUC18minusKpnI est soumise à une digestion par l'endonucléase de restriction HindIII selon les recommandations du fabricant. Suite à cette digestion, l'ADN est traité par la phosphatase alcaline d'intestins de veaux (CIP, Promega, TM) selon les recommandations du fabricant. Cette étape permettra d'éviter l'autoligation de ce clone pendant l'étape de ligation.

Après digestion, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) et centrifugée pendant 2 min à 10 000 g. La phase aqueuse est extraite au chloroforme/alcool isoamylique (rapport 24/1) et centrifugée quelques secondes à 10 000 g. La phase aqueuse est reprise, ajustée à une concentration finale en NaCl de 0,2 M, et précipitée 16 heures à -20°C après apport de deux volumes d'éthanol 100.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 10 ng/µl du plasmide pUC18minusKpnI (HindIII, déphosphorylé).

La ligation du fragment HindIII du clone pBluescript II SK(+)-fragment ADEFG ou ADEFH ou BCDEFG au vecteur pUC18minusKpnI (HindIII, déphosphorylé) est réalisée pendant 4 heures à température ambiante.

Pour la transformation, les bactéries compétentes utilisées sont les DH5alpha (Gibco BRL, TM).

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega, TM), qui permet de purifier de l'ADN plasmidien super enroulé. Cette manipulation est réalisée suivant le protocole fourni par le fabricant. Environ 10 µg d'ADN plasmidien est purifié grâce à ce kit, lorsque le plasmide utilisé est le pUC18.

Une fraction aliquote (2 µl sur les 60 obtenus) est soumise à une digestion enzymatique par l'endonucléase de restriction HindIII, afin de vérifier le clonage du fragment HindIII du clone pBluescript II SK(+)-fragment ADEFG ou ADEFH ou BCDEFG au clone pUC18minusKpnI (HindIII). Après électrophorèse, le gel est coloré pendant 15 min dans une solution de bromure d'éthidium à 40 ng/ml, et la présence du fragment HindIII de 4400 pb est vérifiée sous UV.

Les réactions de séquences réalisées avec le kit "T7 sequencing" (Pharmacia TM) qui utilise la technique de terminaison de chaînes et la T7 DNA polymérase (cf PREPARATION 1) permet de valider l'intégrité du clonage.

### 3/ Echange des fragments KpnI-DraIII recombinants des clones ADEFG, ADEFH et BCDEFG par le fragment KpnI-DraIII sauvage du clone ADNc lalpha3.

Pour cela 500 ng du clone ADNc 1alpha3 sont digérées par les endonucléases de restriction KpnI (Promega, TM) et DraIII (Boehringer Mannhein) selon les recommandations des fabricants.

Suite à la digestion enzymatique, 4 µl de tampon stop (30% sucrose, 0,25% bleu de bromophénol) sont ajoutés à la solution d'ADN qui est analysée par électrophorèse sur gel d'agarose 0,8% à bas point de fusion SeaPlaque-GTG (FMC, Rockland, TM) à un voltage constant de 60 volts. Le tampon d'électophorèse est du TBE 0,5X (Tris borate 45 mM, EDTA (Acide éthylène-diamine-tétra-acétique) 1mM, pH 8). Après migration, le gel est coloré pendant 15 min dans une solution de TBE 0,5X contenant 40 ng/ml de bromure d 'éthidium.

La bande correspondant au fragment KpnI-DraIII est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) et centrifugée pendant 2 min à 10 000 g. La phase aqueuse est extraite au chloroforme/alcool isoamylique (rapport 24/1) et centrifugée quelques secondes à 10 000 g. La phase aqueuse est reprise, ajustée à une concentration finale en NaCl de 0,2 M, et précipitée 16 heures à -20°C après apport de deux volumes d'éthanol 100.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega, TM) sont analysés sur gel d'agarose type II (Sigma) 0,8%, ce qui nous a permis d'estimer la concentration de la solution purifiée du fragment KpnI-DraIII du clone ADNc 1alpha3 à 5 ng/µl.

Le plasmide utilisé pour cloner le fragment KpnI-DraIII du clone ADNc 1alpha3 est le clone pUC18minus KpnI-fragment ADEFG ou ADEFH ou BCDEFG digéré par les enzymes KpnI-DraIII. Ce clone comporte un gène de résistance à l'ampicilline.

Une quantité de 250 ng du plasmide pUC18minusKpnI-fragment ADEFG ou ADEFH ou BCDEFG est soumise à une digestion par les endonucléases de restriction KpnI et DraIII selon les recommandations du fabricant.

Suite à la digestion enzymatique, 4 µl de tampon stop (30% sucrose, 0,25% bleu de bromophénol) sont ajoutés à la solution d'ADN qui est analysée par électrophorèse sur gel d'agarose 0,8% à bas point de fusion SeaPlaque-GTG (FMC,TM) à un voltage constant de 60 volts. Le tampon d'électophorèse est du TBE 0,5X (Tris borate 45 mM, EDTA (Acide éthylène-diamine-tétra-acétique) 1mM, pH 8). Après migration, le gel est coloré pendant 15 min dans une solution de TBE 0,5X contenant 40 ng/ml de bromure d'éthidium.

La bande correspondant au vecteur pUCl8minusKpnI-fragment ADEFG ou ADEFH ou BCDEFG KpnI-DraIII est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) et centrifugée pendant 2 min à 10 000 g. La phase aqueuse est extraite au chloroforme/alcool isoamylique (rapport 24/1) et centrifugée quelques secondes à 10 000 g. La phase aqueuse est reprise, ajustée à une concentration finale en NaCl de 0,2 M, et précipitée 16 heures à -20°C après apport de deux volumes d'éthanol 100.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega, TM) sont analysés sur gel d'agarose type II (Sigma) 0,8%, ce qui nous a permis d'estimer la concentration de la solution purifiée du vecteur pUC18minusKpnI-fragment ADEFG ou ADEFH ou BCDEFG KpnI-DraIII à 8 ng/µl.

La ligation du fragment KpnI-DraIII du clone ADNc 1alpha3 au vecteur pUC18minusKpnI-fragment ADEFG ou ADEFH ou BCDEFG KpnI-DraIII est réalisée pendant 4 heures à température ambiante.

Pour la transformation, les bactéries compétentes utilisées sont les DH5alpha (Gibco BRL, Paris).

Plusieurs colonies blanches sont testées. Pour cela, elles sont individuellement déposées dans 3 ml de L-Broth supplémenté en ampicilline (50 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega, TM), qui permet de purifier de l'ADN plasmidien super enroulé. Cette manipulation est réalisée suivant le protocole fourni par le fabricant. Environ 10 µg d'ADN plasmidien est purifié grâce à ce kit, lorsque le plasmide utilisé est le pUC18.

Une fraction aliquote (2 µl sur les 60 obtenus) est soumise à une digestion enzymatique par l'endonucléase de restriction NheI, afin de vérifier le clonage du fragment KpnI-DraIII du clone ADNc lalpha3 au clone pUC18minusKpnI-fragment ADEFG ou ADEFH ou BCDEFG KpnI-DraIII. L'échange du fragment KpnI-DraIII recombinant par le fragment sauvage entraîne l'élimination d'un site de restriction NheI. Après électrophorèse, le gel est coloré pendant 15 min dans une solution de bromure d'éthidium à 40 ng/ml, et l'élimination du site de restriction NheI est vérifiée sous UV.

Les réactions de séquences réalisées avec le kit "T7 sequencing" (Pharmacia TM) qui utilise la technique de terminaison de chaînes et la T7 DNA polymérase (cf PREPARATION 1) permet de valider l'intégrité du clonage.

Les fragments obtenus sont ADEFGphe159, ADEFHphe159 et BCDEFGphe159.

### 4/ Obtention des constructions pBIOC21-collagène humain recombinant phe159

Cette étape consiste à passer les 3 constructions du plasmide pUC18minusKpnI au plasmide pBIOC21.

Le plasmide pBIOC21 est utilisé pour permettre l'expression du collagène recombinant dans les feuilles de tabac ou de colza. Il apporte les séquences régulatrices suivantes:
a) le promoteur constitutif double-35S (PD35S) du CaMV. Il correspond à une duplication de la séquence activant la transcription située en amont de l'élément TATA du promoteur 35S naturel.
b) la séquence terminatrice de transcription, le terminateur polyA 35S qui correspond à la séquence 3' non codante de la séquence du virus à ADN bicaténaire circulaire de la mosaïque du chou-fleur produisant le transcrit 35S.

Les constructions des différents plasmides via l'utilisation des techniques d'ADN recombinant dérivent de pBIOC4. Le plasmide binaire dérive de pGA442 (An, 1986). Le plasmide dérivant de pBIOC4 et contenant la cassette d'expression "PD35S-T35S" est le plasmide pBIOC21.

Les différents éléments permettant de reproduire ces constructions sont par exemple contenus dans la description de la demande de brevet WO9633277 qui est incorporée par référence.

Clonage des fragments (HindIII/HindIII) des constructions pUC18minusKpnI, dans le site de clonage HindIII du vecteur pBIOC21.

Environ 1 µg du clone pUC18minusKpnI-fragment ADEFGphe159 ADEFHphe159 ou BCDEFGphe159 sont digérés par l'endonucléase de restriction HindIII selon les recommandations du fabricant.

Suite à la digestion enzymatique, 4 µl de tampon stop (30% sucrose, 0,25% bleu de bromophénol) sont ajoutés à la solution d'ADN qui est analysée par électrophorèse sur gel d'agarose 0,8% à bas point de fusion SeaPlaque-GTG (FMC, Rockland) à un voltage constant de 60 volts. Le tampon d'électophorèse est du TBE 0,5X (Tris borate 45 mM, EDTA (Acide éthylène-diamine-tétra-acétique) 1mM, pH 8). Après migration, le gel est coloré pendant 15 min dans une solution de TBE 0,5X contenant 40 ng/ml de bromure d'éthidium.

La bande correspondant au fragment HindIII/HindIII de 4400 bp est découpée sous UV, déposée dans 350 µl d'une solution Tris 20 mM pH 8, EDTA 1 mM pH 8 et incubée pendant 5 min à 65°C. Après dissolution de l'agarose, l'échantillon est extrait par un volume de phénol et centrifugé pendant 5 min à 10 000 g. La phase aqueuse est reprise, extraite par un volume de phénol/chloroforme/alcool isoamylique (rapport 50/48/2) et centrifugée pendant 2 min à 10 000 g. La phase aqueuse est extraite au chloroforme/alcool isoamylique (rapport 24/1) et centrifugée quelques secondes à 10 000 g. La phase aqueuse est reprise, ajustée à une concentration finale en NaCl de 0,2 M, et précipitée 16 heures à -20°C après apport de deux volumes d'éthanol 100.

Après centrifugation à 4°C pendant 10 min à 12 000 g, le culot d'ADN est lavé par 1 ml d'éthanol 70, lyophilisé et repris dans 20 µl d'eau milli-Q stérile. Une fraction aliquote de 2 µl, ainsi que 500 ng des fragments HindIII-EcoRI du phage lambda (Promega, TM) sont analysés sur gel d'agarose type II (SigmaTM) 0,8%, ce qui nous a permis d'estimer la concentration de la solution purifiée du fragment HindIII/HindIII du clone pUC18minusKpnI-fragment ADEFGphe159 ou ADEFHphe159 ou BCDEFGphe159 à 25 ng/µl.

Le plasmide utilisé pour cloner le fragment HindIII/HindIII du clone pUC18minusKpnI-fragment ADEFGphe159 ou ADEFHphe159 ou BCDEFGphe159 est le clone pBIOC21 linéarisé par l'enzyme HindIII. Ce clone comporte un gène de résistance à la tétracycline.

Une quantité de 1 µg du plasmide pBIOC21 est soumise à une digestion par l'endonucléase de restriction HindIII. Cette digestion est réalisée dans un volume de 20 µl en présence de 2 µl de tampon B (Promega, TM), 1 µl d'enzyme HindIII (12 u/µl; Promega, TM), pendant 2 heures à 37°C. Après digestion, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) et centrifugée pendant 2 min à 10 000 g. La phase aqueuse est extraite au chloroforme/alcool isoamylique (rapport 24/1) et centrifugée quelques secondes à 10 000 g. La phase aqueuse est reprise, ajustée à une concentration finale en NaCl de 0,2 M, et précipitée 16 heures à -20°C après apport de deux volumes d'éthanol 100.

Après centrifugation à 4°C pendant 10 min à 12 000 g, le culot d'ADN est lavé par 1 ml d'éthanol 70, lyophilisé et repris dans 17 µl d'eau milli-Q stérile. A cette solution d'ADN sont ajoutés 2 µl de tampon 10X de la phosphatase alcaline d'intestins de veaux (CIP, Promega, TM), 1 µl de CIP (1 u/µl; Promega, TM). La déphosphorylation est réalisée pendant 1 heures à 37°C. Cette étape permettra d'éviter l'autoligation de ce clone pendant l'étape de ligation.

Après digestion, 80 µl de tampon TE (tris 10 mM, EDTA 1 mM, pH 8) sont ajoutés à la solution d'ADN qui est extraite par un volume de phénol/chloroforme/ alcool isoamylique (rapport 50/48/2) et centrifugée pendant 2 min à 10 000 g. La phase aqueuse est extraite au chloroforme/alcool isoamylique (rapport 24/1) et centrifugée quelques secondes à 10 000 g. La phase aqueuse est reprise, ajustée à une concentration finale en NaCl de 0,2 M, et précipitée 16 heures à -20°C après apport de deux volumes d'éthanol 100.

Après centrifugation à 4°C pendant 10 min à 12 000 g, le culot d'ADN est lavé par 1 ml d'éthanol 70, lyophilisé et repris dans 20 µl d'eau milli-Q stérile afin d'obtenir une solution à 40 ng/µl du plasmide pBIOC21 (HindIII, déphosphorylé).

La ligation du fragment HindIII/HindIII du clone pUC18minusKpnI-fragment ADEFGphe159 ou ADEFHphe159 ou BCDEFGphe159 au clone pBIOC21 (HindIII, déphosphorylé) est réalisée pendant 4 heures à température ambiante.

Pour la transformation, les bactéries compétentes utilisées sont les DH5alpha (Gibco BRL, Paris).

Plusieurs colonies blanches sont testées. Pour cela, elles sont individuellement déposées dans 3 ml de L-Broth supplémenté en tétracycline (40 µg/ml), et ces cultures sont incubées sous agitation pendant une nuit à 37°C.

Pour purifier l'ADN plasmidien, la culture est tout d'abord centrifugée à 1500 g pendant 10 min à 4°C, et le culot bactérien est traité avec le kit Wizard (Promega, TM), qui permet de purifier de l'ADN plasmidien super enroulé. Cette manipulation est réalisée suivant le protocole fourni par le fabricant. Environ 1-2 µg d'ADN plasmidien est purifié grâce à ce kit, lorsque le plasmide utilisé est le pBIOC21.

Une fraction aliquote (5 µl sur les 60 obtenus) est soumise à une digestion enzymatique par l'endonucléase de restriction KpnI, afin de vérifier l'orientation du clonage du fragment HindIII du clone pUC18minusKpnI-fragment ADEFGphe159 ou ADEFHphe159 ou BCDEFGphe159 au clone pBIOC21 (HindIII). L'apparition d'un fragment KpnI de 950 pb est spécifique d'une orientation correcte. Ce fragment KpnI de 950 pb résulte d'un site de restriction KpnI en position 2.8 kb du vecteur pBIOC21 et d'un site KpnI en position 137 de la séquence codant pour la chaîne pro[α1(I) humaine. Après électrophorèse, le gel est coloré pendant 15 min dans une solution de bromure d'éthidium à 40 ng/ml, et la présence du fragment KpnI de 950 bp est vérifiée sous UV.

Les réactions de séquences réalisées avec le kit "T7 sequencing" (Pharmacia TM) qui utilise la technique de terminaison de chaînes et la T7 DNA polymérase (cf PREPARATION 1) permet de valider l'intégrité du clonage.

Les constructions pBIOC21-collagène ainsi obtenues seront nommées ci après:
- pBIOC708 pour la construction contenant le fragment ADEFGphe159
- pBIOC709 pour la construction contenant le fragment ADEFHphe159
- pBIOC710 pour la construction contenant le fragment BCDEFHphe159

### EXEMPLE 2

### Construction de gènes chimériques codant pour la protéine recombinante du collagène humain et permettant une expression dans les semences de mais.

L'expression constitutive dans les semences de maïs du cDNA codant la chaîne pro-[α1(I) du collagène humain a nécessité les séquences régulatrices suivantes :
1. le promoteur du gène de zéine de maïs (Pzéine) contenu dans le plasmide p63. Il permet une expression dans l'albumen des semences de maïs ;
2. la séquence terminatrice de transcription, terminateur polyA NOS, qui correspond à la région en 3' non codante du gène de la nopaline synthase du plasmide Ti d'*Agrobacterium tumefaciens* souche à nopaline.

Les différents éléments permettant de reproduire ces constructions sont par exemple contenus dans la description de la demande de brevet WO9633277 qui est incorporé par référence.

A partir des plasmides, les fragments ADEFG, ADEFH, BDEFG, et BCDEFG ont été isolés par digestion par HindIII, purifiés par électrophorèse sur gel d'agarose 0,8%, électroélués, soumis à la précipitation alcoolique et séchés. Puis ces fragments d'ADN ont été traités par l'enzyme de Klenow (New England Biolabs TM) selon les recommandations du fabricant, soumis à l'extraction phénol-chloroforme, à la précipitation alcoolique et repris dans 10 µl H20.

Le plasmide p63 a été doublement digéré par SacI et BamHI, purifié par électrophorèse sur gel d'agarose 0,8%, électroélué, soumis à la précipitation alcoolique et séché. Puis il a été traité par l'enzyme T4 polymérase (New England Biolabs TM) et déphosphorylé par l'enzyme phosphatase alcaline d'intestin de veau (Boehringer Mannheim) selon les recommandations des fabricants.

Chaque fragment ADEFG, ADEFH, BDEFG, et BCDEFG obtenu comme décrit ci-dessus, a été ligué au plasmide p63 traité comme décrit ci-dessus.

La ligation et la transformation des bactéries compétentes *Escherichia coli* souche DH5a ont été réalisées selon les procédés standard. Les plasmides obtenus sont appelés respectivement pBIOC700, pBIOC701, pBIOC702 et pBIOC703 et contiennent respectivement les fragments ADEFG, ADEFH, BDEFG et BCDEFG.

Les acides aminés Asn et Phe, en positions 158 et 159 de la chaîne proα 1(I) humaine, ont été restaurés dans les clones ADEFG, ADEFH et BCDEFG pour obtenir ADEFGphe159, ADEFHphe159 et BCDEFHphe159, respectivement. Cette modification est décrite dans l'exemple 1.

A partir des plasmides, les fragments ADEFGphe159, ADEFHphe159 et BCDEFHphe159 ont été isolés par digestion par HindIII, purifiés par électrophorèse sur gel d'agarose 0,8%, électroélués, soumis à la précipitation alcoolique et repris dans 10µl H20. Ces fragments ainsi préparés ont été ligués au plasmide p63 traité comme décrit ci-dessus. Les plasmides obtenus sont appelés pBIOC711, pBIOC712 et pBIOC713 et contiennent les fragments ADEFGphe159, ADEFHphe159 et BCDEFHphe159, respectivement.

### EXEMPLE 3

### Obtention de plants de colza transgéniques.

Les graines de colza de printemps *(Brassica napus* cv WESTAR ou lignées Limagrain) sont désinfectées pendant 40 minutes dans une solution de Domestos TM à 15%. Après 4 rinçages à l'eau stérile, les graines sont mises à germer, à raison de 20 graines par pot de 7 cm de diamètre et 10 cm de haut, sur du milieu minéral de Murashige et Skoog (Sigma M 5519) avec 30g/l de saccharose et solidifié avec de l'agargel à 5g/l. Ces pots sont placés dans une chambre de culture à 26°C avec une photopériode de 16h/8h et sous une intensité lumineuse de l'ordre de 80 µE m⁻² S⁻¹.

Après 5 jours de germination, les cotylédons sont prélevés stérilement en coupant chaque pétiole environ 1 mm au-dessus du noeud cotylédonaire.

Parallèlement une préculture d'*Agrobacterium tumefaciens* souche LBA4404, contenant les plasmides, est réalisée en erlenmeyer de 50 ml, pendant 36 h à 28°C dans 10 ml de milieu bactérien 2YT complémenté avec les antibiotiques utiles à la sélection de la souche utilisée.

Cette préculture sert à ensemencer à 1% une nouvelle culture bactérienne effectuée dans les mêmes conditions. Au bout de 14 h la culture est centrifugée 15 min à 3000 g et les bactéries sont reprises dans un volume équivalent de milieu de germination liquide. Cette suspension est distribuée dans des boîtes de pétri de 5 cm de diamètre à raison de 5 ml/boîte.

L'extrémité sectionnée du pétiole est immergée quelques secondes dans la solution d'agrobactéries ainsi préparées, puis le pétiole est enfoncé de quelques millimètres dans le milieu de régénération. Ce milieu a la même composition de base que le milieu de germination avec en plus 4 mg/l de benzyl-amino-purine (BAP), phytohormone favorisant la néoformation de bourgeons. Dix explants (cotylédon avec pétiole) sont mis en culture par boîte de pétri de 9 cm de diamètre .

Après 2 jours de coculture dans les mêmes conditions environnementales que les germinations, les explants sont repiqués dans des boîtes phytatray (Sigma TM, référence P1552) contenant le milieu précédent complémenté avec un agent sélectif: 45 mg/l de sulfate de kanamycine et un bactériostatique: mélange de 1/6 (en poids) de sel de potassium d'acide clavulanique et de 5/6 sel de sodium d'amoxicilline (Augmentin TM injectable) à raison de 600 mg/l.

Deux fois de suite, à 3 semaines d'intervalle, les explants sont repiqués stérilement sur du milieu neuf dans les mêmes conditions.

Les bourgeons verts apparus à la fin du deuxième ou du troisième repiquage sont séparés de l'explant et mis en culture individuellement dans des pots transparents de 5 cm de diamètre et de 10 cm de haut contenant un milieu identique au précédent mais dépourvu de BAP. Après 3 semaines de culture la tige du bourgeon transformé est sectionnée et le bourgeon est repiqué dans un pot de milieu frais. Au bout de trois à quatre semaines les racines sont assez développées pour permettre l'acclimatation de la plantule dans un phytotron. Les bourgeons qui ne sont pas verts ou enracinés sont éliminés. Ces plantules sont alors transplantées dans des godets de 7 cm de côté remplis de terreau (norme NF U44551: 40% tourbe brune, 30% bruyère tamisée et 30% sable) saturé en eau. Après deux semaines d'acclimatation en phytotron (température 21°C, photopériode 16h/8h et 84% d'humidité relative), les plantules sont rempotées dans des pots de 12 cm de diamètre remplis du même terreau enrichi en engrais retard puis transportées en serre (classe S2) régulée à 18°C, avec deux arrosages quotidien de 2 minutes à l'eau.

Lorsque les siliques sont arrivées à maturité, elles sont récoltées, séchées, puis battues. Les graines obtenues servent au dosage de l'activité biochimique. La sélection de la descendance transgénique se fait par germination sur un milieu contenant du sulfate de kanamycine à raison de 100 à 150 mg/l (selon les génotypes). Les conditions opératoires sont identiques à celles décrites ci-dessus à ceci près que les germinations sont effectuées en tubes de verre avec une seule graine par tube. Seules les plantules développant des racines secondaires les trois premières semaines sont acclimatées en phytotron avant d'être passées en serre.

### EXEMPLE 4

### Obtention de plants transgéniques de tabac

Les plants de tabac utilisées pour les expériences de transformation (*Nicotiana tabacum* var. *Xanthi* NC et PBD6) sont cultivées *in vitro* sur le milieu de base de Murashige et Skoog (1962) additionné des vitamines de Gamborg *et al*. (1968, Sigma référence M0404) de saccharose à 20 g/l et d'agar à 8 g/l. Le pH du milieu est ajusté à 5,8 avec une solution de potasse avant autoclavage à 120°C pendant 20 min. Les plantules de tabac sont repiquées par bouture des entre-noeuds tous les 30 jours sur ce milieu de multiplication MS20.

Toutes les cultures *in vitro* sont réalisées en enceinte climatisée, dans les conditions définies ci-dessous:
- intensité lumineuse de 30 µE.m⁻².S⁻¹; photopériode de 16h;
- thermopériode de 26°C le jour, 24°C la nuit.

La technique de transformation utilisée est dérivée de celle de Horsch *et al*. (1985).

Une préculture d'*Agrobacterium tumefaciens* souche LBA4404 contenant les plasmides est réalisée durant 48h à 28°C sous agitation, dans du milieu LB additionné des antibiotiques adéquats (rifampicine et tétracycline). La préculture est ensuite diluée au 50ème dans le même milieu et cultivée dans les mêmes conditions. Après une nuit, la culture est centrifugée (10 min., 3000 g), les bactéries sont reprises dans un volume équivalent de milieu MS30 (30 g/l saccharose) liquide et cette suspension est diluée au 10ème.

Des explants d'environ 1 cm2 sont découpés à partir des feuilles des plantules décrites ci-dessus. Ils sont ensuite mis au contact de la suspension bactérienne pendant 1h, puis séchés rapidement sur papier filtre et placés sur un milieu de coculture (MS30 solide).

Après 2 jours, les explants sont transférés en boîtes de Pétri sur le milieu de régénération MS30, contenant un agent sélectif, la kanamycine (200 mg/l), un bactériostatique, l'augmentin (400 mg/l) et les hormones nécessaires à l'induction de bourgeons (BAP, 1 mg/l et ANA, 0,1 mg/l). Un repiquage des explants est effectué sur le même milieu après 2 semaines de culture. Après 2 semaines supplémentaires, les bourgeons sont repiqués en boîtes de Pétri sur le milieu de développement composé du milieu MS20 additionné de kanamycine et d'augmentin. Après 15 jours, les bourgeons sont repiqués de moitié. L'enracinement prend environ 20 jours, au terme desquels les plantules peuvent être clonées par bouture d'entre-noeuds ou sorties en serre.

### EXEMPLE 5

### Obtention de plantes de maïs transgéniques.

### a) Obtention et utilisation de cal de maïs comme cible pour la transformation génétique.

La transformation génétique du maïs, quelle que soit la méthode employée (électroporation; *Agrobacterium,* microfibres, canon à particules), requiert généralement l'utilisation de cellules indifférenciées en divisions rapides ayant conservé une aptitude à la régénération de plantes entières. Ce type de cellules compose le cal friable embryogène (dit de type II) de maïs.

Ces cals sont obtenus à partir d'embryons immatures de génotype H1 II ou (A188 x B73) selon la méthode et sur les milieux décrits par Armstrong (1994). Les cals ainsi obtenus sont multipliés et maintenus par repiquages successifs tous les quinze jours sur le milieu d'initiation.

Des plantules sont ensuite régénérées à partir de ces cals en modifiant l'équilibre hormonal et osmotique des cellules selon la méthode décrite par Vain et al (1989). Ces plantes sont ensuite acclimatées en serre où elles peuvent être croisées ou autofécondées.

### b) Utilisation du canon à particules pour la transformation génétique du maïs.

Le paragraphe précédent décrit l'obtention et la régénération des lignées cellulaires nécessaires à la transformation ; on décrit ici une méthode de transformation génétique conduisant à l'intégration stable des gènes modifiés dans le génome de la plante. Cette méthode repose sur l'utilisation d'un canon à; les cellules cibles sont des fragments de cals décrits dans le paragraphe 1. Ces fragments d'une surface de 10 à 20 mm² ont été disposés, 4 h avant bombardement, à raison de 16 fragments par boite au centre d'un boite de petri contenant un milieu de culture identique au milieu d'initiation, additionné de 0,2 M de mannitol + 0,2 M de sorbitol. Les plasmides portant les gènes à introduire sont purifiés sur colonne Qiagen TM, en suivant les instructions du fabricant. Ils sont ensuite précipités sur des particules de tungsten (M10) en suivant le protocole décrit par Klein (Nature (1987) 327:70-73). Les particules ainsi enrobées sont projetées vers les cellules cibles à l'aide du canon .

Les boites de cals ainsi bombardées sont ensuite scellées , puis cultivées à l'obscurité à 27°C. Le premier repiquage a lieu 24 h après, puis tous les quinze jours pendant 3 mois sur milieu identique au milieu d'initiation additionné d'un agent sélectif dont la nature et la concentration peuvent varier selon le gène utilisé (voir paragraphe 3). Les agents sélectifs utilisables consistent généralement en composés actifs de certains herbicides (Basta, Round up) ou certains antibiotiques (Hygromycine, Kanamycine...).

On obtient après 3 mois ou parfois plus tôt, des cals dont la croissance n'est pas inhibée par l'agent sélection, habituellement et majoritairement composés de cellules résultant de la division d'une cellule ayant intégré dans son patrimoine génétique une ou plusieurs copies du gène de sélection. La fréquence d'obtention de tels cals est d'environ 0,8 cal par boite bombardée.

Ces cals sont identifiés, individualisés, amplifiés puis cultivés de façon à régénérer des plantules (cf. paragraphe a). Afin d'éviter toute interférence avec des cellules non transformées toutes ces opérations sont menées sur des milieux de culture contenant l'agent sélectif.

Les plantes ainsi régénérées sont acclimatées puis cultivées en serre où elles peuvent être croisées ou autofécondées.

### EXEMPLE 6 : EXTRACTION ET CRIBLAGE

### Extraction à partir de plants de tabac génétiquement transformés selon l'invention

45 plants numérotés de 1 à 22 lorsqu'ils ont été transfectés avec la construction pBIOC707 (comprenant le N-propeptide, la triple hélice majeure et le C-propeptide) de 23 à 45 lorsqu'ils ont été transfectés par la construction pBIOC706 (comprenant la triple hélice majeure et le C-propeptide) sont coupés, pesés et congelés dans l'azote liquide. Les différents plants sont alors broyés dans l'azote liquide à l'aide d'un homogénéiseur pendant 2 min afin d'obtenir une poudre fine puis stockés jusqu'à leur utilisation à -20°C.

### Extraction par l'acide acétique:

La poudre obtenue pour chaque plant y compris les plants témoins non-transformés, est reprise par une solution d'acide acétique 0.5M contenant 200 mM NaCl et des inhibiteurs de protéases :1mM phényl méthyl sulfonide (PMSF), 1 mM N-ethyl maléimide (NEM), 2.5 mM acide tetra-acétique diamine éthylène (EDTA), à raison de 1 gramme de poudre pour 4 ml de solution. Le mélange est agité à 4°C pendant 60 heures. Après centrifugation de chaque échantillon à 20 000 g (rotor 12148, Sigma TM) pendant 30 minutes à 4°C. Les culots sont lavés 2 fois en eau distillée et stockés à -20°C. Une fraction des surnageants (100 µl) est prélevée pour réaliser un dosage protéique sur l'extrait total. Le reste des surnageants est stockés à -20°C.

### Dosage protéique de l'extrait total:

Les fractions prélevées 100µl pour l'extrait acido-soluble et 20µl pour l'extrait en tampon neutre sont dosés par l'utilisation d'une solution de Coomassie prête à l'emploi. Le dosage est réalisé en plaque multipuits et la lecture est faite sur un lecteur de plaques (SLT Lab Instruments, Austria) à une longueur d'onde de 620nm. Une gamme étalon est réalisée a partir d'une solution de BSA à 100µg/ml de 0 à 10 µg/puits: 0, 10, 20, 30, 40, 50, 60, 70, 80, 90, 100µl sont déposés dans chaque puits et complétés lorsque nécessaire à 100µl final. Les différents échantillons sont également déposés dans les puits et complétés lorsque nécessaire à 100µl dans le tampon d'extraction. Les références se font par 100µl d'eau distillée pour la gamme étalon et 100µl de tampon d'extraction pour les échantillons. 100µl de réactif sont alors ajoutés dans chaque puits, la lecture est immédiate. Les valeurs obtenues pour chacune des références seront déduites respectivement des valeurs obtenues pour la gamme étalon et celles des échantillons. Une courbe étalon est tracée et la concentration en protéine des échantillons est déduite à partir de cette courbe.

### Criblage des positifs:

Le criblage s'opère de deux façons :
1. Criblage des plants (après broyage et extraction) par gel d'électrophorèse suivi d'un immunotransfert. Le collagène témoin utilisé sera du collagène I extrait de tissus humain ou bovin par l'acide acétique et purifié par précipitation par les sels. La forme moléculaire du collagène I la plus répandue dans les tissus est l'hétérotrimère contenant deux chaînes α1 et α 2: [α1(I) 2α2(I)]. Dans ce cas, la chaîne d'intérêt α1 migre légèrement au dessus de 116KDa, elle ne contient que la triple hélice majeure et les télopeptides, les N et C-propeptides ont été clivés lors de la maturation du collagène et ne sont donc plus présents.
**2.** Criblage des positifs par immunofluorescence indirecte sur coupes congelées des différents plants.

Dans les 2 cas, l'anticorps utilisé est un anticorps polyclonal fait chez le lapin spécifique du collagène I, il reconnaît le collagène I humain et bovin et indifféremment les chaînes α1(I) et α2(I) (référence 20121, Institut Pasteur, Lyon ); Un autre anticorps peut être également utilisé: il s'agit d'un anticorps monoclonal Sp1D8 (Hybridoma Bank, USA) dirigé contre le site de clivage du N-propeptide de type I. Ces deux anticorps peuvent être utilisés sur du collagène I dénaturé (après gel d'électrophorèse et transfert des protéines) et sur du collagène natif (en immunofluorescence).

### 1. Criblage après analyse des plants par électrophorèse suivie d'un immunotransfert.

### Méthode analytique

Le criblage est réalisé sur les extraits en acide acétique. Les surnageants et culots sont analysés.

### Electrophorèse:

### Les surnageants:

500µl des surnageants, soit 15 à 25 µg de protéines totales, sont précipités par l'addition de 50 µl d'acide trichloroacétique 100% pendant 30 minutes à température ambiante. Les échantillons sont centrifugés à 20 000g pendant 15 minutes à 4°C, les culots sont lavés 2 fois en éthanol 100% froid. Les précipités sont alors repris par 20µl de tampon'porteur (Tris-HCl 60mM pH 6,8, contenant 2% sodium dodecyl sulfate, 10% glycérol, 0.002% de bleu de bromophénol) en présence d'un agent réducteur le dithiothreitol (DTT) à 10 mM final, puis dénaturés pendant 3 minutes à 100°C.

### Les culots:

Les culots, lavés en eau distillée pour éviter la présence d'acide et de sels provenant du tampon d'extraction, sont repris dans 50µl d'eau distillée. 10µl de la suspension sont prélevés auxquels on ajoute 20µl de tampon porteur concentré 3 fois (Tris-HCl 180mM pH 6,8, contenant 6% sodium dodecyl sulfate, 30% glycérol, 0.006% de bleu de bromophénol) en présence d'un agent réducteur le dithiothreitol (DTT) à 10 mM final, puis dénaturés pendant 3 minutes à 100°C.

Les différents échantillons sont déposés sur un gel d'acrylamide à 6% dans une solution de Tris-HCl 0,4M pH 8,8 contenant 0.1% de sodium dodecyl sulfate, avec un gel de concentration à 4% dans une solution Tris-HCl 0,4M pH6.8 contenant 0.1% sodium dodecyl sulfate. Sur chaque gel, trois puits sont réservés pour respectivement les poids moléculaires standards (6H Sigma TM), le collagène I témoin (bovin ou humain, 3µg/puits), et l'échantillon correspondant au plant non transformé. La migration s'effectue dans un tampon de migration Tris 0.025M-glycine 0.2M contenant 0.1% de sodium dodecyl sulfate sous une tension de 100 volts dans le gel de concentration, augmentée à 200 volts pour le gel de séparation.

### Immunotransfert:

Après migration, les gels sont apposés à une membrane poly(vinylidene difluoride) (Immobilon-P, Millipore TM) préalablement mouillée par du méthanol 100% puis imbibée dans le tampon de transfert CAPS (10mM cyclohexylamino-1-propanesulfonic acid, pH11, méthanol 5%). L'ensemble gel plus membrane est placé dans un appareil de transfert (Biorad TM) et soumis à une tension de 60 volts pendant 16 heures, réfrigéré par un système de circulation d'eau froide.

Afin de vérifier l'efficacité du transfert, les gels sont colorés en bleu de Coomassie R-250 (solution à 0.2% dans le méthanol 40% et l'acide 10%) pendant 30 minutes. Ils sont ensuite décolorés sélectivement dans une solution de méthanol 15% et d'acide acétique 7,5% et les protéines non transférées ou partiellement transférées sont repérées par la présence de bandes restant sur le gel. Par ailleurs, les membranes sont colorées au Ponceau S (0,2% dans l'acide acétique 1%) pendant 10 minutes puis décolorées dans l'eau distillée. Les différentes protéines ayant transférées correctement sont repérées, la piste correspondant aux standards de poids moléculaire excisée, séchée à l'air et conservée entre deux feuilles de papier filtre. Le reste des membranes est complètement décoloré.

Les membranes sont ensuite saturées pendant une heure dans du lait en poudre écrémé 10% puis brièvement rincées en Tris-HCl 25mM pH 7,4, NaCl 150mM, KCl 2.5mM (TBS). Les membranes sont alors incubées avec les anticorps polyclonaux dirigés contre la triple hélice majeure du collagène I bovin faits chez le lapin dilués au 1:400 dans le tampon TBS contenant 0.05% Tween 20 (TTBS), 1% albumine sérique bovine, pendant 2 heures à température ambiante. Après incubation avec les anticorps, les membranes sont rincées 6 fois 5 minutes en TTBS, puis incubées avec le conjugué dilué au 1:2000 (anti-IgG de lapin fait chez le porc, couplées à la phosphatase alkaline, Dako TM, Danemark) pendant 1 heure à température ambiante. Les membrane sont à nouveau rincées en TTBS (6 fois 5 minutes) puis révélées à l'aide des réactifs du kit APColor (Biorad TM). Les pistes présentant une ou plusieurs bandes aux poids moléculaires attendus correspondent aux positifs, les numéros des plants correspondants sont répertoriés. Tous les positifs sont soumis à un deuxième criblage (gel + immunotransfert).

### Résultats:

pBIOC706
- La construction pBIOC706 présente 40% de positifs sous la forme d'une bande majoritaire migrant au niveau de la chaîne α1(I) témoin. Ce résultat suggère que le C-propeptide (environ 30 Kda) de la chaîne α1(I) recombinante a été clivé. Les pistes correspondant au plant témoin non transformé ne présentent aucune bande révélée par l'anticorps anti-collagène I.
- les plants 23, 25, 28, 32, 36, 38, 39, 40, 45 sont identifiés comme positifs, les plants 40 et 45 sont déterminés comme étant les plus productifs par leur présence dans les surnageants et leur abondance dans le culots.

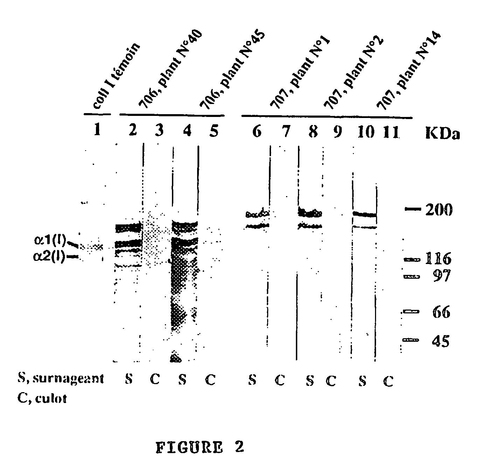

pBIOC707
- La construction pBIOC707 présente 27% de positifs sous la forme d'une bande majoritaire migrant au dessus de la chaîne alphal(I) témoin correspondant à un poids moléculaire d'environ 140KDa. La masse moléculaire attendue pour la chaîne recombinante entière étant d'environ 160KDa, ce résultat suggère que le N-propeptide (environ 20KDa) ou le C-propeptide (environ 30KDa) de la chaîne α1(I) recombinante a été clivé. Les pistes correspondant au plant témoin non transformé ne présentent aucune bande révélée par l'anticorps anti-collagène I.
- les plants 1, 2, 14, 16, 17, 19 sont identifiés comme positifs, les plants 1, 2 et 14 sont déterminés comme étant les plus productifs par leur présence dans les surnageants et leur abondance dans le culots (fig. 2).

### 2. Immunofluorescence:

### Méthode analytique

Les feuilles des plants sont enrobés dans un milieu d'inclusion à froid (Tissue-Tek, Miles, USA) et sont congelés sur la platine refroidie à -30°C du cryostat.

Des lames histologiques traitées par l'aminoalkylsilane sont préparées selon le protocole suivant :
- Immersion dans une solution de 3-aminopropyltriethoxysilanel 2% dans l'acétone, 5 secondes
- Lavage à l'acétone, 2X 1 minutes
- Lavage à l'eau distillée
- Séchage pendant une nuit à 42°C.

Des coupes à congélation de 7µm sont réalisées pour tous les plants à l'aide d'un cryostat et déposées sur les lames traitées. Les lames sont alors traitées comme suit:
- Rinçage des lames dans un tampon phosphate (PBS pour phosphate buffer saline, 137 mM NaCl, 2,7mM KCl, 10mM sodium phosphate, pH7,2).
- Blocage des sites aspécifiques par une incubation des lames dans du PBS contenant 1% d'albumine sérique bovine (BSA)
- Incubation dans les anticorps anti-collagène I (référence 20121, Institut Pasteur, Lyon) dilués au 1:50 dans du PBS-BSA 1% pendant 2 heures à température ambiante. Des lames contrôles sont réalisées où les anticorps anti-collagène I sont remplacés par du PBS seul ou par un anticorps polyclonal spécifique du collagène IV.
- Rinçage dans du PBS, 3 fois 10 minutes
- Incubation dans le conjugué (anti-IgG de lapin fait chez la chèvre couplés à l'isothiocyanate de fluoresceine, Biosys TM, France) dilué au 1:300 dans du PBS-BSA 1% pendant 1 heure à température ambiante. Pendant l'incubation et jusqu'à l'observation les lames sont placées à l'obscurité.
- Rinçage au PBS, 3 fois 10 minutes
- Traitement des lames à l'Eriochrome Black T 0.3% dans du PBS pour masquer l'autofluorescence des échantillons, pendant 1 minute;
- Rinçage intensif au PBS
- Monter les lamelles sur les lames avec une goutte de PBS-glycérol 1:1.

L'observation se fait sur un microscope à epifluorescence Zeiss TM Universal équipé du filtre Zeiss TM exciter BP 430-490.

### Résultats

L'observation des lames nous ont permis les conclusions suivantes :
- les amas formés par les chloroplastes auto-fluorescent en rouge mais ne gènent pas l'observation de fluorescence jaune-vert des plants positifs.
- Une auto-fluorescence jaune-vert est également observée sur des structures particulières de la plante et bien délimitées (canaux).
   Les plants positifs sont cependant facilement identifiables par la présence de petits amas trés colorés dans les cellules. On remarque également dans les plants correspondant à la construction pBIOC706 (α1 + C-pro) un réseau filamenteux diffus qui apparait localisé dans la partie extracellulaire de la feuille.
- On note une parfaite correspondance entre les plants positifs déterminés par la méthode gel+transfert et par immunofluoresecence surtout pour les plus positifs c'est-à-dire 1, 2, 14, 40 et 45.

### Extraction en tampon neutre:

Afin d 'améliorer l'extraction, une extraction en milieu neutre également utilisée pour le collagène tissulaire et le procollagène (molécule de collagène possédant encore les N- et C-propeptides) est réalisée sur les culots des positifs de la précédente extraction: 1, 2, 14, 40 et 45.

Les résidus issus de l'extraction en milieu acide de chaque plant y compris les plants témoins non transformés sont lavés à l'eau distillée deux fois avant d'être repris par un tampon Tris-HCl 50 mM pH 7.4 contenant 150 mM NaCl et des inhibiteurs de protéases :1mM phényl méthyl sulfonide (PMSF), 1 mM N-ethyl maléimide (NEM), 2.5 mM acide tetra-acetique diamine ethylène (EDTA), à raison de 1 gramme de poudre de départ pour 4 ml de solution. Le mélange est agité à 4°C pendant 60 heures. Après centrifugation de chaque échantillon à 20 000 g (rotor 12148, Sigma TM) pendant 30 minutes à 4°C. Les culots sont lavés 2 fois en eau distillée et stockés à - 20°C. Une fraction des surnageants (20 µl) est prélevée pour réaliser un dosage protéique sur l'extrait total. Le reste des surnageants est stocké à -20°C.

### Méthode analytique

cf ci-dessus

### Résultats

### 1. Les pistes correspondant aux plants transformés avec la construction pBIOC706:

On retrouve pour les plants 40 et 45 la bande majeure observée dans les surnageants après extraction en milieu acide migrant au niveau de la chaîne α1 du collagène I témoin, mais aussi une autre bande d'intensité égale migrant au environ de 140 KDa. Ce résultat témoigne de la présence d'une chaîne α1(I) recombinante complète correspondant à la construction pBIOC706. En effet la bande supérieure correspond à la masse moléculaire attendue pour la chaîne α1(I) recombinante comprenant le C-propeptide.

### 2. Les pistes correspondant aux plants transformés avec la construction pBIOC707:

On retrouve pour chaque plant (1, 2 et 14) la bande majeure observée dans les surnageants après extraction en milieu acide migrant au environ de 140 KDa, mais aussi une autre bande d'intensité égale migrant au environ de 160 KDa. Ce résultat témoigne de la présence d'une chaîne α1(I) recombinante complète pour la construction pBIOC707. En effet la bande supérieure correspond à la masse moléculaire attendue pour la chaîne α1(I) recombinante comprenant les N- et C-propeptides.

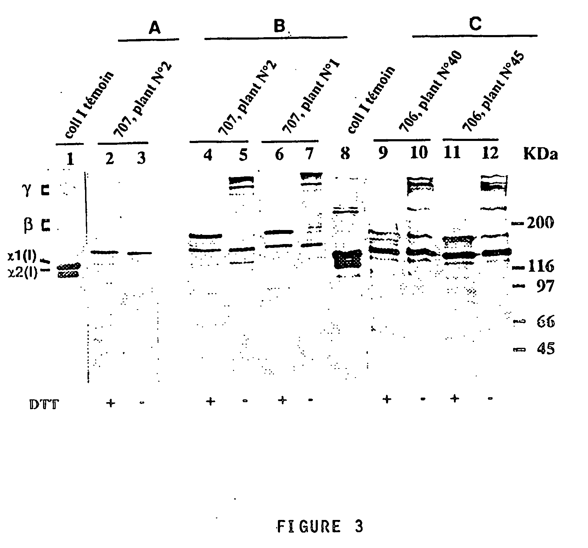

Pour les deux constructions pBIOC706 et pBIOC707, les pistes correspondant aux culots après extraction et centrifugation des échantillons ne présentent plus aucune bande révélée par l'anticorps anti-collagène I, démontrant l'éfficacité de l'extraction en pH neutre (fig3).

### EXEMPLE 7 : CONFORMITE DU COLLAGENE

### Détermination de la présence des propeptides N- et C-terminaux dans les chaînes α1(I) recombinantes extraites en milieu acide:

L'extraction des plants transformés avec la construction pBIOC707 en pH acide ne permet l'extraction que d'une seule bande migrant légèrement au dessus de la bande α1(I) témoin. La masse moléculaire attendue pour le polypeptide recombinant entier étant de 160KDa, l'hypothèse d'un clivage lors de la maturation de la chaîne α1(I) recombinante est envisagée. Il en est de même pour la construction pBIOC706 pour laquelle une bande majoritaire est observée au niveau de la bande α1(I) témoin. Cette migration ne peut s'expliquer que par un clivage d'une partie de la chaîne recombinante. Afin d'appuyer notre hypothèse selon laquelle les bandes majoritaires obtenues pour les constructions pBIOC706 et pBIOC707 correspondent aux chaînes α1(I) recombinantes dont le C-propeptide aurait été clivé au cours de la production des chaînes collagèniques, un gel d'électrophorèse en milieu réducteur ou non sera réalisé sur un plant positif de chaque construction et analysé après immunotransfert. En effet le N-propeptide lorsqu'il est correctement replié comprend des pontage intrachaînes entre les cystéines alors que les cystéines du C-propeptide sont pontées interchaînes, processus qui initie la formation de la triple hélice.

### Méthode analytique

fois 500µl des plants 2 (positif de la construction pBIOC707) et 40 (positif de la construction pBIOC706) sont précipités par l'addition de 50 µl d'acide trichloroacétique 100% (TCA) pendant 30 minutes à température ambiante. Les échantillons sont centrifugés à 20 000g pendant 15 minutes à 4°C (rotor 12148, Sigma TM), les culots sont lavés 2 fois en éthanol 100% froid. Les précipités sont alors repris par 20µl de tampon porteur (Tris-HCl 60mM pH 6,8, contenant 2% sodium dodecyl sulfate, 10% glycérol, 0.002% de bleu de bromophénol) en alternant une piste en présence de DTT 10 mM final et une piste sans DTT, puis dénaturés pendant 3 minutes à 100°C. Les gels sont analysés après immunotransfert.

### Résultats

Après transfert et révélation avec les anticorps anti-collagène I (référence 20121, Institut Pasteur, Lyon), on observe que la chaîne recombinante du plant 2 migre en présence d'agent réducteur légèrement plus lentement que la chaîne recombinante en l'absence d'agent réducteur. Cette faible différence de migration n'est pas observée pour la chaîne recombinante du plant 40 (construit délété en N-propeptide). On en déduit que cette différence de migration est dûe au correct repliement du N-propeptide permettant les pontages intrachaîne des cystéines. Le C-propeptide serait donc absent des deux polypeptides recombinants (fig. 4, partie A)

### Présence d'un pontage interchaine entre les cystéines du C-propeptide:

Un gel d'electrophorèse en présence ou non d'agent réducteur est réalisé sur les échantillons extraits en pH neutre pour lesquels on obtient deux bandes majoritaires dont la supérieure doit comprendre le C-propeptide. En effet , cette bande supérieure migre à la masse moléculaire attendue pour le polypeptide non clivé qu'il s'agisse de la construction pBIOC706 (triple hélice majeure + C-propeptide) ou pBIOC707 (N-propeptide+ triple hélice majeure+ C-propeptide) soit 140 KDa et 160 KDa respectivement. Dans le cas d'un pontage entre les cystéines de trois chaînes, étape nécessaire à l'initiation de la formation de la triple hélice, la migration de la bande supérieure non réduite devrait migrer au niveau des bandes γ du collagène I témoin correspondant à la migration de trois chaînes non dissociées (environ 300 KDa).

### Méthode analytique

Les essais ont été réalisés sur les plants 1, 2 et 14 (construction pBIOC707) et les plants 40 et 45 (construction pBIOC706) extraits en tampon neutre. 2 fois 150 µl de chaque échantillon soit 7,5µg à 11µg de protéines totales par prise d'essai, sont prélevés et précipités par 15µl d'acide trichloroacétique 100% (TCA) comme indiqué précédemment. Une série d'échantillon est alors reprise par 20µl de tampon porteur en l'absence d'agent réducteur, l'autre série par 20µl de tampon porteur en présence de 10mM de DTT. Sur chaque gel deux puits sont réservés pour respectivement les poids moléculaires standard (6H Sigma TM), le collagène I témoin (bovin ou humain, 3µg/puits). La migration s'effectue dans un tampon de migration Tris 0.025M-glycine0.2M contenant 0.1% de sodium dodecyl sulfate sous une tension de 100 volts dans le gel de concentration, augmentée à 200 volts pour le gel de séparation.

### Immunotransfert:

cf ci dessus dans la partie Criblage par analyse des plants par électrophorèse suivie d'un immunotransfert.

### Résultats

### 1. Les pistes correspondant aux plants transformés avec la construction pBIOC707:

En présence de l'agent réducteur, on retrouve pour chaque plant 1, 2 et 14, deux bandes majeures l'une migrant aux environs de 140 KDa, l'autre aux environs de 160 KDa. En absence d'agent réducteur, la bande à 140KDa migre légèrement plus rapidement (pontage intrachaîne des cystéines contenues dans le N-pro lui conférerant une structure plus compacte et donc migrant plus vite dans le gel que la forme réduite non-repliée). La bande à 160KDa en l'absence d'agent réducteur disparait et apparait une bande majoritaire migrant aux alentours de 300 KDa (légèrement au dessus des bandes γ du collagène I témoin). Ce résultat témoigne de la présence de pontage interchaîne entre les cystéines du C-propeptide de la chaîne α1(I) recombinante (fig. 4 partie B).

### 2. Les pistes correspondant aux plants transformés avec la construction pBIOC706:

En présence de l'agent réducteur, on retrouve pour les plants 40 et 45, deux bandes majeures l'une migrant au niveau de la chaîne α1 du collagène I témoin, l'autre aux environs de 140 KDa. En absence d'agent réducteur, la migration de la bande inférieure n'est pas modifiée (confirmant l'absence du C-propeptide). La bande à 140KDa en l'absence d'agent réducteur disparaît partiellement et apparait une bande majoritaire migrant aux alentours de 300 KDa (au niveau des bandes γ du collagène I témoin). Ce résultat témoigne de la présence de pontage interchaînes entre les cystéines du C-propeptide de la chaîne α1(I) recombinante (fig. 4 partie C).

### EXEMPLE 8:Formation de la triple hélice

### Digestion protéolytique

Une digestion ménagée à la trypsine ou à la pepsine permet d'attester la formation d'une triple hélice et donc de l'homotrimère [α1(I)]3 . En effet, les extrémités N- et C- terminales sont sensibles aux protéases alors que la partie centrale de la molécule, lorsque la triple hélice est formée, est résistante. Les conditions de digestion permettant la digestion du collagène I dénaturé sans altérer le collagène I natif sont retenues. Les échantillons extraits en milieu neutre sont digérés par la trypsine selon un rapport enzyme-substrat de 1:20, à 25°C pendant 10 minutes. La réaction est arrêtée par addition de TCA 10% final suivie de deux lavages éthanol 100% froid. Les échantillons sont repris dans du tampon échantillon contenant 10mM DTT, chauffés à 100°C pendant 3 minutes et déposés sur un gel d'électrophorèse 6%. Les gels sont colorés au bleu de Coomassie ou immunotransférrés et révélés par les anticorps anti-collagène I (référence 20121, Institut Pasteur Lyon) comme indiqué précédemment. Les plants pour lesquels la triple hélice a été formée permettront l'observation d'une bande unique migrant au niveau de la chaîne α1(I) témoin.

La formation de la triple hélice peut-être également mise en évidence par l'utilisation de la pepsine. Dans ce cas les échantillons sont dialysés contre de l'acide acétique 0.5M, 200mM NaCl, pH 2.5 puis soumis à une digestion par la pepsine. Les conditions de digestion utilisées sont celles qui permettent la digestion complète de collagène I témoin dénaturé. La réaction est arrêtée par neutralisation du milieu de digestion. Les échantillons sont alors précipités au TCA 10% final et traités comme indiqué ci-dessus.

On peut également utiliser les digestions aux protéases pour déterminer la température de dénaturation du collagène recombinant et donc la stabilité de la triple hélice formée dans les plantes. Pour cela les échantillons sont digérés par la trypsine à des températures de réaction croissantes (de 25°C à 45°C), puis traités comme indiqués ci-dessus. L'apparition de bandes correspondant à de la dégradation protéolytique indique la température de dénaturation du collagène recombinant. La température du collagène I natif est de 41°C; celle de l'homotrimère α1(I) recombinant réalisé dans des cellules eucaryotes est similaire à celui du collagène I témoin mais toutefois, l'homotrimère est partiellement clivé dès une température de 38°C (Geddis et al., 1993).

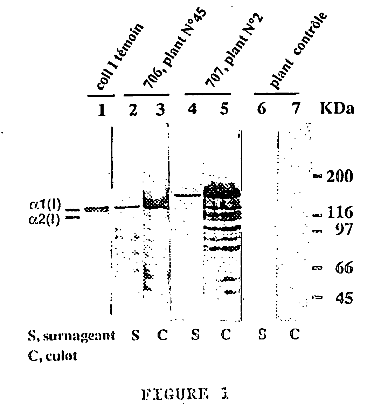

Après digestion à la trypsine du collagène extrait des plants transformés avec la construction pBIOC707 et pBIOC706 comme décrit précédemment, on observe la persistance d'une bande migrant au niveau de la chaine α1(I) du collagène I témoin. Ce résultat montre que le collagène homotrimérique extrait des plants transformés est replié en triple hélice (Fig 1, partie A).

### Ombrage tournant

La formation de la triple hélice peut-être également attester par observation au microscope électronique à transmission de répliques des molécules obtenues après ombrage tournant. En effet, la molécule de collagène lorsqu'elle est repliée en triple hélice se présente sous une forme caractéristique de bâtonnet de 300 nm de long et 1.4 nm de diamètre. Les chaînes α1(I) seules ne sont pas identifiables par cette technique. Les échantillons à une concentration de 10 à 20µg/ml sont dialysés contre du bicarbonate d'ammonium 200 mM pendant une nuit à 4°C, puis mélangés dans un rapport 1:1 avec du glycérol. 5µl sont déposés sur une feuille de mica fraîchement clivée de 1cm², puis placés dans un évaporateur (Med 10, Balzers TM). Environ 2 nm de platine-carbone sont évaporés sur les échantillons à un angle de 8° sous un vide de 2.10⁻⁶ Torr, suivi d'une évaporation de carbone à 90° pendant quelques secondes. Les répliques sont décollées par pénétration à 45° des micas dans un récipient d'eau distillée filtrée et montées sur des grilles de microscopie électronique (600 mesh, cuivre). L'observation se fait sur un microscope à transmission (CM120, Philipps TM).

La formation d'une triple hélice a été confirmée par des observations en microscopie électronique du collagène purifié à partir de plants positifs après ombrage tournant. Les molécules observées se présentent sous la forme caractéristique de bâtonnets de 300 nm de long (Fig 6, partie B). On note l'absence de domaine globulaire à l'une des 2 extrémités de la triple hélice confirmant le clivage du C-propeptide.

### EXEMPLE 9 :

### Ultrastructure des plants transformés:

Un fragment de la feuille de chaque plant transformé positif et d'un témoin non transformé sont fixés dans un mélange fixateur glutaraldéhyde 2%, paraformaldéhyde 0.5% dans un tampon citrate-phosphate 0.1M pH 6.8 pendant 8 heures à température ambiante. Pendant la fixation et ultérieurement si nécessaire, les échantillons sont placés dans une cloche à vide pour les dégazer. Les échantillons sont rincés dans le tampon citrate-phosphate plusieurs bains de 15 minutes puis post-fixés dans une solution d'acide osmique 2% dans le tampon citrate-phosphate 0.1M pendant 2 heures à température ambiante. Les échantillons sont alors déshydratés par des bains successifs d'éthanol de 30° à 100°. L'éthanol absolu est remplacé par un bain d'oxyde de propylène pur, renouvelé 2 fois. La substitution se fait dans des bains contenant un mélange d'oxyde de propylène pur et de résine Epoxy à raison de 1 volume pour 3 volumes respectivement puis 1:1 et finalement 3:1. L'imprégnation se fait dans la résine Epoxy pure sur une nuit et peut se poursuivre en changeant les bains jusqu'à complète immersion des échantillons. Les échantillons sont alors inclus dans des gélules et mis à polymériser pendant 3 jours à 60°C. Des sections ultrafines sont réalisées, contrastées par l'acétate d'uranyle 7% dans du méthanol pendant 10 minutes puis par le citrate de plomb pendant 5 minutes. Les observations se font sur un microscope à transmission électronique (CM120, Philipps TM).

### EXEMPLE 10 :

### Purification du collagène recombinant

Le collagène recombinant est purifié à partir des extraits des plantes en utilisant les propriétés physico-chimiques qui lui sont propres. L'acide fait précipiter grand nombre de protéines et les profils électrophorétiques obtenus à partir d'extraction de plants contrôles en milieu acide montrent que peu de protéines endogènes sont extraites. De plus, la chlorophylle n'est pas présente dans ces extraits. Le collagène étant soluble dans l'acide, les différents extraits sont dialysés contre l'acide acétique 0.5M lorsque ceux-ci n'ont pas été extraits directement en milieu acide. Après centrifugation pour éliminer le matériel insoluble, le collagène sera purifié par précipitation fractionnée par les sels en milieu acide : à 0.7M NaCl en acide acétique 0.5M, puis après centrifugation le surnageant est filtré, précipité à 0.9M NaCl en acide acétique 0.5M, puis à nouveau centrifugé pour obtenir le précipité dans le culot. Les deux précipités 0.7 et 0.9M, contenant l'échantillon sont repris en acide acétique 0.5M puis sont soumis à une électrophorèse sur acrylamide 6% . La pureté des fractions est estimée après coloration des gels en bleu de Coomassie. Pour les échantillons estimés insuffisamment purs, un deuxième cycle de précipitation par les sels peut être réalisé après dialyse de la fraction contre de l'acide acétique 0.5M pour éliminer les traces de sels résiduels. Dans le cas où une étape de purification supplémentaire serait nécessaire, une colonne échangeuse d'ions est réalisée, l'élution se fait par un gradient de NaCl de 0 à 0.5M, les fractions sont soumises à un gel d'électrophorèse comme indiqué précédemment.

Les résultats montrent qu'une majeure partie des protéines des plantes sont éliminées par une étape de précipitation par NaCl 0.4M. Après précipitation par NaCl 0.7M puis 0.9M comme indiquée dans l'exemple 10, le collagène homotrimérique est observé pur à 80-90% dans le précipité NaCl 0.9M .

Un site de liaison à l'héparine situé sur le collagène hétérotrimèrique a été décrit dans la littérature (San Antonio et coll., 1994, J. Cell Biol., 125:1179-1188). De tels sites sont responsables de la fixation de protéoglycannes présents dans la matrice extracellulaire mais aussi de l'adhésion des cellules via des récepteurs de type protéoglycannes membrannaires. Ces interactions permettent ainsi d'assurer la cohésion du réseau collagénique. La persistance de ce site sur l'homotrimère sera analysée par fixation de l'homotrimère sur colonne d'affinité à l'héparine dans des conditions physiologiques de pH (7.4) et de force ionique (NaCl 0.15M). Cette méthode peut servir de deuxième étape de purification.

### EXEMPLE 11 :

### Composition en acide aminé et microséquençage:

Les différents produits obtenus après extraction des plants transformés sont hydrolysés sous vide par de l'HCl 6N à 115°C pendant 24 heures dans un système PicoTag (Waters, TM). La composition en acide aminés est déterminée à l'aide d'un analyseur automatique Beckman TM. Cette analyse permet en particulier de vérifier le taux de proline hydroxylée, l'hydroxylation des prolinés garantissant la stabilité de la triple hélice.

Le microséquençage s'effectue selon la méthode d'Edman avec un appareil permettant le séquençage N-terminal automatique des protéines (Applied Biosystems 473 A protein sequencer TM). Les collagènes ou procollagènes recombinants peuvent être chargés sur l'appareil de 2 façons:
- après purification, la protéine se présente sous la forme d'une solution concentrée. La protéine est fixée par liaisons hydrophobes sur une pastille de fibre de verre traitée au polybrène. La pastille de verre est directement introduite dans l'appareil.
- après électrophorèse et électrotransfert, les bandes d'intérêt sont repérées après coloration au rouge Ponceau 0.02% dans l'acide acétique 0.1% et décoloration à l'eau distillée filtrée, découpées au scalpel puis introduites dans une cellule adaptée à l'appareil.

Selon le principe de la méthode d'Edman, chaque cycle libère un acide aminé sous forme d'un complexe phénylthiohydantoine-acide aminé. Ils sont successivement et automatiquement injectés sur une colonne de chromatographie haute pression (HPLC) en phase inverse et détectés par leur absorbance UV à 269 nm. Par comparaison entre leur temps de rétention et le spectre des standards, on détermine ainsi cycle après cycle la séquence N-terminale de la protéine analysée.

Le collagène obtenu par précipitation à NaCl 0.9M est soumis à une étape supplémentaire de purification (colonne de chromatographie en phase inverse (C8) en HPLC) avant de procéder à l'analyse en acides aminés. L'analyse obtenue est conforme à celle prédite à partir de l'ADNc de la chaîne α1(I) humaine en particulier pour les résidus caractéristiques du collagène, la Glycine et la Proline:

| | Glycine | Proline (Pro+OHPro) |
|---|---|---|
| α1(I) prédiction ADNc | 32.8% | 22.7% |
| α1(I) extraite des plantes | 28.4% | 20.6% |

D'autre part, afin de vérifier si le clivage du peptide signal de la plante présent dans les constructions pBIOC707 et pBIOC706 s'est effectué correctement mais également pour s'assurer qu'aucune dégradation protéolytique ne s'est produite en extrémité N-terminale au cours de l'extraction, chacune des bandes inférieures observées sur gel d'électrophorèse pour le collagène extrait des plants transformés avec la construction pBIOC707 et pBIOC706 a été soumise à un séquençage en N-terminal par dégradation d'Edmann après electrotransfert.

Les séquences obtenues sont: ELAPQLSY pour le collagène correspondant à la construction pBIOC706 et AQVEGQDE pour la construction pBIOC707. Ces résultats montrent que le clivage du peptide signal s'est effectivement produit et d'autre part confirment que les extrémités N-terminales (le N-télopeptide la construction pBIOC706 et le N-propeptide pour la construction pBIOC707) sont intactes. Par ailleurs, l'ensemble des résultats (exemples 6, 7, 8 et 11) montrent que le collagène extrait de plantes à maturité correspond à un collagène homotrimérique dont le C-propeptide est clivé.

### Exemple 12: Accumulation du collagène dans les plantes matures.

Les jeunes plants transformés qui se sont avérés positifs par immunotransfert ont été amenés jusqu'à maturité. Les feuilles des plantes ont été cueillies et, soit directement congelées puis broyées à froid, soit lyophilisées. Quelle que soit la méthode de stockage des feuilles (congelation ou lyophilisation), le collagène est extrait aisément en milieu acide selon les conditions décrites dans l'exemple 6. Après extraction, on retrouve une seule bande majoritaire pour chacun des extraits obtenus à partir de plantes transformés avec la construction pBIOC707 et pBIOC706. Dans chacun des cas cette bande correspond à l'homotrimère après clivage du C-propeptide (voir exemple 6). D'autre part, il est montré que le collagène peut s'accumuler dans les plantes matures et générer ainsi une quantité importante de collagène par gramme de poids frais. Un jeune plant (plant 45 par exemple) peut fournir un extrait contenant environ 6-8µg/ml de collagène homotrimèrique alors qu'arrivée à maturité la même plante permettra l'extraction de 40-50µg/ml soit 0.5mg/g de poids frais. On appelle procollagène la molécule de collagène contenant les N- et C-propeptides, pN-collagène et pC-collagène les molécules qui contiennent l'un ou l'autre des domaines terminaux. Enfin le collagène est la forme moléculaire ne présentant que le domaine en triple hélice et correspond dans les tissus animaux à la forme mature. Par conséquent, les produits extraits sont identifiés comme pN-collagène (construction pBIOC707) et collagène (construction pBIOC706).

### Exemple 13: Fibrillogénèse et test d'adhésion

Le collagène purifié est dilué à 0.2-0.5 mg/ml dans de l'acide acétique 0.1M puis dialysé contre un tampon phosphate salin (PBS), pH 7.4 à 4°C pendant une nuit. L'échantillon est alors placé dans un bain-marie et la températutre est augmentée progressivement jusqu'à 30°C et stabilisée à cette température pendant 2 heures. La formation des fibres est observée en microscopie électronique après coloration négative ou positive des échantillons. La cinétique de formation des fibres est suivie par turbidimétrie à l'aide d'un spectrophotomètre, la formation des fibres modifiant la densité optique (Wood et Keech, 1960, Biochem. J., 75: 588-598).

Le collagène I est une protéine adhésive c'est-à-dire que de nombreux types cellulaires sont capables de le reconnaître et de se fixer dessus de façon spécifique par l'intermédiaire de récepteurs membranaires. Il est possible de tester les propriétés adhésives d'une protéine à l'aide d'un test colorimétrique réalisé en microplaques (Aumailley et coll. 1989, Exp. Cell Res. 181, 463-474).

Le collagène extrait est adsorbé pendant une nuit à 4°C sur des plaques multi-puits, puis mis en contact avec une suspension cellulaire à 37°C pendant 20 à 30 minutes. Les cellules non-adhérentes sont éliminées par lavage, les cellules adhérentes sont fixées au glutaraldéhyde 1%, puis colorées au cristal violet 0.1%. Après lavage intensif des plaques, le colorant fixé par les cellules adhérentes est solubilisé par du Triton X100. Les plaques sont lues sur un lecteur ELISA à 570nm, la valeur de la densité optique est fonction du nombre de cellules adhérentes.

### REFERENCES BIBLIOGRAPHIQUES

- Amstrong et al. (Malze Handbook; (1994) M. Freeling, V. Walbot Eds.; pp. 665-671).
- An et al, Plant Physiol, 81, 301-305 (1986)
- Anderson O.D. et Greene F.C., T.A.G., 77, 689-700 (1989).
- Barta *et al*., Plant Mol. Biol., 6, 347-357 (1986).
- Bednareck et al Plant cell 3, 1195-1206 (1991).
- Cornelissen et al, Nature, 321, 531-532 (1986)
- Depigny-This et al., Plant. Mol. Biol., 20, 467-479 (1992).
- Gaubier et al., Mol. Gen. Genet., 238, 409-418 (1993).
- Gedis et al Matrix , 13, 399-405 (1993)
- Holsters et al, Mol Gen Genet, 163, 181-187 (1978)
- Horsch R.B et al. Science, 227, 1229-1231 (1985).
- Kay *et al.*, Science, 236, 1299-1302 (1987).
- Li S-W et al. Matrix Biology 14, 593-595 (1994)
- Mc Elroy et al, Mol Gen Genet, 231, 150-160 (1991)
- Marx, Science, 216, 1305-1307 (1982).
- Matsuoka K Proc. Natl. Acad. Sci. USA, 88, 834-838 (1991).
- Morikawa et al. Biochemistry 19, 2646-2650 (1980)
- Murakami *et al*., Plant Mol. Biol., 7, 343-355 (1986).
- Ni *et al*., Plant J., 7, 661-676 (1995).
- Parkany M, Molecular biomaterials G.W. Hastings, P. Ducheyne eds, CRC Press, Boca Raton, 111-117 (1984)
- Reina *et al*., Nucleic Acid Research, 18, 6426 (1990).
- Schroeder M.R. et al, Plant Physiol., 101, 451-458 (1993).
- Vain P. *et al*., Plant Cell Tissue and Organ Culture, 18, 143-151 (1989).

## Revendications

1. Utilisation d'une séquence nucléotidique recombinante contenant d'une part un ADNc codant pour une ou plusieurs chaînes de collagène de mammifère et d'autre part, les éléments permettant à une cellule végétale de produire la ou les chaînes de collagène, codées par ledit ADNc, dont un promoteur et un terminateur de transcription reconnus par la machinerie transcriptionnelle des cellules végétales, pour la transformation de cellules de plantes en vue de l'obtention, à partir de ces cellules, ou de plantes obtenues à partir de ces dernières, de la ou des chaînes de collagène.

2. Séquence nucléotidique recombinante, **caractérisée en ce qu'**elle contient d'une part, la séquence codante pour une ou plusieurs chaînes de collagène de mammifère, et d'autre part, les éléments permettant à une cellule végétale de produire la ou les chaînes de collagène codées par ladite séquence, dont un promoteur et un terminateur de transcription reconnus par la machinerie transcriptionnelle des cellules végétales.

3. Vecteur, contenant une séquence nucléotidique selon la revendication 2, insérée en un site non essentiel pour sa réplication.

4. Vecteur selon la revendication 3, **caractérisé en ce qu'**il est un plasmide, contenant une séquence nucléotidique selon la revendication 2, insérée en un site non essentiel pour sa réplication.

5. Hôte cellulaire, transformé par un vecteur selon la revendication 3 ou la revendication 4.

6. Hôte cellulaire selon la revendication 5, choisi parmi les bactéries telle que Agrobacterium tumefaciens.

7. Procédé d'obtention d'une ou plusieurs chaînes de collagène, **caractérisé en ce qu'**il comprend :
- la transformation de cellules végétales, à l'aide d'un hôte cellulaire selon la revendication 5 ou 6, lui-même transformé par un vecteur selon la revendication 3 ou 4, de manière à intégrer dans le génome de ces cellules une séquence recombinante selon la revendication 2,
- l'obtention de plantes transformées à partir des cellules transformées susmentionnées,
- la récupération de la ou des chaînes de collagène recombinants produits dans lesdites cellules ou plantes transformées susmentionnées, par extraction, suivie, par une purification.

8. Plantes, ou parties de plantes, feuilles et/ou fruits et/ou semences et/ou cellules de plantes, transformés génétiquement, **caractérisées en ce qu'**elles contiennent une (ou plusieurs) séquence (s) nucléotidique (s) recombinante (s) selon la revendication 2, intégrée (s) de façon stable dans leur génome, ou exprimant du collagène recombinant codées par lesdites séquences nuctéotidiques ces plantes étant choisies parmi le colza, le tabac, le maïs, le pois, la tomate, la carotte, le blé, l'orge, la pomme de terre, le soja, le tournesol, la laitue, le riz, la luzerne, et la betterave.

9. Chaîne de collagène' recombinante, **caractérisée en ce qu'**elle est susceptible d'être obtenue selon le procédé de la revendication 7.

10. Collagène **caractérisé en ce qu'**il est susceptible d'être obtenue selon le procédé de la revendication 7 et est homotrimérique, et que le Cpropeptide est clivé.

11. Produit collagénique, constitué de chaînes de collagène ou de collagène selon les revendications 9 ou 10.

12. Gélatine constituée de chaînes de collagène selon les revendications 9 ou 10.

13. Biomatériau et composition pharmaceutique, médicale, odontologique, cosmétique ou biotechnologique comprenant un produit selon la revendication 11.

14. Plantes, ou parties de plantes, feuilles et/ou fruits et/ou semences et/ou cellules de plantes, transformées génétiquement, **caractérisées en ce qu'**ils contiennent des chaînes de collagène, ou du collagène selon les revendications 10 ou 11, ces plantes étant choisies parmi le colza, le tabac, le maïs, le pois, la tomate, la carotte, le blé, l'orge, la pomme de terre, le soja, le tournesol, la laitue, le riz, la luzerne, et la betterave.

15. Utilisation de plantes, ou parties de plantes selon la revendication 8 ou 14, et/ou de produits selon les revendications 9 à 12, pour l'obtention de compositions pharmaceutiques, médicales, odontologiques, cosmétiques ou biotechnologiques.

## Patentansprüche

1. Verwendung einer rekombinierten Nucleotidsequenz einerseits enthaltend eine cDNA, kodierend für eine oder mehrere Kollagenketten von Säugern und andererseits Elemente, die es einer Pflanzezelle ermöglichen, eine oder mehrere Kollagenketten zu produzieren, kodiert durch besagte cDNA, deren Promotor und Terminationsstelle der Transkription von der Transkriptionsmaschinerie der Pflanzenzelle erkannt werden, zur Umwandlung einer Pflanzenzelle im Hinblick auf die Gewinnung einer oder mehrerer Kollagenketten, ausgehend von diesen Zellen oder Pflanzen, die ausgehend von letzteren gewonnen werden.

2. Rekombinierte Nucleotidsequenz, dadurch charakterisiert, dass sie einerseits die kodierende Sequenz für eine oder mehrere Kollagenketten von Säugern enthält und andererseits Elemente, die es einer Pflanzenzelle ermöglichen eine oder mehrere Kollagenketten zu produzieren, kodiert durch besagte Sequenz, deren Promotor und Terminationsstelle der Transkription durch die Transkriptionsmaschinerie der Pflanzenzelle erkannt werden.

3. Vektor enthaltend eine Nucleotidsequenz gemäß Anspruch 2, eingefügt an einer für seine Replikation nicht essentiellen Stelle.

4. Vektor gemäß Anspruch 3, dadurch charakterisiert, dass er ein Plasmid ist, enthaltend eine Nucleotidsequenz gemäß Anspruch 2, eingefügt an einer für seine Replikation nicht essentiellen Stelle.

5. Wirtszelle, transformiert mit einen Vektor gemäß Anspruch 3 oder 4.

6. Wirtzelle gemäß Anspruch 5, ausgewählt aus Bakterien wie Agrobacterium tumefaciens.

7. Verfahren zur Gewinnung einer oder mehrerer Kollagenketten, dadurch charakterisiert, dass es umfasst:
- die Transformation einer Pflanzenzelle mit Hilfe einer Wirtszelle gemäß Anspruch 5 oder 6, selbst transformiert mit einem Vektor gemäß Anspruch 3 oder 4 auf die Weise, dass in das Genom dieser Zellen eine rekombinierte Sequenz gemäß Anspruch 2 eingefügt wurde,
- die Gewinnung transformierter Pflanzen ausgehend von den oben genannten transformierten Zellen
- die Rückgewinnung einer oder mehrerer rekombinanter Kollagenketten hergestellt in den oben genannten transformierten Zellen oder Pflanzen, durch Extraktion, nachfolgend durch Aufreinigung.

8. Pflanzen oder Pflanzenteile, Blätter und/oder Früchte und/oder Samen und/oder Zellen der Pflanzen, genetisch transformiert, dadurch charakterisiert, dass sie eine (oder mehrere) rekombinierte Nucleotidsequenz(en) gemäß Anspruch 2 enthalten, die stabil in ihr Genom eingefügt ist (sind), oder rekombinantes Kollagen exprimieren, kodiert durch besagte Nucleotidsequenzen, wobei diese Pflanzen ausgewählt sind aus der Gruppe Raps, Tabak, Mais, Erbse, Tomate, Karotte, Weizen, Gerste, Kartoffel, Soja, Sonnenblume, grüner Salat, Reis, Luzerne und Rübe.

9. Rekombinante Kollagenkette, dadurch charakterisiert, dass sie gemäß dem Verfahren nach Anspruch 7 erhältlich ist.

10. Kollagen, dadurch charakterisiert, dass es erhältlich ist gemäß dem Verfahren nach Anspruch 7 und ein Homotrimer ist, und dass das Propeptid gespalten wird.

11. Kollagenprodukt bestehend aus Kollagenketten oder Kollagen gemäß der Ansprüche 9 oder 10.

12. Gelatine bestehend aus Kollagenketten gemäß der Ansprüche 9 oder 10.

13. Biomaterial und pharmazeutische, medizinische, zahnmedizinische, kosmetische oder biotechnologische Zusammensetzung umfassend ein Produkt gemäß Anspruch 11.

14. Pflanzen oder Pflanzenteile, Blätter und/oder Früchte und/oder Samen und/oder Pflanzenzellen, genetisch transformiert, dadurch charakterisiert, dass sie Kollagenketten oder Kollagen gemäß der Ansprüche 10 oder 11 enthalten, wobei diese Pflanzen ausgewählt sind aus der Gruppe Raps, Tabak, Mais, Erbse, Tomate, Karotte, Weizen, Gerste, Kartoffel, Soja, Sonnenblume, grüner Salat, Reis, Luzerne und Rübe.

15. Verwendung von Pflanzen oder Pflanzenteilen gemäß Anspruch 8 oder 14 und/oder von Produkten gemäß der Ansprüche 9 bis 12, um eine pharmazeutische, medizinische, zahnmedizinische, kosmetische oder biotechnologische Zusammensetzung zu gewinnen.

## Claims

1. Use of a recombinant nucleotide sequence comprising on the one hand a cDNA coding for one or more mammalian collagen chains and on the other hand the elements permitting a vegetable cell to produce the collagen chain(s) coded for by said cDNA, including a transcription promoter and terminator recognised by the transcription machinery of the vegetable cells, for the transformation of plant cells in order to obtain collagen chain(s) from those cells or from plants obtained from those cells.

2. Recombinant nucleotide sequence, **characterised in that** it comprises on the one hand the sequence coding for one or more mammalian collagen chains and on the other hand the elements permitting a vegetable cell to produce the collagen chain(s) coded for by said sequence, including a transcription promoter and terminator recognised by the transcription machinery of the vegetable cells.

3. Vector comprising a nucleotide sequence according to claim 2 inserted at a non-essential site for its replication.

4. Vector according to claim 3, **characterised in that** it is a plasmid comprising a nucleotide sequence according to claim 2 inserted at a non-essential site for its replication.

5. Host cell transformed by a vector according to claim 3 or claim 4.

6. Host cell according to claim 5, selected from bacteria such as Agrobacterium tumefaciens.

7. Method of obtaining one or more collagen chains, **characterised in that** it comprises:
- transforming vegetable cells with the aid of a host cell according to claim 5 or 6 which has itself been transformed by a vector according to claim 3 or 4, in such a manner as to integrate into the genome of those cells a recombinant sequence according to claim 2,
- obtaining transformed plants from the transformed cells mentioned above,
- recovering the recombinant collagen chain(s) produced in said cells or transformed plants mentioned above, by extraction followed by purification.

8. Genetically modified plants, or parts of plants, leaves and/or fruits and/or seeds and/or cells of plants, **characterised in that**'they comprise one (or more) recombinant nucleotide sequence(s) according to claim 2, stably integrated into their genome, or expressing recombinant collagen coded for by said nucleotide sequences, the plants being selected from rape, tobacco, maize, pea, tomato, carrot, wheat, barley, potato, soybean, sunflower, lettuce, rice, lucerne and beet.

9. Recombinant collagen chain, **characterised in that** it is obtainable according to the method of claim 7.

10. Collagen, **characterised in that** it is obtainable according to the method of claim 7 and is homotrimeric, and **in that** the C propeptide has been cleaved.

11. Collagen product composed of collagen chains or of collagen according to claim 9 or 10.

12. Gelatin composed of collagen chains according to claim 9 or 10.

13. Biomaterial and pharmaceutical, medical, odontological, cosmetic or biotechnological composition comprising a product according to claim 11.

14. Genetically modified plants, or parts of plants, leaves and/or fruits and/or seeds and/or cells of plants, **characterised in that** they comprise collagen chains or collagen according to claim 10 or 11, the plants being selected from rape, tobacco, maize, pea, tomato, carrot, wheat, barley, potato, soybean, sunflower, lettuce, rice, lucerne and beet.

15. Use of plants, or parts of plants, according to claim 8 or 14 and/or of products according to claims 9 to 12 for obtaining pharmaceutical, medical, odontological, cosmetic or biotechriological compositions.
